# EUROPEAN PATENT SPECIFICATION

(11) **EP 0 819 672 B1**
(45) Date of publication and mention of the grant of the patent: **18.04.2001**
(21) Application number: 97117814.0
(22) Date of filing: 25.08.1995
(51) Int. Cl.: C07C 259/04, C07C 259/06, C07D 207/46, C07D 211/94, G03C 1/34

(54) **Hydroxamic acid derivatives and their use for improving latent image stability of a silver halide photographic material**
Hydroxamsäure-Derivate und deren Verwendung zur Verbesserung der Latentbild-stabilität eines photographischen Silberhalogenidmaterials.
Dérivés d'acide hydroxamique et leur utilisation pour l'amélioration de la stabilité de l'image latente d'un matériau photographique à l'halogénure d'argent.

(30) Priority: 25.08.1994 JP 22273194; 11.04.1995 JP 10918295
(43) Date of publication of application: 21.01.1998
(62) Divisional of application: 95113374.3
(73) Proprietor: FUJI PHOTO FILM CO., LTD., Kanagawa-ken (JP)
(72) Inventor: Mikoshiba, Hisashi, Minami, Ashigara-shi, Kanagawa (JP); Takizawa, Hiroo, Minami, Ashigara-shi, Kanagawa (JP); Hosokawa, Junichiro, Minami, Ashigara-shi, Kanagawa (JP); Ishii, Yoshio, Minami, Ashigara-shi, Kanagawa (JP); Mihayashi, Keiji, Minami, Ashigara-shi, Kanagawa (JP); Morigaki, Masakazu, Minami, Ashigara-shi, Kanagawa (JP)
(74) Representative: Lethem, David J.

(56) References cited:
- EP-A- 0 384 912
- EP-A- 0 451 833
- WO-A-92/09556
- US-A- 4 330 606
- US-A- 5 254 724
- DATABASE WPI Derwent Publications Ltd., London, GB; AN 84-315719 XP002009989 & JP 59 198 453 A (FUJI SHASHIN FILM KK) 10 November 1984
- Y. OKAHATA ET AL.: "Nucleophilic Cleavage of Phosphate Triesters in Dialkylammonium Bilayer Membranes" BULL.CHEM.SOC.JPN., vol. 54, no. 7, 1981, pages 2072-2078, XP002009987
- S.K. SHARMA ET AL.: "Spermexatin and Spermexatol: New Synthetic Spermidine-based Siderophore Analogues" J.MED.CHEM., vol. 32, no. 2, 1989, pages 357-367, XP002009988

## Description

### FIELD OF THE INVENTION

The present invention relates to a light-sensitive silver halide photographic material and, more particularly, to a silver halide photographic material which produces less fluctuation in the photographic properties during storage after production and after photographing until development processing.

The present invention also relates to a silver halide photographic material which generates less fog.

Further, the present invention relates to a novel hydroxamic acid based compound which provides photographically useful effect.

### BACKGROUND OF THE INVENTION

A silver halide photographic material is required to be high sensitive and produce less fluctuation in the photographic properties during storage after production and after photographing until development processing.

Of the fluctuation of the photographic properties after photographing until development processing, with respect to the prevention of intensification of latent image, a method of using a hardening agent having an active vinyl group in combination with a triazine based compound is disclosed in JP-A-59-162546 (the term "JP-A" as used herein means a "published unexamined Japanese patent application").

However, the above method is not sufficient in the preventing effect and a further improvement has been desired.

On the other hand, in a full color photographic material, a multilayer structure comprising a plurality of emulsions having different spectral sensitivities is used to achieve the object of a full color photograph. However, although the emulsions for such a usage have been considerably improved, fog, intensification and fading of a latent image are liable to occur, therefore, they are not necessarily sufficient. 2-Hydroxyamino-1,3,5-triazines, for example, are useful for the improvement of such storage stabilities. However, the storage stability of each layer varies according to the emulsion used in each layer. Therefore, a method to improve the storage stability of the latent image of the emulsion of a specific layer has been strongly desired in recent years.

Many of known 2-hydroxylamine-1,3,5-triazines are diffusible, therefore, they have a drawback such that their effects are exerted to emulsions of layers other than the objective layer. Moreover, these compounds affect film strength and gradation, and have a drawback of reacting with a hardening agent. On the other hand, hydroxamic acids having a specific structure are disclosed in U.S. Patent 4,339,515, 4,330,606, JP-A-59-198453 and JP-A-3-293666, but the use purposes of them are different from the object of the present invention.

That is, the hydroxamic acids disclosed in JP-A-3-293666 and JP-A-59-198453 are used by adding to the silver halide photographic material or a processing bath as a nucleophilic agent and not to improve the storage stability of the latent image which is the object of the present invention.

In addition, the hydroxamic acids disclosed in U.S. Patents 4,339,515, 4,330,606, JP-A-6-175317 and JP-A-6-157447 are used by adding to the photographic material as a discoloration inhibitor and not to improve the storage stability of the latent image which is the object of the present invention.

WO 92/09556 discloses improved matrix metalloprotease inhibitors. This document mentions two general formulae (1 and 2) which to some degree encompass hydroxamic acids.

EP-A-0 384 912 discloses a hydroxamate bleach stabiliser. Specific examples of the hydroxamate acids used in the invention include N-methyl-β-n-decylsuccinomonohydroxamic acid and N-methyl-α-dodecenylsuccinomonohydroxamic acid.

Accordingly, although the use of hydroxamic acid having a specific structure in the photographic material has been known, the use purpose and the usage are completely different and the fact that hydroxamic acid'has the effect on the storage of the latent image was not known absolutely.

The present invention is to provide a method of improving storage stability of the emulsion of the above specific layer and the stability of the latent image.

To resolve the above problems, the present inventors have endeavored to study the method of improving the storage stability of the emulsion prepared and that of the latent images. As a result, the present inventors have found that hydroxamic acid based compounds have, other than the nucleophilic function and discoloration inhibiting effect, the effect of improving the storage stability of the latent images which is a novel effect.

Further, it has been found that the compound of the present invention can achieve the object of the present invention without changing the hue of the dye formed, affecting the dye forming speed of the couplers, accelerating the decomposition of the coupler and the dye formed, degrading the film strength, further fogging the emulsion.

### SUMMARY OF THE INVENTION

The object of the present invention is to provide a use of a compound which improves the storage stability of the latent image of solely a specific layer and a method of improving the storage stability of the latent image using said compound.

Another objects of the present invention are to provide a use of a compound for improving the storage stability of the silver halide emulsion, in particular, the fluctuation of fog during storage and the storage stability of the latent images, in particular, sensitivity change after exposure until processing.

A further object of the present invention is to provide a use of a compound which improves the storage stability of the latent image without adversely affecting various photographic properties when added to a photographic material and a method of improving the storage stability of the latent image using said compound.

The above objects of the present invention have been achieved by the following (1), (2), (3) and (4).
(1) A compound of formula (II)': 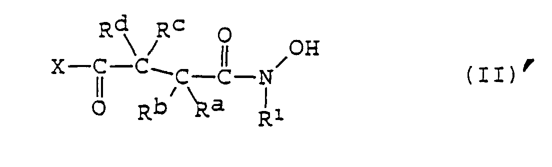 wherein R¹ is a substituted or unsubstituted alkyl group having from 1 to 6 carbon atoms; R^{a}, R^{b}, R^{c} and R^{d} are each independently a hydrogen atom, a substituted or unsubstituted alkyl group having from 1 to 18 carbon atoms, or a substituted or unsubstituted alkenyl group having from 2 to 18 carbon atoms; X is -OR⁶ or -N(R⁶) (R⁷); R⁶ is a substituted or unsubstituted alkyl group having from 6 to 22 carbon atoms, or a substituted or unsubstituted alkenyl group having from 6 to 22 carbon atoms, and R⁷ is an alkyl group having from 6 to 18 carbon atoms, provided that when all of R^{a}, R^{b}, R^{c} and R^{d} are hydrogen atoms, R⁶ and R⁷, if present, are each a substituted or unsubstituted alkyl group having 14 or more carbon atoms; and that the compound of formula (II) is not N-methyl-3-(dioctadecylcarbamoyl) propanohydroxamic acid.
(2) A silver halide photographic material comprising a support having thereon at least one light-sensitive silver halide emulsion layer, wherein said silver halide photographic material contains a compound of formula (II) : 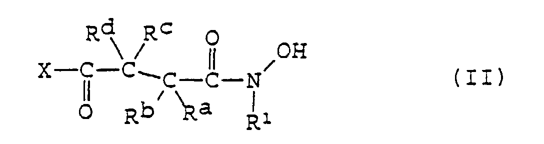 wherein R¹ is a substituted or unsubstituted alkyl group having from 1 to 6 carbon atoms; R^{a}, R^{b}, R^{c} and R^{d} are each independently a hydrogen atom, a substituted or unsubstituted alkyl group having from 1 to 18 carbon atoms, or a substituted or unsubstituted alkenyl group having from 2 to 18 carbon atoms; X is -OR⁶ or -N(R⁶) (R⁷) ; and R⁶ and R⁷, which may be the same or different, are each a hydrogen atom, a substituted or unsubstituted alkyl group having from 1 to 22 carbon atoms, a substituted or unsubstituted alkenyl group having from 2 to 22 carbon atoms, or an aryl group having from 6 to 22 carbon atoms.
(3) A use of a compound represented by formula (II) for improving the storage stability of a latent image of a silver halide photographic material: 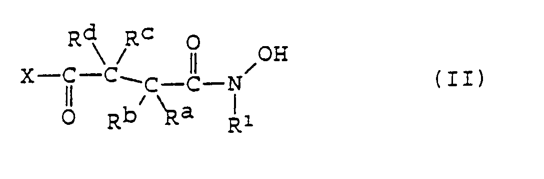 wherein R¹ is a substituted or unsubstituted alkyl group having from 1 to 6 carbon atoms; R^{a}, R^{b}, R^{c} and R^{d} are each independently a hydrogen atom, a substituted or unsubstituted alkyl group having from 1 to 18 carbon atoms, or a substituted or unsubstituted alkenyl group having from 2 to 18 carbon atoms; X is -OR⁶ or -N(R⁶) (R⁷); and R⁶ and R⁷, which may be the same or different, are each a hydrogen atom, a substituted or unsubstituted alkyl group having from 1 to 22 carbon atoms, a substituted or unsubstituted alkenyl group having from 2 to 22 carbon atoms, or an aryl group having from 6 to 22 carbon atoms.
(4) Use of a compound of formula (I) for improving storage stability of a latent image of a silver halide photographic material: 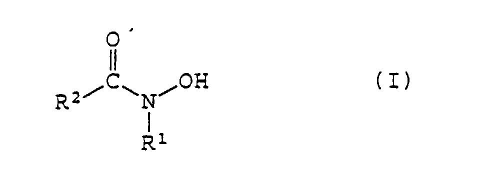 wherein R¹ is a substituted or unsubstituted alkyl group in which the alkyl moiety has from 1 to 6 carbon atoms and R² is a group of formula (I)-A:

   R⁴ - L - R³- (I)-A

   wherein R³ is an alkylene or alkenylene group having from 2 to 20 carbon atoms; R⁴ is a substituted or unsubstituted alkyl group having from 1 to 30 carbon atoms, a substituted or unsubstituted aryl group having from 6 to 20 carbon atoms, or a substituted or unsubstituted heterocyclic group having from 2 to 20 carbon atoms; L is -CO-, -N(R⁵), -S-, -SO₂-, -O-, -SO-, -COO-, -OCO-, -SO₂O-, -OSO₂, -SO₂N(R⁵)-, -N(R⁵)SO₂-, -N(R⁵)CO- or -CON(R⁵)-; and R⁵ is a hydrogen atom or a substituted or unsubstituted alkyl group having from 1 to 18 carbon atoms, provided that when L is -O-, R⁴ is a substituted or unsubstituted alkyl group having from 1 to 40 carbon atoms.

The formula (I) is described in detail below.

In formula (I), R¹ represents a substituted or unsubstituted alkyl group in which the alkyl moiety has from 1 to 6 carbon atoms.

Examples of the alkyl group include methyl, ethyl, sec-butyl, cyclohexyl, chloromethyl, dimethylaminomethyl, trifluoromethyl, 3,3,3-trichloropropyl, and methoxycarbonylmethyl.

The substituents for these alkyl groups are, e.g., an alkyl group, an alkenyl group, an aryl group, a heterocyclic group, a halogen atom, an alkoxyl group, an aryloxy group, an alkylthio group, an arylthio group, a cyano group, a nitro group, an alkoxycarbonyl group, an aryloxycarbonyl group, a hydroxyl group, an acyl group, an acyloxy group, an alkylsulfonyl group, an arylsulfonyl group, an acylamino group, an alkylsulfonamido group, and an arylsulfonamido group, and specific examples include 2-chloroethyl, 2-methoxyethyl, 2-cyanoethyl, 2-ethoxycarbonylethyl, 3-methylthiopropyl, 2-acetylaminoethyl, 3-hydroxypropyl, 2-acetyloxyethyl, 3-chloroethyl, 3-methoxyethylallyl, and prenyl.

R¹ preferably represents an unsubstituted alkyl group.

When the molecular weight of the compound represented by formula (I) of the present invention is small, the compound is easily diffused in the photographic material and the effect of the present invention can be imparted to all the layers of the multilayer structure. In this case, the molecular weight of the compound represented by formula (I) is preferably less than 300, more preferably less than 200.

On the other hand, when the molecular weight of the compound represented by formula (I) is large, it does not diffuse in the photographic material and is fixed in the specific layer where it is added, therefore, the storage stability of the latent image of the objective layer can be improved.

R² is represented by the following formula (I)-A:

R⁴-L-R³- (I)-A

wherein R³ represents an alkylene or alkenylene group having from 2 to 20 carbon atoms; R⁴ represents a substituted or unsubstituted alkyl group having from 1 to 30 carbon atoms, a substituted or unsubstituted aryl group having from 6 to 20 carbon atoms, or a substituted or unsubstituted heterocyclic group having from 2 to 20 carbon atoms; L represents -CO-, -N(R⁵)-, -S-, -SO₂-, -O-, -SO-, -COO-, -OCO-, -SO₂O-, -OSO₂-, -SO₂N(R⁵)-, -N(R⁵)SO₂-, -N(R⁵)CO-or -CON(R⁵)-; and R⁵ represents a hydrogen atom or a subsituted or unsubstituted alkyl group having from 1 to 18 carbon atoms, provided that when L represents -O-, R⁴ represents a substituted or unsubstituted alkyl group having from 1 to 40 carbon atoms. The total carbon atom number of R³, L and R⁴ is preferably 12 or more.

In formula (I)-A, R³ represents an alkylene group or an alkenylene group having from 2 to 20 carbon atoms, and preferably represents a 1,2-ethylene group, a 1,3-propylene group or a substituted 1,2-ethylene group.

Specific examples of R³ are shown below.
-CH₂-CH₂-, -CH₂-CH₂-CH₂-, -CH₂-CH₂-CH₂-CH₂-CH₂-.

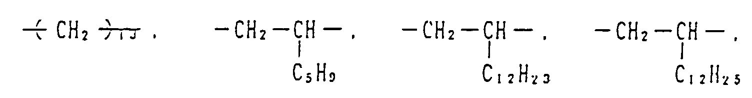

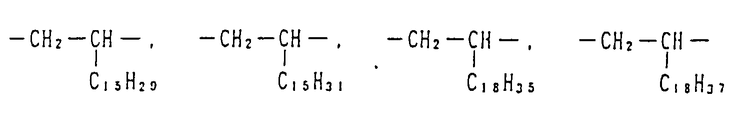

Of the above, R⁴ preferably represents a substituted or unsubstituted alkyl or alkenyl group having from 1 to 30 carbon atoms, and more preferably represents a substituted or unsubstituted alkyl or alkenyl group having from 6 to 22 carbon atoms.

An unsubstituted group represented by R⁴ includes stearyl, palmityl, myristyl, dodecyl, and methyl group. A substituted alkyl group represented by R⁴ includes 2-methoxyethyl and 2-diethylaminoethyl group. An unsubstituted aryl group represented by R⁴ includes phenyl and naphthyl group. A substituted aryl group represented by R⁴ includes 2,4-di-t-octylphenyl and p-n-dodecylphenyl group. An unsubstituted heterocyclic group represented by R⁴ includes morpholino and piperazino group. A substituted heterocyclic group represented by R⁴ includes, for example, 2-methylimidazo-1-yl group.

An unsubstituted alkyl group represented by R⁵ includes n-octyl, methyl, ethyl, isopropyl, n-dodecyl, stearyl, myristyl and 2-ethylhexyl group. A substituted alkyl group represented by R includes 2-nethoxyethyl, 3-methoxypropan-1-yl and 2-chloroethyl group.

L represents -CO-, -N(R⁵)-, -O-, -SO₂-, -S-, -COO-, -OCO-, -CON(R⁵)- or -N(R⁵)CO-; of these, L more preferably represents -O-, -COO-, -CON(R⁵) or -N(R⁵)-.

L represents -CO-, -N(R⁵)-, -O-, -SO₂-, -S-, -COO-, -OCO-, or -CON(R⁵)-; of these, L more preferably represents -O-, -COO-, -CON(R⁵)-, or -N(R⁵)-.

Of these, R⁴-L- preferably represents an alkoxycarbonyl group, an alkenoxycarbonyl group, an aryloxycarbonyl group, a carbamoyl group, an acyl group, an alkoxy group, an alkylthio group, an arylthio group, an alkylsulfonyl group, an arylsulfonyl group.

The total carbon atom number of R⁴, L and R³ is preferably from 12 to 60.

The compound represented by formula (I) is most preferably represented by the following formula (II): 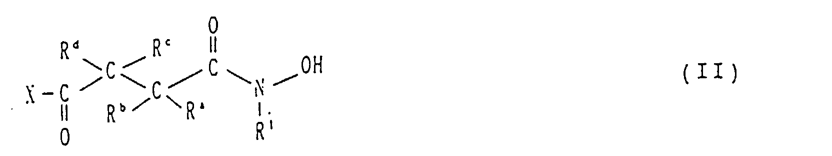 wherein R¹ represents a substituted or unsubstituted alkyl group having from 1 to 6 carbon atoms; R^{a}, R^{b}, R_{c} and R^{d} each independently represents a hydrogen atom, a substituted or unsubstituted alkyl group having from 1 to 18 carbon atoms, or a substituted or unsubstituted alkenyl group having from 2 to 18 carbon atoms; X represents -OR⁶ or -N(R⁶)(R⁷); and R⁶ and R⁷, which may be the same or different, each represents a hydrogen atom, a substituted or unsubstituted alkyl group having from 1 to 22 carbon atoms, a substituted or unsubstituted alkenyl group having from 2 to 22 carbon atoms, or an aryl group having from 6 to 22 carbon atoms.

Substituent which may be substituted on the alkyl, alkenyl and aryl group represented by R^{a}, R^{b}, R^{c}, R^{d}, R⁶ or R⁷ is the same as that substituted on the alkyl group represented by R¹.

R¹ preferably represents a methyl group, an ethyl group or an n-hexyl group. Preferably all of R^{a}, R^{b}, R_{c} and R^{d} represent hydrogen atoms, or at least one of R^{a}, R^{b}, R_{c} and R^{d} represents an alkyl or alkenyl group having from 5 to 18 carbon atoms and others represent hydrogen atoms.

The present invention specifically encompasses compounds of formula (II)', which are a sub-class of formula (II) in which R⁶ represents an alkyl or alkenyl group having from 6 to 22 carbon atoms, provided that when all of R^{a}, R^{b}, R_{c} and R^{d} represent hydrogen atoms, R⁶ has 14 or more carbon atoms; R⁷ represents an alkyl group having from 6 to 18 carbon atoms. -OR⁶ is preferred to -NR⁶R⁷ as X.

One most preferred structure of formula (II) is such that R^{a} represents an alkenyl or alkyl group having from 12 to 18 carbon atoms, R^{b}, R^{c} and R^{d} represent hydrogen atoms, R¹ represents a methyl group or an ethyl group, X represents - OR⁶, and R⁶ represents an alkyl or alkenyl group having from 12 to 18 carbon atoms.

Another most preferred structure of formula (II) is such that R^{c} represents an alkenyl or alkyl group having from 12 to 18 carbon atoms, R^{a}, R^{b} and R^{d} represent hydrogen atoms, R¹ represents a methyl group or an ethyl group, X represents -OR⁶, and R⁶ represents an alkyl or alkenyl group having from 12 to 18 carbon atoms.

Other most preferred structure of formula (II) is such that all of R^{a}, R^{b}, R^{c} and R^{d} represent hydrogen atoms, X represents -OR⁶, and R⁶ represents an alkyl or alkenyl group having from 12 to 18 carbon atoms.

Of the compounds represented by formula (II) of the present invention, most preferably R^{a}, R^{b}, R^{c} and R^{d} all represent hydrogen atoms, R⁶ represents a myristyl group, a palmityl group or a stearyl group, and R¹ represents a methyl group, an ethyl group or an n-hexyl group.

The compound represented by formula (I) preferably has a molecular weight of 300 or more, more preferably 350 or more, and most preferably 450 or more, and 800 or less and more preferably 600 or less.

The compound of the present invention should be substantially water-insoluble as it is nondiffusible in gelatin film. "Substantially water-insoluble" means the solubility in water of 25°C is 5% or less, preferably 1% or less.

The raw material of the compound of the present invention (e.g., acid anhydrides and alcohols as described below) is sometimes available only as a mixture of isomer and homolog. Therefore, the compound of the present invention is sometimes easier to synthesize as a mixture of isomer and homolog. In such a case, the compound of the present invention is preferably added to a silver halide photographic material as a mixture.

Specific examples of the compounds of and/or for use in the present invention are shown below, but the present invention is not limited thereto.

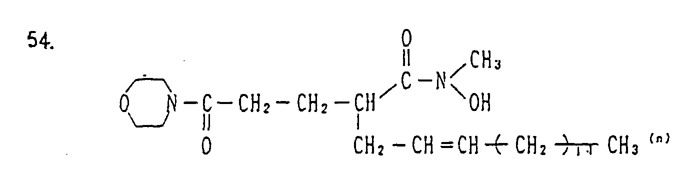

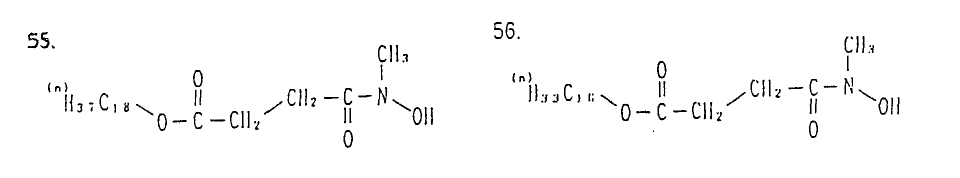

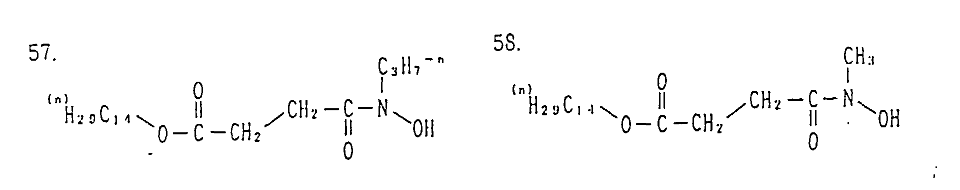

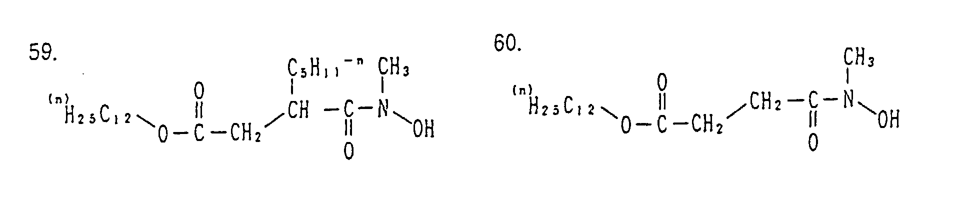

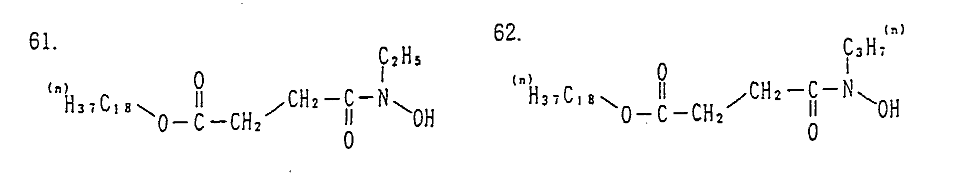

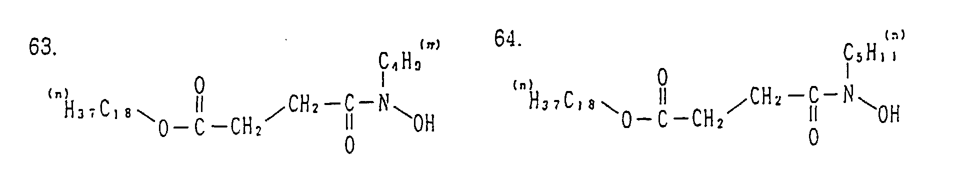

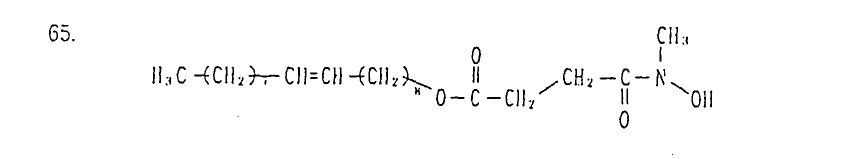

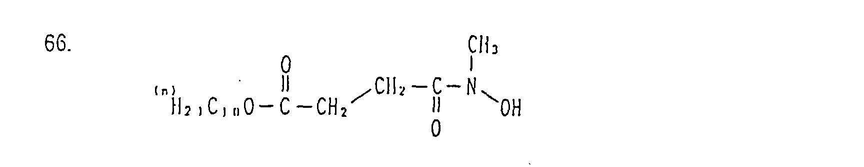

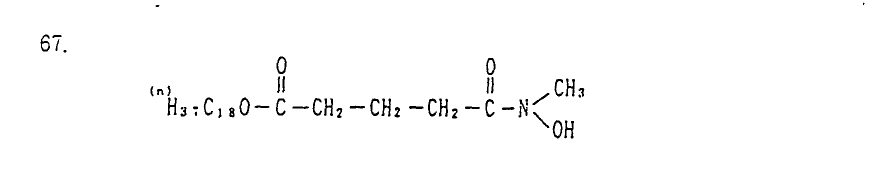

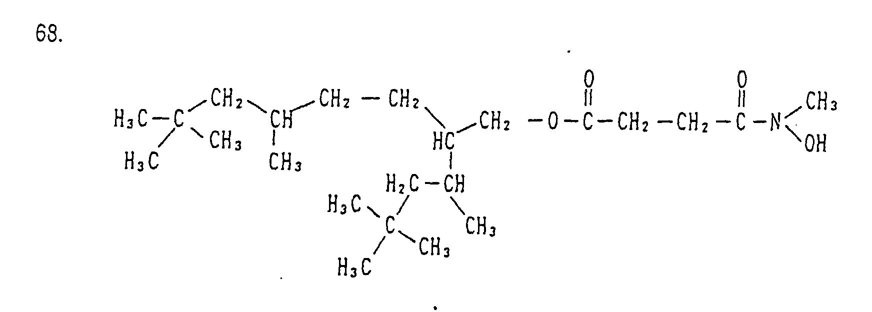

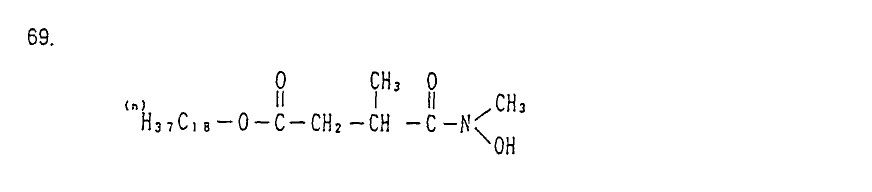

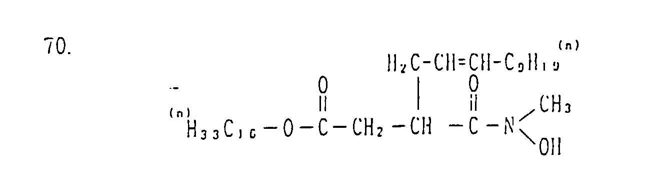

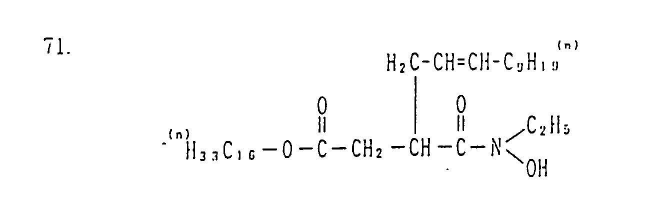

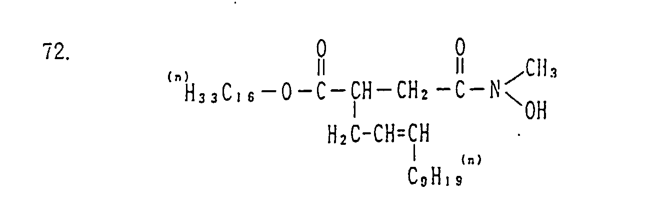

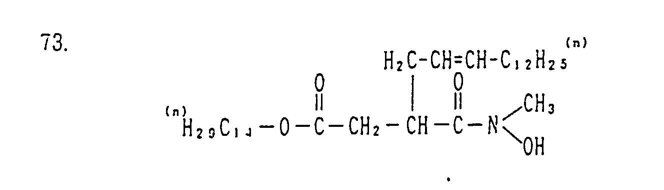

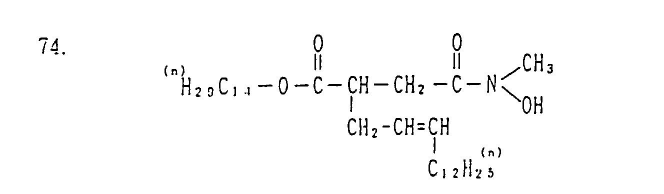

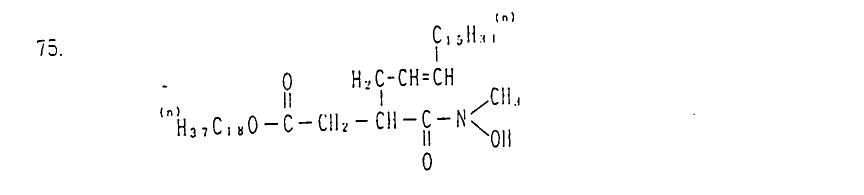

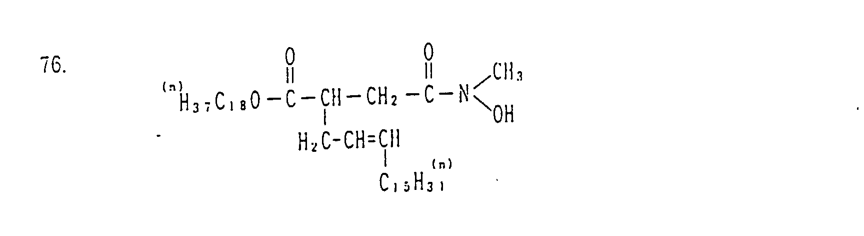

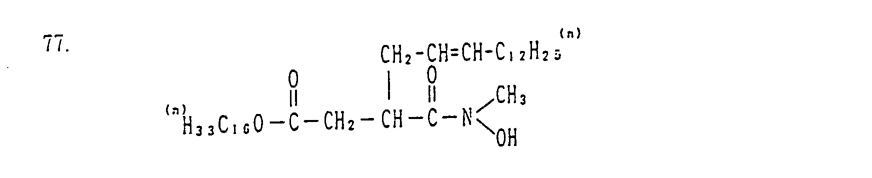

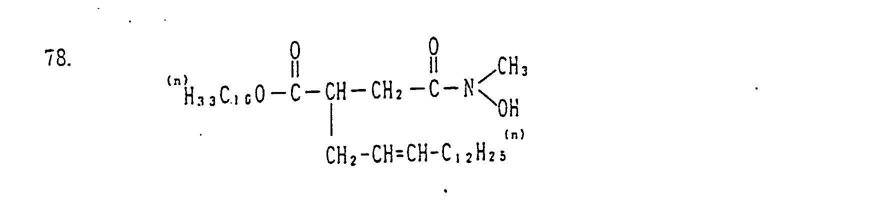

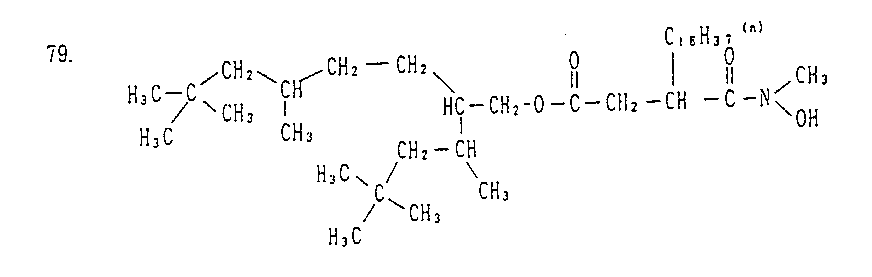

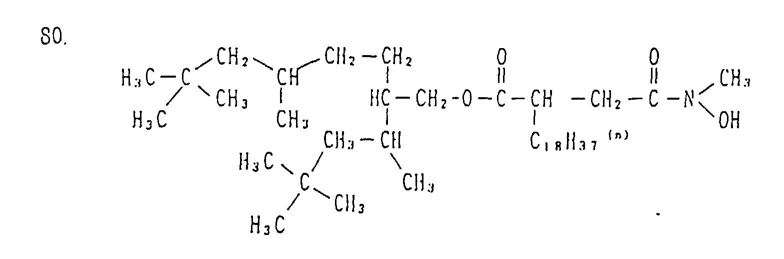

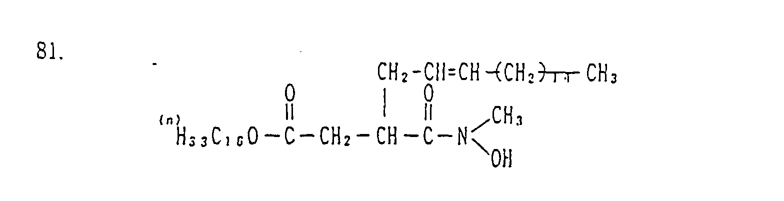

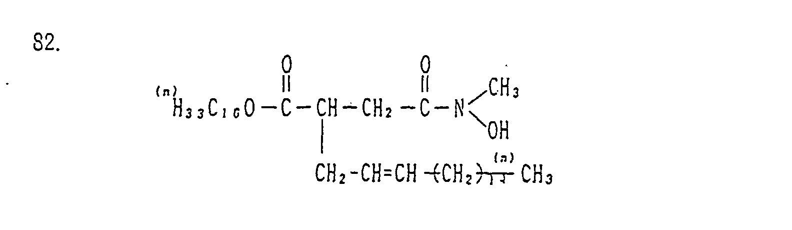

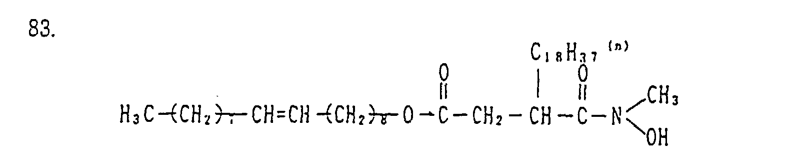

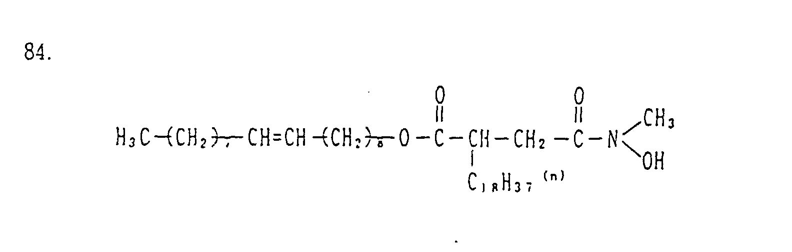

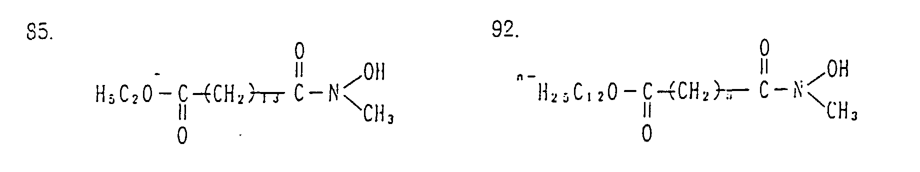

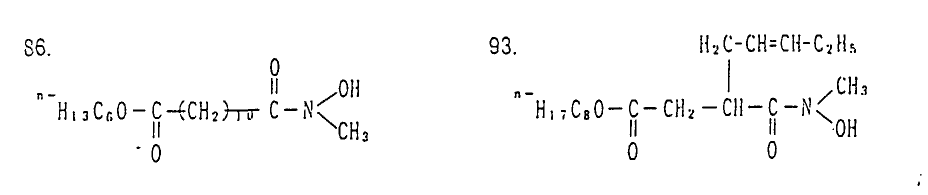

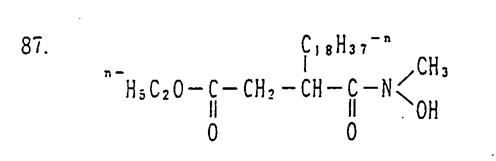

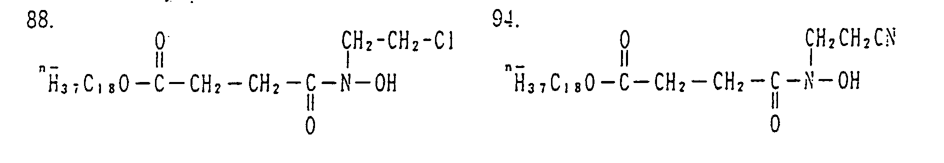

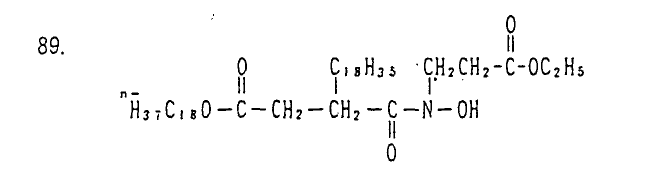

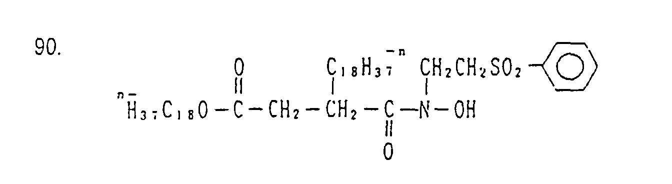

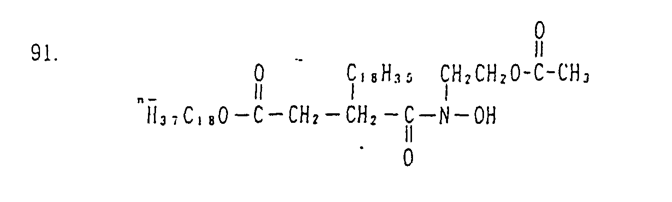

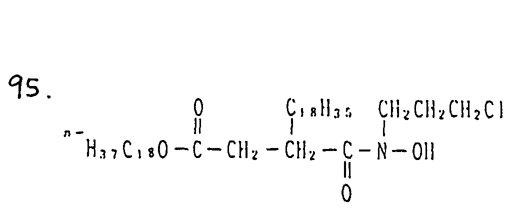

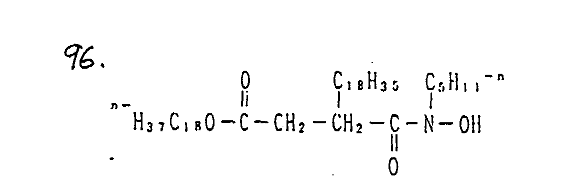

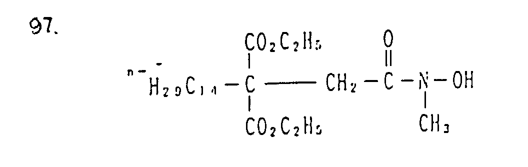

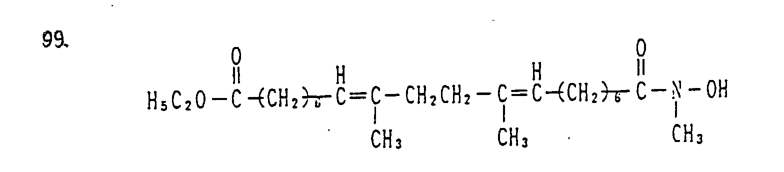

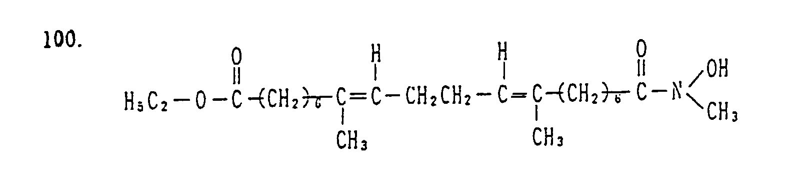

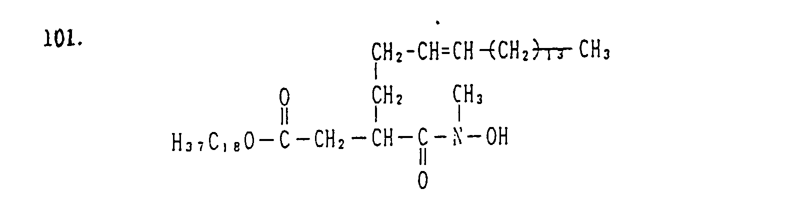

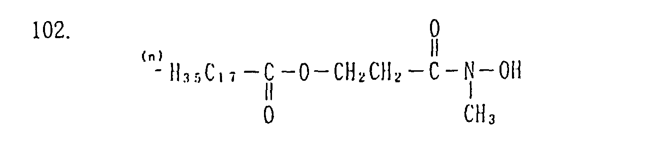

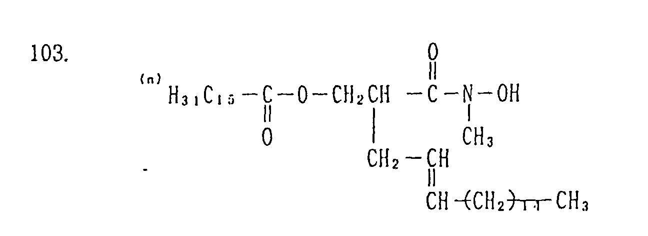

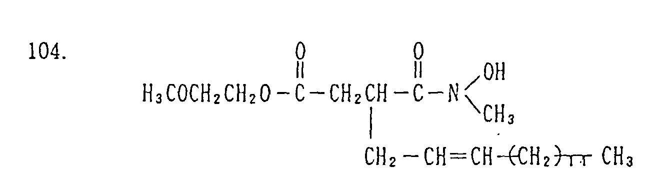

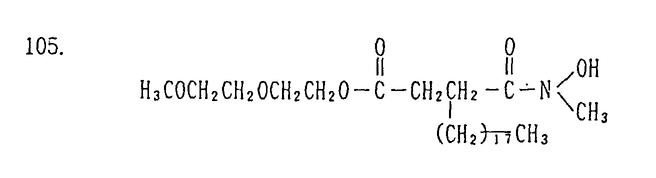

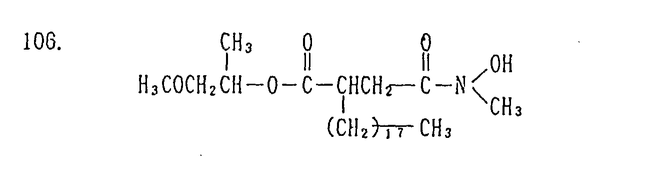

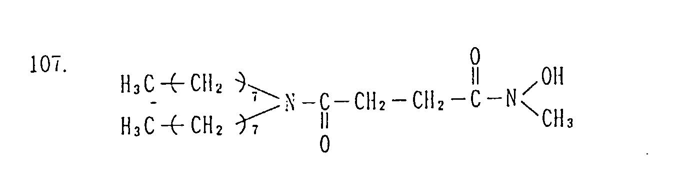

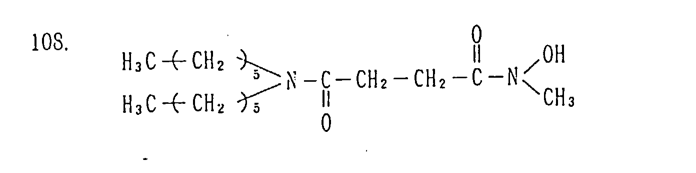

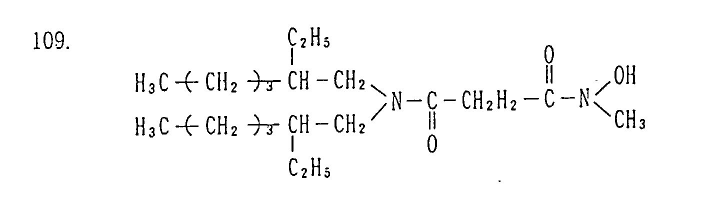

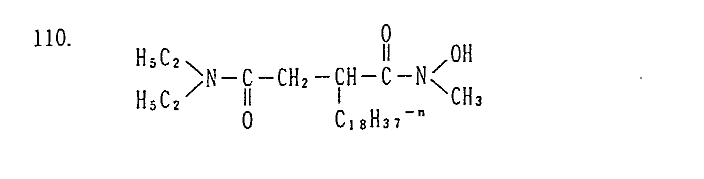

The compounds for use in the present invention can easily be synthesized according to the methods disclosed in JP-A-3-293666, JP-A-59-198453, U.S. Patents 4,330,606 and 4,339,515.

General synthesis methods of the compound of the present invention are described below.

The compound of the present invention can be obtained by condensing the corresponding carboxylic acid chloride and N-alkylhydroxylamine. When the corresponding carboxylic acid is easily available, carboxylic acid chloride can easily be obtained by treating the carboxylic acid with thionyl chloride and and oxalyl chloride. When a carboxylic acid is complicated, after synthesizing a carboxylic acid according to the corresponding synthesis method, carboxylic acid chloride can be obtained by treating the carboxylic acid with thionyl chloride and oxalyl chloride. A carboxylic acid can be synthesized according to synthesis methods described below.

On the other hand, N-alkylhydroxylamine whose alkyl group is a methyl group is commercially available. Others can be synthesized according to the following methods.

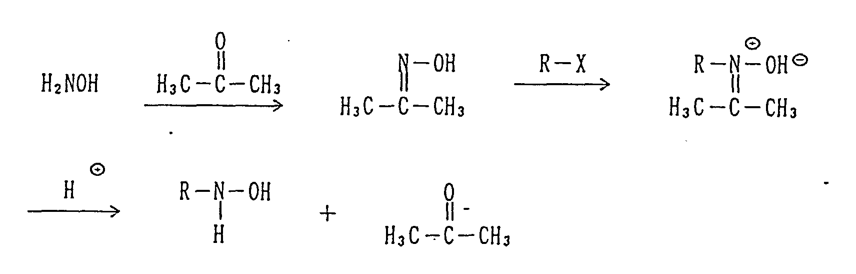

Acetone is added to hydroxylamine and converted to acetoxime, the converted product is reacted with an alkylating agent to synthesize an N-alkyl product (nitron). An acid treatment is conducted thereafter to eliminate the acetone and N-alkylhydroxylamine can be obtained.

The present invention will be further described in detail below by the synthesis examples of the compounds of the present invention.

### SYNTHESIS EXAMPLE 1

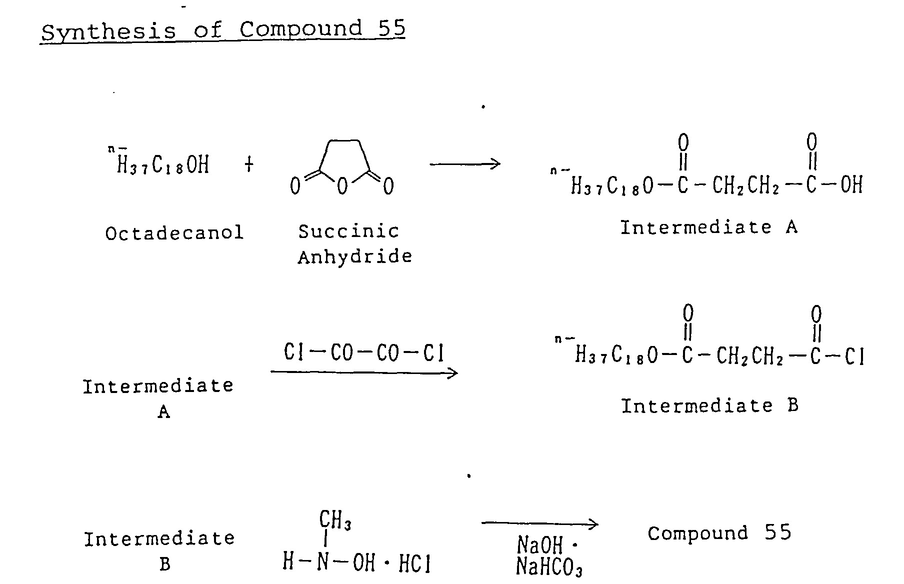

(1) 135 g of n-octadecanol and 50 g of succinic anhydride were put in a three-neck flask having a capacity of 300 ml and allowed to react at 120°C for 6 hours. 50 ml of ethyl acetate was added thereto to crystallize. The obtained crystals were recovered by filtration under reduced pressure and washed with ethyl acetate. The crystals were air-dired and 165 g of Intermediate A was obtained (yield: 89.1%).
(2) 30 g of Intermediate A and 30 ml of methylene chloride were put in a three-neck flask having a capacity of 200 ml and 1 ml of dimethylformamide was added thereto with stirring. 12.3 g of oxalyl chloride was dropwise added thereto over 30 minutes with the inner temperature of the flask maintaining 20°C and the reaction mixture was allowed to react for further 1 hour. The remaining oxalyl chloride and methylene chloride were removed under reduced pressure by an aspirator and Intermediate B was obtained. The obtained Intermediate B was used in the next step as it was.
(3) 2.40 g of sodium hydroxide and 100 ml of water were put in a three-neck flask having a capacity of 1.0 liter and dissolved, then the solution was ice-cooled. Under the nitrogen atmosphere at the inner temperature of 10°C, 5.0 g of N-hydroxylamine hydrochloride was added, subsequently 10.1 g of sodium bicarbonate and 100 ml of ethyl acetate were added thereto. With vigorously stirring and maintaining the inner temperature at 5 to 10°C, a mixture of 25 g of Intermediate B and 200 ml of ethyl acetate was dropwise added over 30 minutes. After the system was allowed to react for 2 hours, the temperature of the reaction solution was raised to 50°C and the solution was separated. The organic layer was washed with water two times and dried with magnesium sulfate. After filtration, the solvent was removed under reduced pressure by an evaporator. The thus obtained crude product was refined with a silica gel column chromatography (ethyl acetate/hexane = 1/10) and 19.1 g of Compound 55 obtained (yield: 79.8%).

### SYNTHESIS EXAMPLE 2

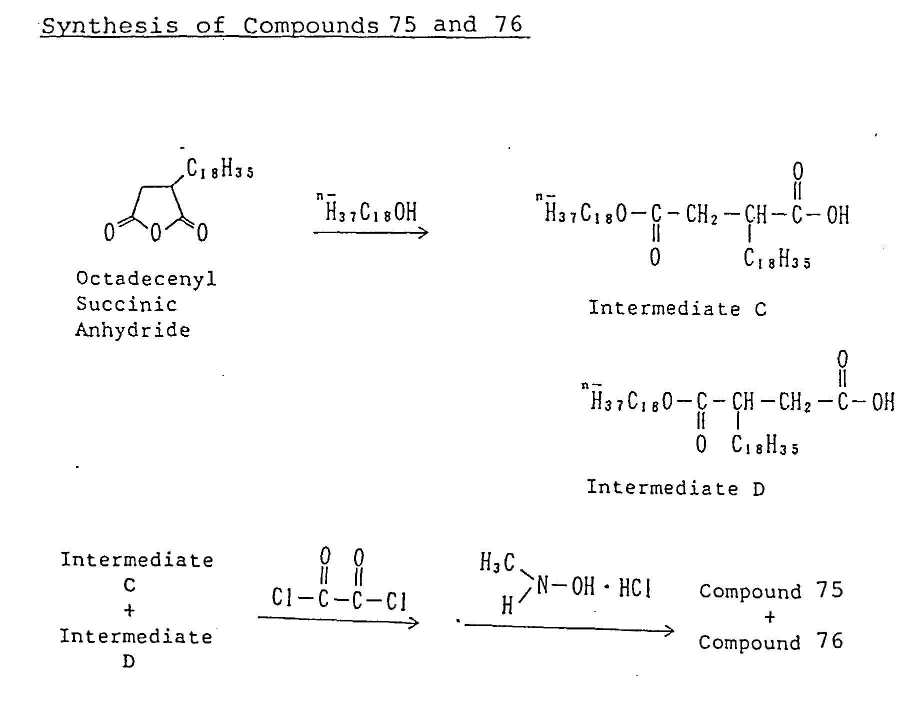

(1) 300 g of octadecenyl succinic anhydride and 232 g of octadecanol were put in a three-neck flask having a capacity of 1.0 liter and allowed to react for 9 hours at 80°C. After the reaction system was cooled to 60°C, 300 ml of acetonitrile was added, and the reaction system was allowed to stand to cool to room temperature to crystallize. 200 ml of acetonitrile was added thereto, and filtrated under reduced pressure. The obtained crystals were thoroughly washed with acetonitrile, and recrystallized with acetonitrile and 511 g of a mixture of Intermediate C and Intermediate D was obtained (yield: 96.0%).
(2) 200 g of the mixture of Intermediates C and D and 200 ml of methylene chloride were put in a three-neck flask having a capacity of 1.0 liter, and then 2.0 ml of dimethylformamide was added. 53.2 g of oxalyl chloride was dropwise added to the mixed solution over 30 minutes at 20°C. The reaction mixture was allowed to stand to react for further 2 hours, then the remaining oxalyl chloride and methylene chloride were removed under reduced pressure by an aspirator and acid chloride of Intermediates C and D was obtained. The obtained acid chloride was used in the next step as it was.
(3) Reaction was conducted in the same manner as in step (3) of Synthesis Example 1 using 29.4 g of N-methyl-hydroxamic acid hydrochloride, 14.1 g of sodium hydroxide, 59.1 g of sodium bicarbonate, the whole amount of the acid chloride of Intermediates C and D obtained in step (2) above, 500 ml of water, and 900 ml of ethyl acetate, and crystals of a mixture of Compounds 25 and 26 were obtained. The crystals were separated with a silica gel column chromatography, thus 70 g of Compound 75 and 25 g of Compound 76 were obtained.

### SYNTHESIS EXAMPLE 3

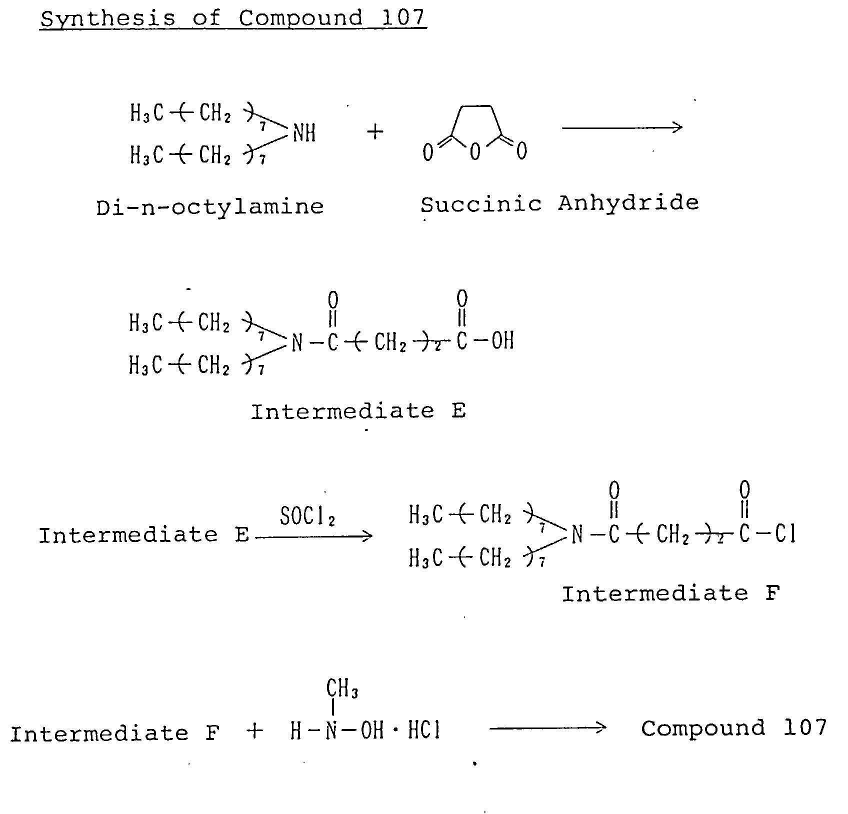

(1) 81 g of di-n-octylamine, 33.6 g of succinic anhydride and 100 ml of acetonitrile were put in a three-neck flask and allowed to react at 40°C for 1 hour. Water was added thereto and the reaction solution was extracted with ethyl acetate. The organic phase was washed with water two times, dried with magnesium sulfate and concentrated to thereby obtaine 106 g of oily Intermediate E. The obtained Intermediate E was used in the next step as it was.
(2) 50 g of Intermediate E, 100 ml of methylene chloride, and 0.2 ml of dimethylformaide were put in a threeneck flask and stirred. 19.2 g of thionyl chloride was dropwise added thereto under room temperature and the reaction mixture was allowed to react for 30 minutes at 40°C. The remaining thionyl chloride and methylene chloride were removed under reduced pressure by an aspirator and 52.7 g of Intermediate F was obtained.
(3) 200 ml of water, 6.1 g of sodium hydroxide, and 12.8 g of N-methylhydroxylamine hydrochloride were stirred under the nitrogen atmosphere. Subsequently, 12.8 g of N-methylhydroxylamine, 25.7 g of sodium hydrogencarbonate, and 200 ml of ethyl acetate were added thereto. 42.7 g of Intermediate F was dissolved in 50 ml of methylene chloride and the solution was dropwise added to the above reaction mixture with ice-cooling. After the system was allowed to react for 30 minutes, the temperature thereof was raised to 40°C and the solution was separated. After the organic phase was washed with water two times, the system was concentrated under reduced pressure and the crude product of Compound 107 was obtained. The thus obtained crude product was refined with a silica gel column chromatography and 39.2 g of refined product was obtained.

All of the above exemplified compounds can easily be synthesized according to the above synthesis method.

Values of physical properties of the representative compounds of the present invention are shown below.

### Compound 58

Appearance: white crystal
Melting Point: 68-69°C
NMR Spectrum: (300 MHz)
δ (CDCl₃): 0.90 (3H, d, J6), 1.27 (22H, bs), 1.61 (2H, d.t, J₁ 12, J₂ 6), 2.61 (2H, bs), 2.70 (2H, bs), 3.24 (1.5H, bs), 3.40 (1.5H, bs), 4.08 (2H, t, J6), 8.10 (1H, bs)

### Compound 75

Appearance: pasty solid
Melting Point: 52°C
NMR Spectrum: (300 MHz)
δ (CDCl₃) 0.90 (6H, t, J6), 1.30 (56H, bs), 1.61 (2H, t, J6), 1.99 (2H, d.d, J₁ 12, J₂ 6), 2.10 (1H, m), 2.32 (1H, m), 2.51 (1H, m), 2.73 (1H, m), 3.00 (1H, bs), 3.23 (1.5H, bs), 3.42 (1.5H, bs), 4.03 (2H, t, J6), 5.30 (1H, m), 5.50 (1H, d.t, J₁ 16, J₂ 6), 8.12 (1H, bs)

### Compound 76

Appearance: white crystal
Melting Point: 68°C
NMR Spectrum: (300 MHz)
δ (CDCl₃): 0.89 (6H, t, J6), 1.27 (56H, bs), 1.62 (2H, t, J6), 1.98 (2H, d.d, J₁ 12, J₂ 6), 2.32 (3H, m), 2.69 (1H, m), 3.01 (1H, bs), 3.21 (1.5H, bs), 3.34 (1.5H, bs), 4.08 (2H, m), 5.30 (1H, d.t, J₁ 16, J₂ 6), 5.49 (1H, d.t, J₁ 16, J₂ 6), 8.10 (1H, bs)

### Compound 81

Appearance: pasty solid
Melting Point: 47°C
NMR Spectrum: (300 MHz)
δ (CDCl₃): 0.90 (6H, t, J6), 1.30 (54H, bs), 1.61 (2H, t, J6), 1.99 (2H, d.d, J₁ 12, J₂ 6), 2.10 (1H, m), 2.32 (1H, m), 2.51 (1H, m), 2.73 (1H, m), 3.00 (1H, bs), 3.23 (1.5H, bs), 3.42 (1.5H, bs), 4.03 (2H, t, J6), 5.30 (1H, m), 5.50 (1H, d.t, J₁ 16, J₂ 6), 8.12 (1H, bs)

### Compound 82

Appearance: white crystal
Melting Point: 65°C
NMR Spectrum: (300 MHz)
δ (CDCl₃): 0.90 (6H, t, J6), 1.30 (54H, bs), 1.61 (2H, t, J6), 1.99 (2H, d.d, J₁ 12, J₂ 6), 2.10 (1H, m), 2.32 (1H, m), 2.51 (1H, m), 2.73 (1H, m), 3.00 (1H, bs), 3.23 (1.5H, bs), 3.42 (1.5H, bs), 4.03 (2H, t, J6), 5.30 (1H, m), 5.50 (1H, d.t, J₁ 16, J₂ 6), 8.12 (1H, bs)

Structures of other compounds were also confirmed by NMR spectrum or mass spectrum.

Compounds 68, 70, 71, 72, 73, 74, 77, 78, 79, 80, 81 and 88 were oily products.

The addition amount of the compound of the present invention is not particularly limited, but when added to a light-sensitive silver halide emulsion layer, the amount is preferably from 1.0 × 10⁻⁴ to 1.0 × 10⁻¹ mol, more preferably from 1.0 × 10⁻³ to 5.0 × 10⁻² mol, per mol of Ag in the same layer. When the compound is added to a light-insensitive layer, preferably from 1 × 10⁻⁶ to 3 × 10⁻⁴ mol/m², more preferably from 1 × 10⁻⁵ to 1 × 10⁻⁴ mol/m².

The compound of the present invention may be dissolved in a water-soluble solvent (e.g., methanol, ethanol, acetone) and added, may be added in the form of an emulsified dispersion with couplers and the like, or may be added at the time of the preparation of the emulsion, but the addition in the form of an emulsified dispersion is most preferred.

There is no particular limitation on the layers to which the compound of the present invention is added but the compound is preferably added to a silver halide emulsion layer, and is more preferably added to a red-sensitive layer and/or a green-sensitive layer.

The silver halide photographic material of the present invention can be used as a color photographic material for general use and for movie such as a color-negative film, a reversal film, a color negative film for movie, a color positive film, and a positive film for movie, and as a black-and-white photographic material such as a black-and-white negative film, a microfilm, an X-ray film, but it is preferably used as a general color and a black-and-white photographic materials for photographing.

When the present invention is applied to a color photographic material, the material can comprise at least one light-sensitive layer on a support. In a typical embodiment, the silver halide photographic material of the present invention comprises at least one light-sensitive layer consisting of a plurality of silver halide emulsion layers having substantially the same color sensitivity but different degrees of sensitivity on a support. The light-sensitive layer is a unit light-sensitive layer having a color sensitivity to any of blue light, green light and red light. In the multilayer silver halide color photographic material, these unit light-sensitive layers are generally arranged in the order of red-sensitive layer, green-sensitive layer and blue-sensitive layer from the support side. However, the order of arrangement can be reversed depending on the purpose, alternatively, the light-sensitive layers may be arranged in such a way that a layer having a different color sensitivity is interposed between layers having the same color sensitivity. Light-insensitive layers may be provided between the above described silver halide light-sensitive layers, and on the uppermost layer and beneath the lowermost layer of the silver halide light-sensitive layers. These light-insensitive layers can contain couplers, DIR compounds, color mixing preventives described below and compounds which release a dye imagewise or reversely imagewise to provide a difference in diffusibility between a released dye and a compound before releasing. As the plurality of silver halide emulsion layers constituting each unit light-sensitive layer, a two-layer structure of a high sensitivity emulsion layer and a low sensitivity emulsion layer can be preferably used as disclosed in German Patent 1,121,470 and British Patent 923,045. It is usually preferred that the emulsion layers are arranged so as to decrease in sensitivity toward a support in this order. Further, a low sensitivity emulsion layer may be provided farther from the support and a high sensitivity emulsion layer may be provided nearer to the support as disclosed in JP-A-57-112751, JP-A-62-200350, JP-A-62-206541, and JP-A-62-206543.

In one specific example, a low sensitivity blue-sensitive layer (BL)/a high sensitivity blue-sensitive layer (BH)/a high sensitivity green-sensitive layer (GH)/a low sensitivity green-sensitive layer (GL)/a high sensitivity red-sensitive layer (RH)/a low sensitivity red-sensitive layer (RL), or BH/BL/GL/GH/RH/RL, or BH/BL/GH/GL/RL/RH can be arranged in this order from the side farthest from the support.

A blue-sensitive layer/GH/RH/GL/RL can be arranged in this order from the side farthest from the support as disclosed in JP-B-55-34932 (the term "JP-B" as used herein means an "examined Japanese patent publication"). Further, a blue-sensitive layer/GL/RL/GH/RH can be arranged in this order from the side farthest from the support as disclosed in JP-A-56-25738 and JP-A-62-63936.

Further, useful arrangements include the arrangement in which there are three layers having different degrees of sensitivities with the sensitivity being lower towards the support such that the uppermost layer is a silver halide emulsion layer having the highest sensitivity, the middle layer is a silver halide emulsion layer having a lower sensitivity than that of the uppermost layer, and the lowermost layer is a silver halide emulsion layer having a lower sensitivity than that of the middle layer, as disclosed in JP-B-49-15495. In the case of the structure of this type comprising three layers having different degrees of sensitivity, the layers in the unit layer of the same color sensitivity may be arranged in the order of a middle sensitivity emulsion layer/a high sensitivity emulsion layer/a low sensitivity emulsion layer, from the side farthest from the support, as disclosed in JP-A-59-202464.

Alternatively, the layers can be arranged in the order of a high sensitivity emulsion layer/a low sensitivity emulsion layer/a middle sensitivity emulsion layer, or a low sensitivity emulsion layer/a middle sensitivity emulsion layer/a high sensitivity emulsion layer.

Moreover, the arrangement may be varied as indicated above in the case where there are four or more layers.

A donor layer (CL) having an interlayer effect and a different spectral sensitivity distribution from a main light-sensitive layer such as BL, GL and RL may preferably be provided adjacent or close to the main light-sensitive layer to improve color reproducibility, as disclosed in U.S. Patents 4,663,271, 4,705,744, 4,707,436, JP-A-62-160448 and JP-A-63-89850.

The preferred silver halides for use in the present invention are silver iodobromide, silver iodochloride or silver iodochlorobromide containing about 30 mol% or less of silver iodide, and particularly preferably silver iodobromide or silver iodochlorobromide containing from about 2 mol% to about 10 mol% of silver iodide.

The silver halide grains in the photographic emulsion may have a regular crystal form such as a cubic, octahedral or tetradecahedral form, an irregular crystal form such as a spherical or plate form, a form which has crystal defects such as twin crystal planes, or a form which is a composite of these forms.

The silver halide grains may be a fine grain having a grain size of about 0.2 µm or less, or a large grain size having a projected area diameter of up to about 10 µm, and the emulsion may be a polydisperse emulsion or a monodisperse emulsion.

The silver halide photographic emulsions for use in the present invention can be prepared using the methods disclosed, for example, in Research Disclosure (hereinafter abbreviated to RD), No. 17643 (December, 1978), pages 22 and 23, "I. Emulsion Preparation and Types", RD, No. 18716 (November, 1979), page 648, RD, No. 307105 (November, 1989), pages 863 to 865, P. Glafkides, Chimie et Physique Photographique, Paul Montel (1967), G.F. Duffin, Photographic Emulsion Chemistry, Focal Press (1966), and V.L. Zelikman, et al., Making and Coating Photographic Emulsion, Focal Press (1964).

The monodisperse emulsions disclosed in U.S. patents 3,574,628, 3,655,394 and British Patent 1,413,748 are also preferred.

Further, tabular grains having an aspect ratio of about 3 or more can be used in the present invention. Tabular grains can be easily prepared according to the methods disclosed, for example, in Gutoff, Photographic Science and Engineering, Vol. 14, pages 248 to 257 (1970), U.S. Patents 4,434,226, 4,414,310, 4,433,048, 4,439,520 and British Patent 2,112,157.

The crystal structure may be uniform, or the interior and exterior parts of the grains may comprise different halogen compositions, or the grains may have a layered structure. Silver halides which have different compositions may be joined with an epitaxial junction or may be joined with compounds other than a silver halide, such as silver thiocyanate or lead oxide. Further, mixtures of grains which have various crystal forms may also be used.

The above described emulsions may be of the surface latent image type wherein the latent image is primarily formed on the surface, or of the internal latent image type wherein the latent image is formed within the grains, or of a type wherein the latent image is formed both at the surface and within the grains. The emulsion may be a negative type or a positive type (so called auto-positive emulsion). The negative type emulsion may be conventional emulsion or heat-developable emulsion. Of the internal latent image types, the emulsion may be a core/shell type internal latent image type emulsion as disclosed in JP-A-63-264740, and a method for preparation of such a core/shell type internal latent image type emulsion is disclosed in JP-A-59-133542. The thickness of the shell of this emulsion varies depending on the development process and the like, but is preferably from 3 to 40 nm, and particularly preferably from 5 to 20 nm.

The silver halide emulsion for use in the present invention is generally subjected to physical ripening, chemical ripening and spectral sensitization. Additives for use in such processes are disclosed in RD, No. 17643, RD, No. 18716, and RD, No. 307105, and the locations of these disclosures are summarized in a table below.

Two or more different types of emulsions which are different in terms of at least one of the characteristics of grain size, grain size distribution, halogen composition, the form of the grains and the sensitivity of the light-sensitive silver halide emulsion can be used in admixture in the same layer in the photographic material of the present invention.

It is preferred to use the silver halide grains having a fogged grain surface as disclosed in U.S. Patent 4,082,553, the silver halide grains having a fogged grain interior as disclosed in U.S. Patent 4,626,498 and JP-A-59-214852, or colloidal silver in light-sensitive silver halide emulsion layers and/or substantially light-insensitive hydrophilic colloid layers. Silver halide grains having a fogged grain interior or surface are silver halide grains which can be developed uniformly (not imagewise) irrespective of whether these grains are present in an unexposed part or an exposed part of the photographic material, and methods for the preparation of such grains are disclosed in U.S. Patent 4,626,498 and JP-A-59-214852. The silver halide which forms the internal nuclei of a core/shell type silver halide grains having a fogged grain interior may have different halogen compositions. The silver halide having a fogged grain interior or surface may be any of silver chloride, silver chlorobromide, silver iodobromide, or silver chloroiodobromide. The average grain size of these fogged silver halide grains is preferably from 0.01 to 0.75 µm, and particularly preferably from 0.05 to 0.6 µm. Further, the form of the grains may be regular grains and may be a polydisperse emulsion, but a monodisperse emulsion (at least 95% of which have a grain size within ±40% of the average grain size in terms of the weight or number of silver halide grains) is preferred.

The use of light-insensitive fine grained silver halides is preferred in the present invention. Light-insensitive fine grained silver halides are fine grained silver halides which are not sensitive to light upon imagewise exposure for obtaining color images and which do not substantially undergo development during development process, and they are preferably not pre-fogged. The fine grained silver halide has a silver bromide content of from 0% to 100 mol%, and may contain silver chloride and/or silver iodide, if necessary. The fine grained silver halides which have a silver iodide content of from 0.5 to 10 mol% are preferred. The average grain size of the fine grained silver halides (the average value of the diameters of the circles corresponding to the projected areas) is preferably from 0.01 to 0.5 µm, and more preferably from 0.02 to 0.2 µm.

The fine grained silver halide can be prepared by the same methods as generally used for the preparation of light-sensitive silver halides. In the preparation of the fine grained silver halide, the surface of the silver halide grains does not need to be optically sensitized and also there is no need for spectral sensitization. However, it is preferred to previously incorporate known stabilizers such as triazole based, azaindene based, benzothiazolium based, or mercapto based compounds, or zinc compounds in the fine grained silver halide before addition to the coating solution. Colloidal silver can be included in the layer containing the fine grained silver halide grains.

The coating weight of silver in the photographic material of the present invention is preferably 6.0 g/m² or less, and most preferably 4.5 g/m² or less.

Photographic additives which can be used in the present invention are disclosed in RD and the locations related thereto are indicated in the table below.

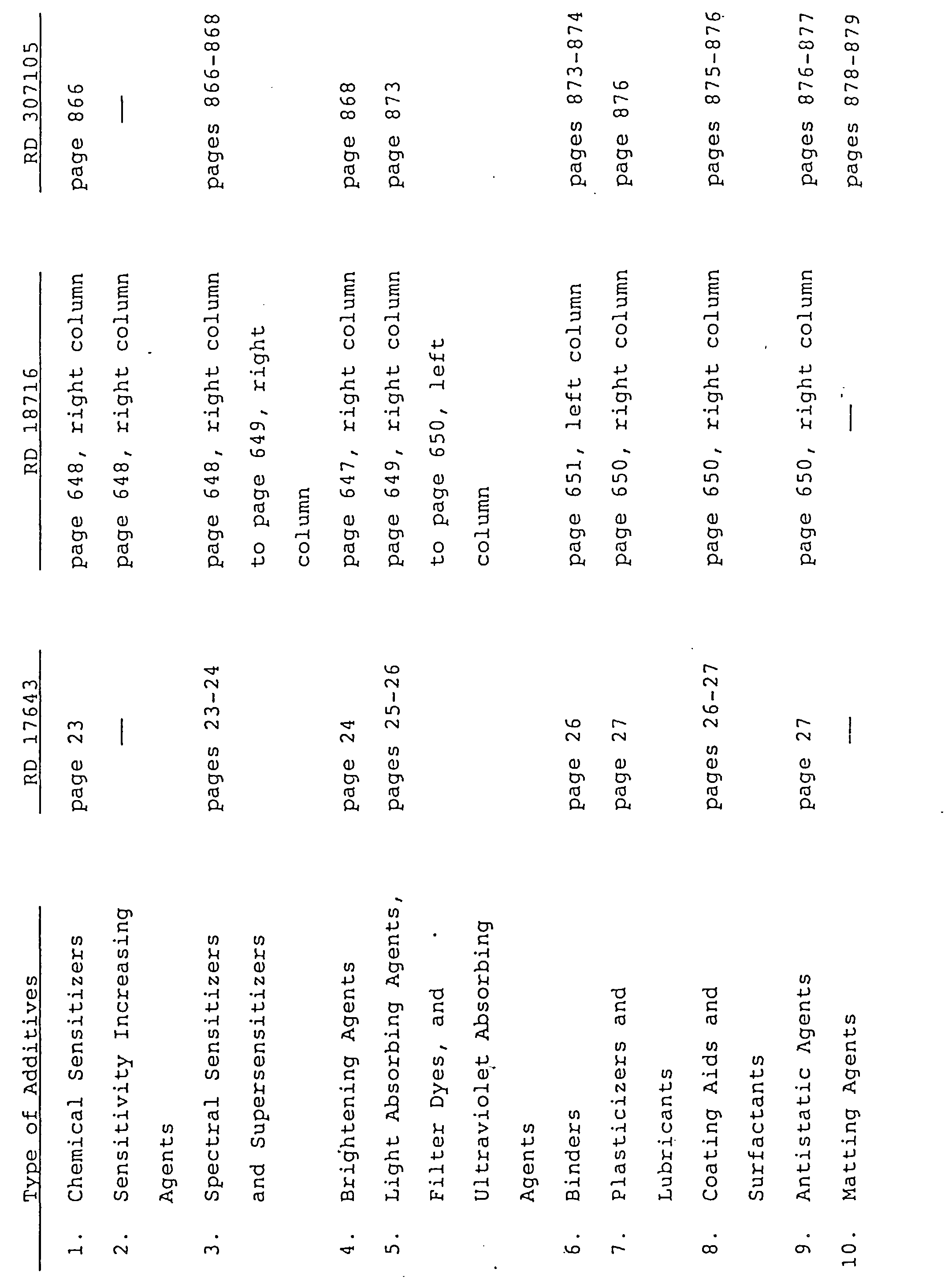

Various dye-forming couplers can be used in the present invention, and the following couplers are particularly preferred.

### Yellow Couplers:

The couplers represented by formulae (I) and (II) disclosed in EP 502424A; the couplers represented by formulae (1) and (2) disclosed in EP 513496A (particularly Coupler Y-28, page 18); the couplers represented by formula (I) disclosed in claim 1 of Japanese Patent Application No. 4-134523; the couplers represented by formula (I), lines 45 to 55, column 1 of U.S. Patent 5,066,576; the couplers represented by formula (I), column 0008 of JP-A-4-274425; the couplers disclosed in claim 1 on page 40 of EP 498381A1 (particularly D-35, page 18); the couplers represented by formula (Y) on page 4 of EP 447969A1 (particularly Y-1 (page 17) and Y-54 (page 41)); and the couplers represented by formulae (II) to (IV), lines 36 to 58, column 7 of U.S. Patent 4,476,219 (particularly II-17 and II-19 (column 17), and II-24 (column 19)).

### Magenta Couplers:

L-57 (page 11, right lower column), L-68 (page 12, right lower column), and L-77 (page 13, right lower column) disclosed in JP-A-3-39737; [A-4]-63 (page 134), and [A-4]-73 and [A-4]-75 (page 139) disclosed in EP 456257; M-4 and M-6 (page 26) and M-7 (page 27) disclosed in EP 486965; M-45, column 0024 of Japanese Patent Application No. 4-234120; M-1, column 0036 of Japanese Patent Application No. 4-36917; and M-22, column 0237 of JP-A-4-362631.

### Cyan Couplers:

CX-1, -3, -4, -5, -11, -12, -14 and -15 (pages 14 to 16) disclosed in JP-A-4-204843; C-7 and C-10 (page 35), C-34 and C-35 (page 37), (I-1) and (I-17) (pages 42 and 43) disclosed in JP-A-4-43345; and the couplers represented by formula (Ia) or (Ib) disclosed in claim 1 of Japanese Patent Application No. 4-236333.

### Polymer Couplers:

P-1 and P-5 (page 11) disclosed in JP-A-2-44345.

The couplers disclosed in U.S. Patent 4,366,237, British Patent 2,125,570, EP 96873B and German Patent 3,234,533 are preferred as couplers the colored dyes of which have an appropriate diffusibility.

Preferred couplers for correcting the unnecessary absorption of colored dyes include the yellow colored cyan couplers represented by formulae (CI), (CII), (CIII) and (CIV) disclosed on page 5 of EP 456257A1 (particularly YC-86, page 84); the yellow colored magenta couplers ExM-7 (page 202), EX-1 (page 249), and EX-7 (page 251) disclosed in EP 456257A1; the magenta colored cyan couplers CC-9 (column 8) and CC-13 (column 10) disclosed in U.S. Patent 4,833,069; the coupler (2) (column 8) disclosed in U.S. Patent 4,837,136; and the colorless masking couplers represented by formula (A) disclosed in claim 1 of WO 92/11575 (particularly the compounds on pages 36 to 45).

Examples of compounds (including couplers) which release photographically useful residual groups of compounds upon reacting with the oxidation product of a developing agent include the following: development inhibitor releasing compounds: the compounds represented by formulae (I), (II), (III) and (IV) disclosed on page 11 of EP 378236A1 (particularly T-101 (page 30), T-104 (page 31), T-113 (page 36), T-131 (page 45), T-144 (page 51) and T-158 (page 58)), the compounds represented by formula (I) disclosed on page 7 of EP 436938A2 (particularly D-49 (page 51)), the compounds represented by formula (1) disclosed in Japanese Patent Application No. 4-134523 (particularly (23), column 0027), and the compounds represented by formulae (I), (II) and (III) disclosed on pages 5 and 6 of EP 440195A2 (particularly I-(1), page 29); bleaching accelerator releasing compounds: the compounds represented by formulae (I) and (I') disclosed on page 5 of EP 310125A2 (particularly (60) and (61), page 61), and the compounds represented by formula (I) disclosed in claim 1 of Japanese Patent Application No. 4-325564 (particularly (7), column 0022); ligand releasing compounds: the compounds represented by LIG-X disclosed in claim 1 of U.S. Patent 4,555,478 (particularly the compounds in lines 21 to 41, column 12); leuco dye releasing compounds: compounds 1 to 6, columns 3 to 8 of U.S. Patent 4,749,641; fluorescent dye releasing compounds: the compounds represented by COUP-DYE disclosed in claim 1 of U.S. Patent 4,774,181 (particularly compounds 1 to 11, columns 7 to 10); development accelerator or fogging agent releasing compounds: the compounds represented by formulae (1), (2) and (3) disclosed in U.S. Patent 4,656,123 (particularly (I-22), column 25), and ExZK-2, lines 36 to 38, page 75 of EP 450637A2; and compounds which release dyes the color of which is restored after being released: the compounds represented by formula (I) disclosed in claim 1 of U.S. Patent 4,857,447 (particularly Y-1 to Y-19, columns 25 to 36).

Preferred additives other than couplers are listed below.

Dispersion mediums of oil-soluble organic compound: P-3, -5, -16, -19, -25, -30, -42, -49, -54, -55, -66, -81, -85, -86 and -93 (pages 140 to 144) of JP-A-62-215272; latexes for impregnation of oil-soluble organic compound: the latexes disclosed in U.S. Patent 4,199,363; scavengers for the oxidation product of a developing agent: the compounds represented by formula (I), lines 54 to 62, column 2 of U.S. patent 4,978,606 (particularly I-(1), -(2), -(6) and -(12) (columns 4 and 5)), and the compounds represented by the formulae disclosed in lines 5 to 10, column 2 of U.S. Patent 4,923,787 (particularly compound 1 (column 3)); stain inhibitors: the compounds represented by formulae (I) to (III), lines 30 to 33, page 4 of EP 298321A (particularly I-47, -72, III-1, -27 (pages 24 to 48)); discoloration inhibitors: A-6, -7, -20, -21, -23, -24, -25, -26, -30, -37, -40, -42, -48, -63, -90, -92, -94 and -164 (pages 69 to 118) disclosed in EP 298321A, II-1 to III-23, columns 25 to 38 of U.S. Patent 5,122,444 (particularly III-10), I-1 to III-4, pages 8 to 12 of EP 471347A (particularly II-2), and A-1 to -48, columns 32 to 40 of U.S. Patent 5,139,931 (particularly A-39 and -42); compounds for reducing the using amount of supersensitizers or color mixing preventives: I-1 to II-15, pages 5 to 24 of EP 411324A (particularly I-46); formalin scavengers: SCV-1 to -28, pages 24 to 29 of EP 477932A (particularly SCV-8); hardening agents: H-1, -4, -6, -8 and -14 on page 17 of JP-A-1-214845, the compounds represented by formulae (VII) to (XII) (H-1 to -54), columns 13 to 23 of U.S. Patent 4,618,573, the compounds represented by formula (6), right lower column, page 8 of JP-A-2-214852 (H-1 to -76) (particularly H-14), and the compounds disclosed in claim 1 of U.S. Patent 3,325,287; development inhibitor precursors: P-24, -37 and -39, pages 6 and 7 of JP-A-62-168139, the compounds disclosed in claim 1 of U.S. Patent 5,019,492 (particularly compounds 28 and 29, column 7); biocides and fungicides: I-1 to III-43, columns 3 to 15 of U.S. Patent 4,923,790 (particularly compounds II-1, -9, -10, -18 and III-25); stabilizers and antifoggants: I-1 to (14), columns 6 to 16 of U.S. Patent 4,923,793 (particularly I-1, -60, (2), and (13)), and compounds 1 to 65, columns 25 to 32 of U.S. Patent 4,952,483 (particularly compound 36); chemical sensitizers: triphenylphosphine selenide, and compound 50 disclosed in JP-A-5-40324; dyes: a-1 to b-20, pages 15 to 18 (particularly a-1, -12, -18, -27, -35, -36, and b-5), V-1 to -23, pages 27 to 29 (particularly V-1) of JP-A-3-156450, F-I-1 to F-II-43, pages 33 to 55 of EP 445627A (particularly F-I-11 and F-II-8), III-1 to -36, pages 17 to 28 of EP 457153A (particularly III-1 and -3), crystallite dispersions of Dye-1 to -124, pages 8 to 26 of WO 88/04794, compounds 1 to 22, pages 6 to 11 of EP 319999A (particularly compound 1), compounds D-1 to -87 represented by formulae (1) to (3), pages 3 to 28 of EP 519306A, compounds 1 to 22 represented by formula (I), columns 3 to 10 of U.S. Patent 4,268,622, and compounds (1) to (31) represented by formula (I), columns 2 to 9 of U.S. Patent 4,923,788; ultraviolet absorbing agents: compounds (18b) to (18r) represented by formula (1), 101 to 427, pages 6 to 9 of JP-A-46-3335, compounds (3) to (66) represented by formula (I), pages 10 to 44, and compounds HBT-1 to -10 represented by formula (III), page 14 of EP 520938A, and compounds (1) to (31) represented by formula (1), columns 2 to 9 of EP 521823A.

The present invention can be applied to various color photographic materials such as color negative films for general and movie uses, color reversal films for slides and television uses, color papers, color positive films and color reversal papers. The present invention can also preferably be applied to the film units equipped with lenses as disclosed in JP-B-2-32615 and JP-B-U-3-39784 (the term "JP-B-U" as used herein means an "examined Japanese utility model publication). Further, the present invention is applicable to a color photography by a diffusion transfer process, a diffusion transfer photography using auto-positive emulsion, and a wet-type color reversal copying material using auto-positive emulsion.

Suitable supports which can be used in the present invention are disclosed, for example, in RD, No. 17643, page 28, RD, No. 18716, from page 647, right column to page 648, left column, and RD, No. 307105, page 879.

The photographic material of the present invention has a total film thickness of all the hydrophilic colloid layers on the side where the silver halide emulsion layers are located of preferably 28 µm or less, more preferably 23 µm or less, still more preferably 18 µm or less, and most preferably 16 µm or less. Further, the film swelling rate T_{½} is preferably 30 seconds or less, and more preferably 20 seconds or less. T_{½} is defined as the time taken to reach ½ of the saturated film thickness, taking 90% of the maximum swollen film thickness reached when being processed in a color developing solution at 30°C for 3 minutes and 15 seconds as the saturated film thickness. The film thickness means the film thickness measured under conditions of 25°C, 55% relative humidity (stored for 2 days), and T_{½} can be measured using a swellometer of the type described in A. Green, et al., Photogr. Sci. Eng., Vol. 19, No. 2, pages 124 to 129. T_{½} can be adjusted by adding hardening agents to gelatin which is used as a binder, or by changing the aging conditions after coating. Further, a swelling factor of from 150% to 400% is preferred. The swelling factor can be calculated from the maximum swollen film thickness obtained under the conditions described above using the equation: (maximum swollen film thickness - film thickness)/film thickness.

The provision of hydrophilic colloid layers (known as backing layers) having a total dry film thickness of from 2 µm to 20 µm on the side of the support opposite to the side on which emulsion layers are provided is preferred in the photographic material of the present invention. The inclusion of the above described light absorbing agents, filter dyes, ultraviolet absorbing agents, antistatic agents, hardening agents, binders, plasticizers, lubricants, coating aids, and surfactants in the backing layers is preferred. The swelling factor of the backing layer is preferably from 150 to 500%.

The photographic material of the present invention can be development processed by the general methods disclosed in RD, No. 17643, pages 28 and 29, RD, No. 18716, page 651, from left column to right column, and RD, No. 307105, pages 880 and 881.

The color developing solution for use in the development processing of the photographic material of the present invention is preferably an alkaline aqueous solution which contains an aromatic primary amine developing agent as a main component. Aminophenol based compounds are useful as a color developing agent, but the use of p-phenylenediamine based compounds is preferred, and representative examples thereof include the compounds disclosed in lines 43 to 52, page 28 of EP 556700A. Two or more of these compounds can be used in combination according to purposes.

The color developing solution generally contains a pH buffer such as alkali metal carbonate, borate or phosphate, or a development inhibitor or an antifoggant such as chloride, bromide, iodide, benzimidazoles, benzothiazoles, or mercapto compounds. The color developing solution may also contain, if necessary, various preservatives such as hydroxylamine, diethylhydroxylamine, sulfite, hydrazines, e.g., N,N-biscarboxymethylhydrazine, phenylsemicarbazides, triethanolamine and catecholsulfonic acids, an organic solvent such as ethylene glycol and diethylene glycol, a development accelerator such as benzyl alcohol, polyethylene glycol, quaternary ammonium salt, and amines, a dye-forming coupler, a competitive coupler, an auxiliary developing agent such as 1-phenyl-3-pyrazolidone, a thickener, and various chelating agents typified by aminopolycarboxylic acid, aminopolyphosphonic acid, alkylphosphonic acid, and phosphonocarboxylic acid, e.g., ethylenediaminetetraacetic acid, nitrilotriacetic acid, diethylenetriaminepentaacetic acid, cyclohexanediaminetetraacetic acid, hydroxyethyliminodiacetic acid, 1-hydroxyethylidene-1,1-diphosphonic acid, nitrilo-N,N,N-trimethylenephosphonic acid, ethylenediamine-N,N,N,N-tetramethylenephosphonic acid, ethylenediamine-di(o-hydroxyphenylacetic acid) and salts of these acids.

Further, the color development is generally carried out after the black-and-white development in the case of reversal processing. Known black-and-white developing agents such as hydroxybenzenes, e.g., hydroquinone, 3-pyrazolidones, e.g., 1-phenyl-3-pyrazolidone, or aminophenols, e.g., N-methyl-p-aminophenol can be used alone or in combination in the black-and-white developing solution. The pH of these color developing solution and black-and-white developing solution is generally from 9 to 12. The replenishment rate of these developing solutions depends on the color photographic material to be processed but, in general, it is 3 liters or less per square meter of the photographic material, and can be reduced to 500 ml or less by reducing the bromide ion concentration in the replenisher. When the replenishment rate is reduced, it is preferred to prevent evaporation and air oxidation of the solution by minimizing the area of contact of the solution with the air in the processing tank.

The processing effect by the contact of the photographic processing solution with the air in a processing tank can be evaluated by the following equation: Open factor = [Contact area of processing solution with air (cm²)] ÷ [Volume of processing solution (cm³)]. This open factor is preferably 0.1 or less, and more preferably from 0.001 to 0.05. The method using a movable lid as disclosed in JP-A-1-82033 and the slit development processing method as disclosed in JP-A-63-216050 can be used as means of reducing the open factor, as well as the provision of a shielding material such as a floating lid on the surface of the photographic processing solution in the processing tank. Reduction of the open factor is preferred not only in the processes of the color development and the black-and-white development but also in all the subsequent processes such as the bleaching process, the bleach-fixing process, the fixing process, the washing process and the stabilizing process. Further, the replenishment rate can be reduced by suppressing the accumulation of the bromide ion in the developing solution.

The color development processing time is usually set between 2 and 5 minutes, but shorter processing time can be used by raising the temperature and the pH and increasing the concentration of the color developing agent.

The photographic emulsion layer is generally subjected to a bleaching process after color development. A bleaching process may be carried out simultaneously with a fixing process (a bleach-fixing process) or may be carried out separately. In addition, a bleach-fixing process can be carried out after a bleaching process to speed up the processing. Moreover, the processing can be carried out in two connected bleach-fixing baths, a fixing process can be carried out before a bleach-fixing process, or a bleaching process can be carried out after a bleach-fixing process, as required. Compounds of multivalent metals such as iron(III), peracids, quinones, and nitro compounds can be used as a bleaching agent. Representative bleaching agents include organic complex salts of iron(III) with aminopolycarboxylic acids, e.g., ethylenediaminetetraacetic acid, diethylenetriaminepentaacetic acid, cyclohexanediaminetetraacetic acid, methyliminodiacetic acid, 1,3-diaminopropanetetraacetic acid, and glycol ether diaminetetraacetic acid, or citric acid, tartaric acid or malic acid. The use of aminopolycarboxylic acid iron(III) complex salts, in particular, ethylenediaminetetraacetic acid iron(III) complex salts and and 1,3-diaminopropanetetraacetic acid iron(III) complex salts, is preferred for providing rapid processing and preventing environmental pollution. Further, aminopolycarboxylic acid iron(III) complex salts are particularly useful in both of a bleaching solution and a bleach-fixing solution. The pH of the bleaching solution or the bleach-fixing solution in which these aminopolycarboxylic acid iron(III) complex salts are used is generally from 4.0 to 8, but lower pH values can be used to speed up the processing.

Bleaching accelerators can be used, if necessary, in the bleaching solution, the bleach-fixing solution, or the prebaths thereof. Specific examples of useful bleaching accelerators are disclosed in the following publications: for example, there are the compounds which have a mercapto group or a disulfide group disclosed in U.S. Patent 3,893,858, German Patents 1,290,812, 2,059,988, JP-A-53-32736, JP-A-53-57831, JP-A-53-37418, JP-A-53-72623, JP-A-53-95630, JP-A-53-95631, JP-A-53-104232, JP-A-53-124424, JP-A-53-141623, JP-A-53-28426, and RD, No. 17129 (July, 1978); the thiazolidine derivatives disclosed in JP-A-50-140129; the thiourea derivatives disclosed in JP-B-45-8506, JP-A-52-20832, JP-A-53-32735, and U.S. Patent 3,706,561; the iodides disclosed in German Patent 1,127,715 and JP-A-58-16235; the polyoxyethylene compounds disclosed in German Patents 966,410 and 2,748,430; the polyamine compounds disclosed in JP-B-45-8836; the other compounds disclosed in JP-A-49-40943, JP-A-49-59644, JP-A-53-94927, JP-A-54-35727, JP-A-55-26506 and JP-A-58-163940; and bromide ion. Of these compounds, those which have a mercapto group or a disulfide group are preferred for providing large accelerating effect, and the compounds disclosed in U.S. Patent 3,893,858, German Patent 1,290,812 and JP-A-53-95630 are particularly preferred. Further, the compounds disclosed in U.S. Patent 4,552,834 are also preferred. These bleaching accelerators may be contained in the photographic material. These bleaching accelerators are especially effective for bleach-fixing the color photographic material for photographing.

The inclusion of organic acids as well as the compounds described above in the bleaching solution and the bleach-fixing solution is preferred for preventing the generation of bleach staining. Particularly preferred organic acids are compounds which have an acid dissociation constant (pKa) of from 2 to 5, specifically, acetic acid, propionic acid, and hydroxyacetic acid.

Thiosulfate, thiocyanate, thioether based compounds, thioureas, and large amounts of iodide can be used as the fixing agent which is used in a fixing solution and bleach-fixing solution, but thiosulfate is generally used, in particular, ammonium thiosulfate can be most widely used. Further, the combined use of thiosulfate and thiocyanate, thioether based compounds, thiourea is also preferred. Sulfite, bisulfite, carbonyl-bisulfite addition products or the sulfinic acid compounds disclosed in EP 294769A are preferred as preservatives for the fixing solution and the bleach-fixing solution. Moreover, the addition of aminopolycarboxylic acids and organic phosphonic acids to the fixing solution and the bleach-fixing solution is preferred for stabilizing these solutions.

The addition of compounds having a pKa of from 6.0 to 9.0, preferably imidazoles such as imidazole, 1-methylimidazole, 1-ethylimidazole and 2-methylimidazole, in an amount of from 0.1 to 10 mol per liter, to the fixing solution or the bleach-fixing solution is preferred for pH control in the present invention.

The total desilvering processing time is preferably shorter within the range not generating desilvering failure. The desilvering processing time is preferably from 1 minute to 3 minutes and more preferably from 1 minute to 2 minutes. Further, the processing temperature is from 25°C to 50°C, and preferably from 35°C to 45°C. The desilvering rate is increased and the occurrence of staining after processing is effectively prevented in the preferred temperature range.

Stirring as vigorous as possible in the desilvering process is preferred. Specific examples of methods of forced stirring include the method in which a jet of the processing solution is impinged on the surface of the emulsion of the photographic material as disclosed in JP-A-62-183460, the method in which the stirring effect is raised using a rotating means as disclosed in JP-A-62-183461, the method in which the photographic material is moved with a wiper blade, which is installed in the solution, in contact with the surface of the emulsion, and the generated turbulent flow at the surface of the emulsion increases the stirring effect, and the method in which the circulating flow rate of the entire processing solution is increased. These means for increasing the stirring level are effective for the bleaching solution, the bleach-fixing solution and the fixing solution. It is supposed that the increased stirring level increases the rate of supply of the bleaching agent and the fixing agent to the emulsion film and, as a result, increases the desilvering rate. Further, the above means of increasing stirring are more effective when a bleaching accelerator is used, and it is possible to extremely increase the bleaching accelerating effect and to eliminate the fixing hindrance action due to the bleaching accelerator.

The automatic processors for use in processing the photographic material of the present invention preferably have the means of transporting photographic materials as disclosed in JP-A-60-191257, JP-A-60-191258, and JP-A-60-191259. As described in the above JP-A-60-191257, such a transporting means can greatly reduce the carryover of the processing solution from the previous bath to the next bath and is effective for preventing the deterioration of the properties of the processing solution. These effects are especially effective in reducing the processing time of each process and reducing the replenishment rate of each processing solution.

The photographic material of the present invention is generally subjected to a washing process and/or a stabilizing process after the desilvering process. The amount of washing water in the washing process can be selected from a wide range according to the characteristics and the application of the photographic materials (for example, the materials such as couplers, etc., which are used), the temperature of washing water, the number of washing tanks (the number of washing stages), the replenishing system, that is, whether a countercurrent system or a concurrent system, and other various conditions. Of the foregoing conditions, the relationship between the number of washing tanks and the amount of water in a multistage countercurrent system can be obtained by the method described in Journal of the Society of Motion Picture and Television Engineers, Vol. 64, pages 248 to 253 (May, 1955). The amount of the washing water can be greatly reduced according to the multistage countercurrent system of the above literature, however, problems arise such that bacteria proliferate due to the increased residence time of the water in the tanks, and suspended matters produced thereby adhere to the photographic material. The method of reducing the calcium ion and magnesium ion concentrations as disclosed in JP-A-62-288838 can be used as a very effective means for overcoming this problem. Also, the isothiazolone compounds and the thiabendazoles as disclosed in JP-A-57-8542, the chlorine based disinfectants such as chlorinated sodium isocyanurate, the benzotriazole, and the disinfectants disclosed in Hiroshi Horiguchi, Bohkin Bohbai no Kagaku (The Chemistry of Biocides and Fungicides), published by Sankyo Shuppan K.K. (1986), Biseibutsu no Mekkin, Sakkin, Bohbai Gijutsu (Biocidal and Fungicidal Techniques to Microbes), edited by Hygiene Technology Society, published by Industrial Technology Society (1982), and Bohkin Bohbai Zai Jiten (Biocides and Fungicides Thesaurus), edited by Japanese Biocide and Fungicide Society (1986), can also be used.

The pH of the washing water in the processing of the photographic material of the present invention is from 4 to 9 and preferably from 5 to 8. The temperature and the time of the washing process can be selected variously according to the characteristics and the end use purpose of the photographic material to be processed, but is generally from 15°C to 45°C for 20 seconds to 10 minutes, and preferably from 25°C to 40°C for 30 seconds to 5 minutes. Further, the photographic material of the present invention can be processed directly with a stabilizing solution instead of being subjected to a water washing process as described above. Known methods as disclosed in JP-A-57-8543, JP-A-58-14834 and JP-A-60-220345 can be used in such a stabilizing process.

Further, there is also a case in which a stabilizing process is carried out following the above described water washing process, and the stabilizing bath containing a dye stabilizer and a surfactant which is used as a final bath for a photographic material for photographing is one example of such a process. Aldehydes such as formaldehyde and glutaraldehyde, N-methylol compounds, hexamethylenetetramine or an aldehyde-sulfite addition product can be used as a dye stabilizer.

Various chelating agents and fungicides can also be added to such a stabilizing bath.

The overflow generated by the replenishment of the above described washing water and/or stabilizing solution can be reused in other processes such as a desilvering process, etc.

When the above processing solutions are concentrated due to evaporation by the processing using an automatic processor, etc., it is preferred to replenish an appropriate amount of water to correct the concentration.

Color developing agents can be incorporated into a photographic material of the present invention to simplify and speed up the processing. Color developing agent precursors are preferred for the incorporation. For example, the indoaniline based compounds disclosed in U.S. Patent 3,342,597, the Schiff's base type compounds disclosed in U.S. Patent 3,342,599, Research Disclosure, Nos. 14850 and 15159, the aldol compounds disclosed in RD, No. 13924, the metal complex salts disclosed in U.S. Patent 3,719,492 and the urethane based compounds disclosed in JP-A-53-135628 can be used for this purpose.

Various 1-phenyl-3-pyrazolidones may be included, if required, in the photographic material of the present invention to accelerate color development. Typical compounds are disclosed in JP-A-56-64339, JP-A-57-144547 and JP-A-58-115438.

The various processing solutions of the present invention are used at a temperature of from 10°C to 50°C. The standard temperature is generally from 33°C to 38°C, but higher temperatures can be used to accelerate the processing to shorten the processing time, on the contrary, lower temperature can be used to improve the picture quality and stabilization of the processing solutions.

When the present invention is applied to black-and-white photographic materials, the various additives and development processing methods used therefor are not particularly limited, and those disclosed in the following places of JP-A-2-68539, JP-A-5-11389 and JP-A-2-58041 can be preferably used.

| | | |
|---|---|---|
| 1. | Silver-halide emulsion and the preparation method | from 6 lines up from the bottom, right lower column, page 8 to line 12, right upper column, page 10 of JP-A-2-68539 |
| 2. | Chemical sensitization method | from line 13, right upper column, page 10 to line 16, left lower column, page 10 of JP-A-2-68539; selenium sensitization method disclosed in JP-A-5-11389 |
| 3. | Antifoggant and stabilizer | from line 17, left lower column, page 10 to line 7, left upper column, page 11 of JP-A-2-68539; |
| | | from line 2, left lower column, page 3 to left lower column, page 4 of JP-A-2-68539 |
| 4. | Spectral sensitizing dye | from line 4, right lower column, page 4 to right lower column, page 8 of JP-A-2-68539; |
| | | from line 8, left lower column, page 12 to line 19, right lower column, page 12 of JP-A-2-58041 |
| 5. | Surfactant and antistatic agent | from line 14, left upper column, page 11 to line 9, left upper column, page 12 of JP-A-2-68539; |
| | | from line 14, left lower column, page 2 to line 12, page 5 of JP-A-2-58041 |
| 6. | Matting agent, plasticizer and sliding agent | from line 10, left upper column, page 12 to line 10, right upper column, page 12 of JP-A-2-58041; |
| | | from line 13, left lower column, page 5 to line 3, left lower column, page 10 of JP-A-2-58041 |
| 7. | Hydrophilic colloid | from line 11, right upper column, page 12 to line 16, left lower column, page 12 of JP-A-2-68539 |
| 8. | Hardening agent | from line 17, left lower column, page 12 to line 6, right upper column, page 13 of JP-A-2-68539 |
| 9. | Development processing method | from line 14, left upper column, page 15, to line 13, left lower column, page 15 of JP-A-2-68539 |

In addition to the application disclosed above, the present invention is applicable to diffusion transfer photography, so called instant photography. The diffusion transfer photography is disclosed in JP-A-5-297544.

The present invention is also applicable to heat-developable photographic material. The heat-developable photographic material to which the present invention is applicable may be a black-white photographic material or a color photographic material, such as heat-developable photographic materials as disclosed in JP-A-60-162251, JP-A-64-13546, JP-A-1-161236, US Patent Nos. 4,474,867, 4,478,927, 4,507,380, 4,500,626, 4,483,914, 4,783,396, 4,783,396, 4,740,445, JP-A-59-231539, JP-A-60-2950.

The present invention is applicable to a material for wet type reversal color copy. The material is disclosed as in Sample 101 in Example 1 of JP-A-3-152530 and Sample 1 in JP-A-2-90145.

The present invention is further described in detail below with reference to the examples, but the present invention should not be construed as being limited thereto.

### EXAMPLE 1 (Not in accordance with the invention)

A multilayer color photographic material was prepared as Sample 101 by coating each layer having the following composition on an undercoated cellulose triacetate film support.

### Composition of Light-Sensitive Layer

The coated weights of silver halide and colloidal silver are shown in units of g/m² as silver, the coated weights of couplers, additives and gelatin are shown in units of g/m², and the coated weights of sensitizing dyes are shown as the number of mols per mol of the silver halide in the same layer. The meaning of symbols for additives are shown below. When the additives have plural functions, the symbols are shown as the additives for the most typical function.
- UV:: Ultraviolet Absorberbelow.
- Solv:: High Boiling Point Organic Solvent
- ExF:: Dye
- ExS:: Sensitizing Dye
- ExC:: Cyan Coupler
- ExM:: Magenta Coupler
- ExY:: Yellow Coupler
- Cpd:: Additive

| First Layer: Antihalation Layer | |
|---|---|
| Black Colloidal Silver | 0.15 |
| Gelatin | 2.33 |
| UV-1 | 1.9 × 10⁻² |
| UV-2 | 4.7 × 10⁻² |
| UV-3 | 8.6 × 10⁻² |
| ExF-3 | 5.0 × 10⁻³ |
| ExM-3 | 2.3 × 10⁻² |
| Solv-1 | 0.16 |
| Solv-2 | 0.10 |

| Second Layer: Interlayer | |
|---|---|
| Gelatin | 0.88 |
| Polyethyl Acrylate Latex | 2.6 × 10⁻¹ |
| ExC-7 | 5.0 × 10⁻² |

| Third Layer: Low Sensitivity Red-Sensitive Emulsion Layer | |
|---|---|
| Silver Iodobromide Emulsion A | 0.24 |
| | coated weight of silver |
| Silver Iodobromide Emulsion B | 0.65 |
| | coated weight of silver |
| Gelatin | 1.75 |
| ExS-1 | 6.9 × 10⁻⁴ |
| ExS-2 | 4.0 × 10⁻⁴ |
| ExS-5 | 6.7 × 10⁻⁴ |
| ExS-7 | 1.4 × 10⁻⁵ |
| ExC-1 | 3.0 × 10⁻¹ |
| ExC-5 | 2.0 × 10⁻¹ |
| ExC-9 | 2.2 × 10⁻² |
| Cpd-4 | 5.3 × 10⁻² |
| ExC-4 | 6.1 × 10⁻² |

| Fourth Layer: Middle Sensitivity Red-Sensitive Emulsion Layer | |
|---|---|
| Silver Iodobromide Emulsion C | 0.67 |
| | coated weight of silver |
| Gelatin | 0.94 |
| ExS-1 | 3.5 × 10⁻⁴ |
| ExS-2 | 2.0 × 10⁻⁴ |
| ExS-5 | 3.4 × 10⁻⁴ |
| ExS-7 | 6.9 × 10⁻⁶ |
| ExC-1 | 1.3 × 10⁻¹ |
| ExC-4 | 4.6 × 10⁻² |
| ExC-5 | 8.6 × 10⁻² |
| ExC-6 | 1.1 × 10⁻² |
| ExC-7 | 4.6 × 10⁻² |
| Cpd-4 | 2.1 × 10⁻² |

| Fifth Layer: High Sensitivity Red-Sensitive Emulsion Layer | |
|---|---|
| Silver Iodobromide Emulsion D | 0.67 |
| | coated weight of silver |
| Gelatin | 0.68 |
| ExS-1 | 3.2 × 10⁻⁴ |
| ExS-2 | 1.8 × 10⁻⁴ |
| ExS-5 | 3.1 × 10⁻⁴ |
| ExS-7 | 4.8 × 10⁻⁵ |
| ExC-1 | 5.1 × 10⁻² |
| ExC-6 | 9.0 × 10⁻³ |
| ExC-4 | 2.0 × 10⁻² |
| ExC-9 | 1.0 × 10⁻² |
| Cpd-4 | 2.1 × 10⁻³ |
| Solv-1 | 0.08 |
| Solv-2 | 0.04 |

| Sixth Layer: Interlayer | |
|---|---|
| Gelatin | 0.62 |
| Cpd-1 | 0.08 |
| Polyethyl Acrylate Latex | 4.1 × 10⁻² |
| Solv-1 | 4.0 × 10⁻² |

| Seventh Layer: Low Sensitivity Green-Sensitive Emulsion Layer | |
|---|---|
| Silver Iodobromide Emulsion E | 0.14 |
| | coated weight of silver |
| Gelatin | 0.49 |
| ExS-8 | 5.7 × 10⁻⁵ |
| ExS-4 | 9.0 × 10⁻⁴ |
| ExS-5 | 1.8 × 10⁻⁴ |
| ExM-1 | 0.26 |
| Solv-1 | 0.15 |
| Solv-3 | 7.0 × 10⁻³ |

| Eighth Layer: Middle Sensitivity Green-Sensitive Emulsion Layer | |
|---|---|
| Silver Iodobromide Emulsion F | 0.08 |
| | coated weight of silver |
| Silver Iodobromide Emulsion E | 0.01 |
| | coated weight of silver |
| Gelatin | 0.14 |
| ExS-8 | 4.3 × 10⁻⁵ |
| ExS-4 | 6.8 × 10⁻⁴ |
| ExS-5 | 1.3 × 10⁻⁴ |
| ExM-1 | 4.9 × 10⁻² |
| ExM-7 | 1.0 × 10⁻² |
| ExY-1 | 5.0 × 10⁻³ |
| Solv-1 | 3.3 × 10⁻² |
| Solv-3 | 1.5 × 10⁻³ |

| Ninth Layer: High Sensitivity Green-Sensitive Emulsion Layer | |
|---|---|
| Silver Iodobromide Emulsion G | 0.60 |
| | coated weight of silver |
| Gelatin | 0.60 |
| ExS-4 | 5.0 × 10⁻⁴ |
| ExS-5 | 9.9 × 10⁻⁵ |
| ExS-8 | 3.2 × 10⁻⁵ |
| ExM-7 | 2.4 × 10⁻² |
| ExM-1 | 8.4 × 10⁻² |
| ExY-1 | 6.7 × 10⁻² |
| ExC-1 | 6.0 × 10⁻³ |
| ExC-4 | 8.0 × 10⁻³ |
| Cpd-6 | 8.0 × 10⁻³ |
| Solv-1 | 0.12 |
| Solv-2 | 0.06 |
| Solv-3 | 6.0 × 10⁻³ |

| Tenth Layer: Interlayer | |
|---|---|
| Gelatin | 0.39 |
| UV-2 | 1.4 × 10⁻² |
| UV-3 | 1.6 × 10⁻² |
| UV-5 | 4.2 × 10⁻² |
| Cpd-1 | 2.6 × 10⁻² |
| Polyethyl Acrylate Latex | 1.4 × 10⁻² |
| Solv-1 | 2.8 × 10⁻² |

| Eleventh Layer: Donor Layer Having Interlayer Effect for Red-Sensitive Layers | |
|---|---|
| Silver Iodobromide Emulsion H | 1.12 |
| | coated weight of silver |
| Silver Iodobromide Emulsion I | 0.26 |
| | coated weight of silver |
| Gelatin | 1.61 |
| ExS-3 | 6.4 × 10⁻⁴ |
| ExM-2 | 2.7 × 10⁻² |
| ExM-1 | 2.0 × 10⁻¹ |
| ExM-7 | 1.7 × 10⁻¹ |
| ExY-2 | 2.0 × 10⁻¹ |
| Solv-1 | 0.50 |

| Twelfth Layer: Yellow Filter Layer | |
|---|---|
| Yellow Colloidal Silver | 3.3 × 10⁻² |
| Gelatin | 0.61 |
| Cpd-1 | 4.3 × 10⁻² |
| Cpd-2 | 7.9 × 10⁻² |
| Cpd-5 | 1.0 × 10⁻³ |
| Solv-1 | 4.7 × 10⁻² |

| Thirteenth Layer: Low Sensitivity Blue-Sensitive Emulsion Layer | |
|---|---|
| Silver Iodobromide Emulsion J | 0.62 |
| | coated weight of silver |
| Gelatin | 1.67 |
| ExS-9 | 8.8 × 10⁻⁴ |
| ExY-2 | 1.2 × 10⁻¹ |
| ExY-3 | 5.5 × 10⁻¹ |
| ExC-9 | 6.3 × 10⁻² |
| ExC-1 | 3.0 × 10⁻² |
| ExC-10 | 8.4 × 10⁻² |
| Solv-1 | 0.33 |

| Fourteenth Layer: High Sensitivity Blue-Sensitive Emulsion Layer | |
|---|---|
| Silver Iodobromide Emulsion K | 0.14 |
| | coated weight of silver |
| Silver Iodobromide Emulsion L | 0.10 |
| | coated weight of silver |
| Silver Iodobromide Emulsion M | 0.22 |
| | coated weight of silver |
| Gelatin | 1.00 |
| ExS-6 | 4.4 × 10⁻⁴ |
| ExY-2 | 7.6 × 10⁻² |
| ExY-3 | 1.1 × 10⁻¹ |
| ExY-6 | 3.1 × 10⁻¹ |
| ExC-1 | 1.8 × 10⁻² |
| ExC-10 | 2.3 × 10⁻² |
| Solv-1 | 1.7 × 10⁻¹ |

| Fifteenth Layer: First Protective Layer | |
|---|---|
| Fine Grained Silver Iodobromide | Emulsion N 0.06 |
| | coated weight of silver |
| Gelatin | 0.51 |
| UV-2 | 4.0 × 10⁻² |
| UV-3 | 4.9 × 10⁻² |
| UV-5 | 0.12 |
| Cpd-3 | 0.10 |
| ExF-4 | 2.1 × 10⁻³ |
| ExF-5 | 6.3 × 10⁻³ |
| Solv-4 | 2.0 × 10⁻² |
| Polyethyl Acrylate Latex | 9.0 × 10⁻² |

| Sixteenth Layer: Second Protective Layer | |
|---|---|
| Fine Grained Silver Iodobromide Emulsion N | 0.18 |
| Gelatin | 0.84 |
| B-1 (diameter: 2.0 µm) | 8.0 x 10⁻² |
| B-2 (diameter: 2.0 µm) | 8.0 x 10⁻² |
| B-3 | 3.5 x 10⁻² |
| W-5 | 1.8 x 10⁻² |
| H-1 | 0.18 |

In addition to the above, 1,2-benzisothiazolin-3-one (200 ppm in average based on gelatin), n-butyl-p-hydroxy-benzoate (1,000 ppm in average based on gelatin), and 2-phenoxyethanol (10,000 ppm in average based on gelatin) were added to the thus-produced sample. Further, W-1 to W-6, B-1 to B-6, F-1 to F-17, and iron salts, lead salts, gold salts, platinum salts, iridium salts and rhodium salts were appropriately included in each layer to improve storage stability, processing property, pressure resistance, fungicidal and biocidal properties, antistatic property and coating property.

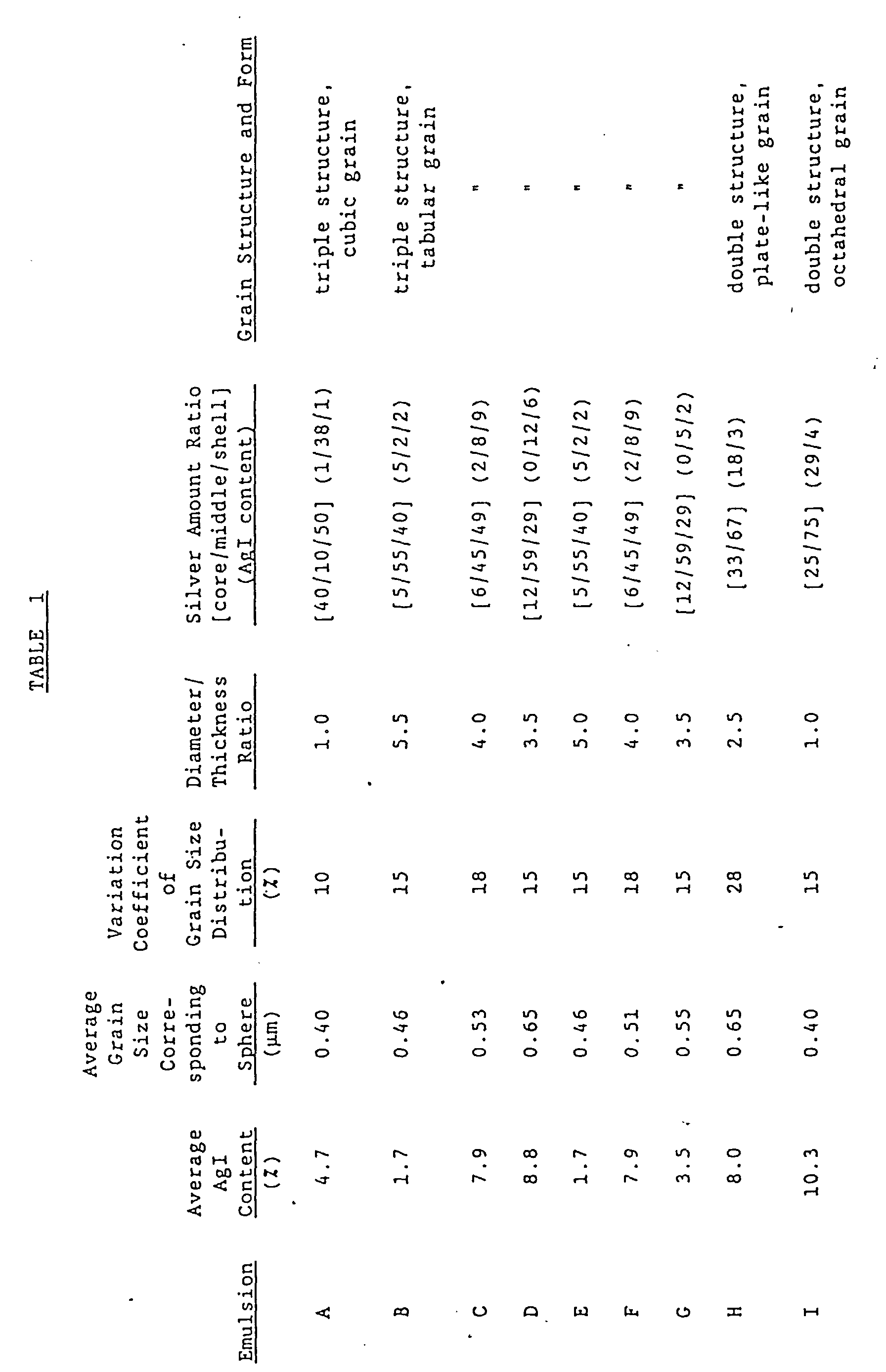

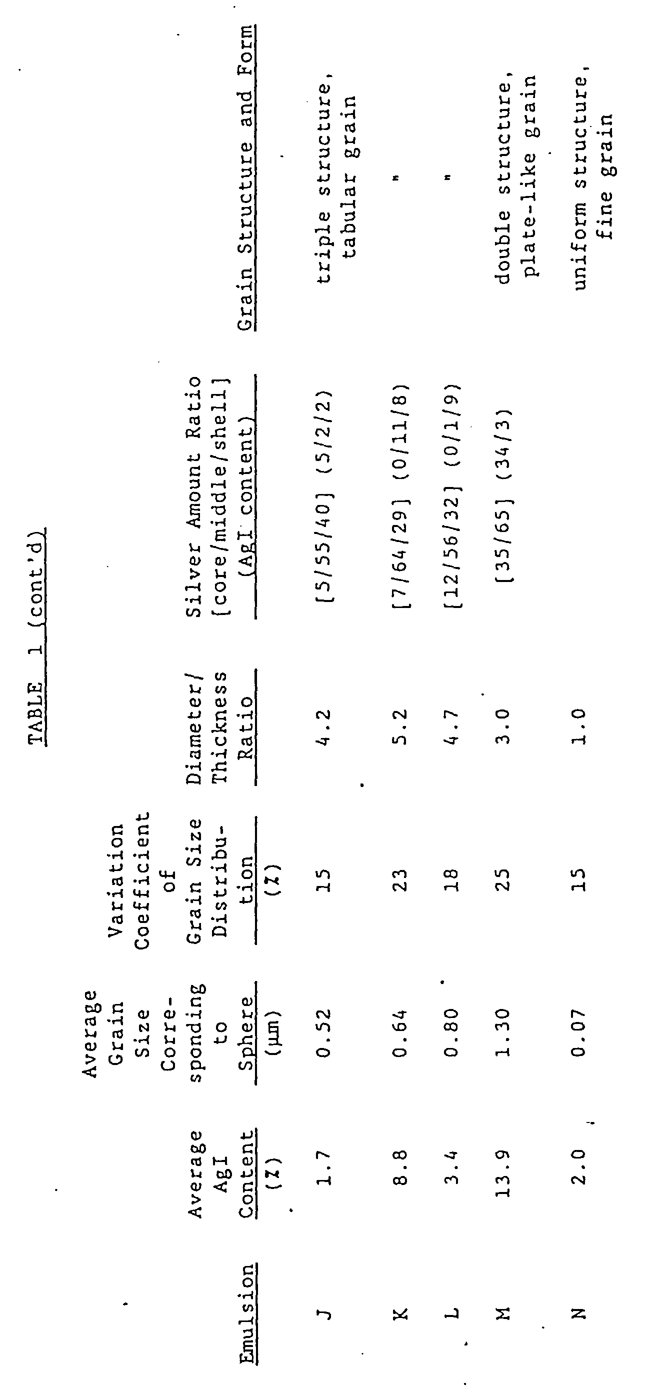

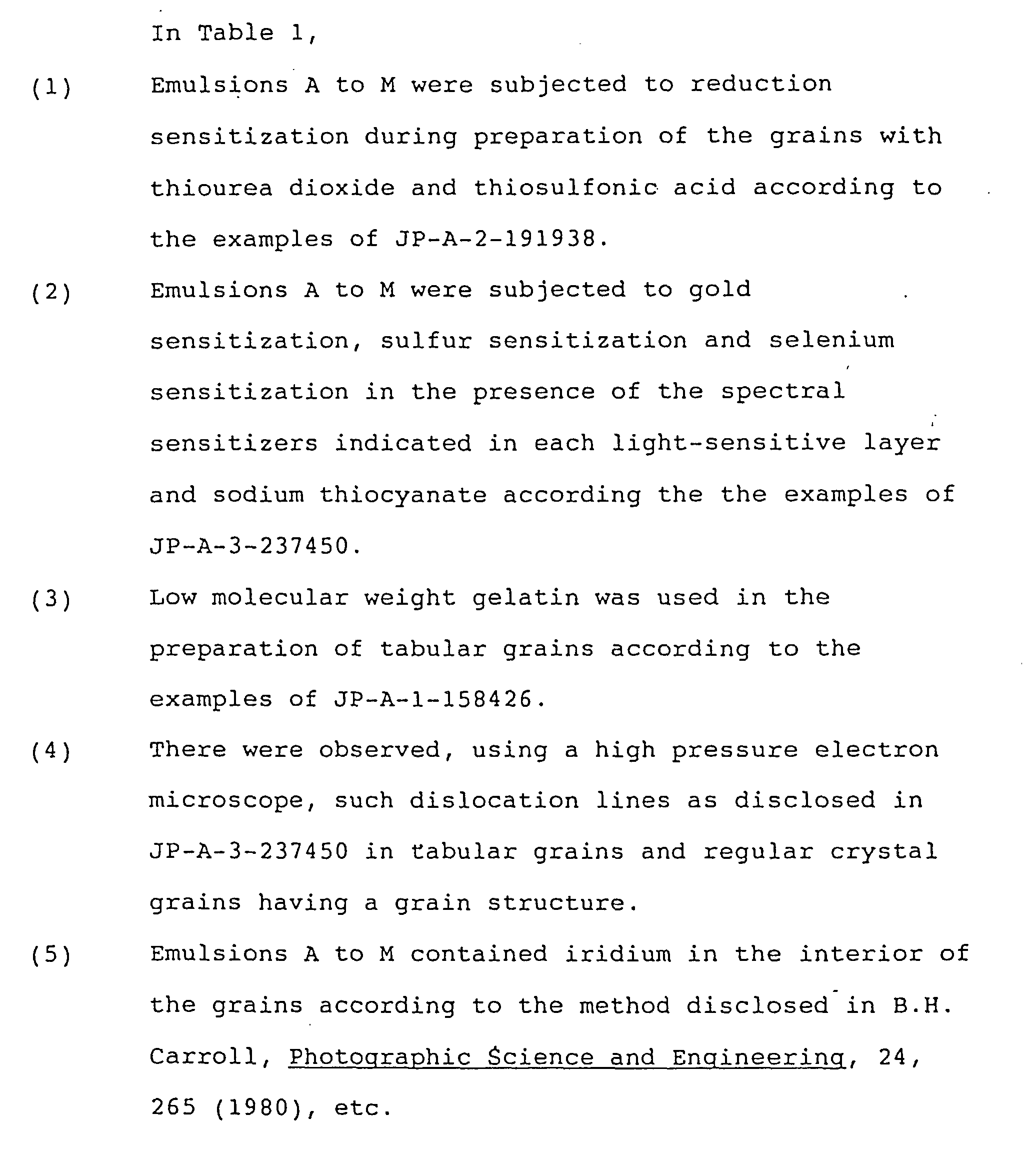

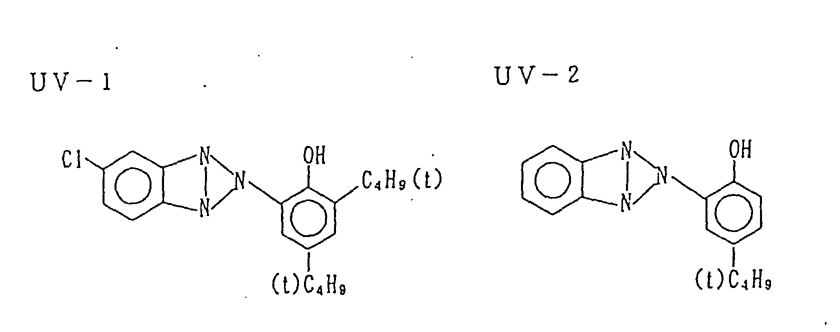

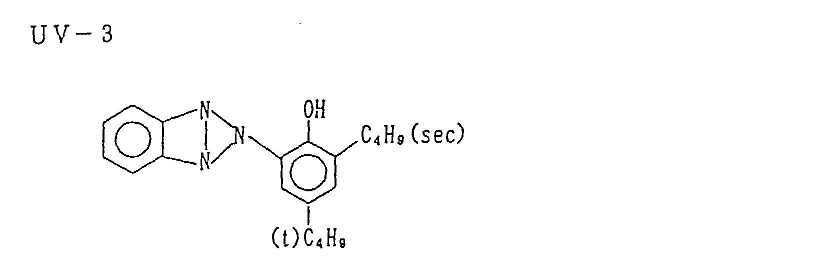

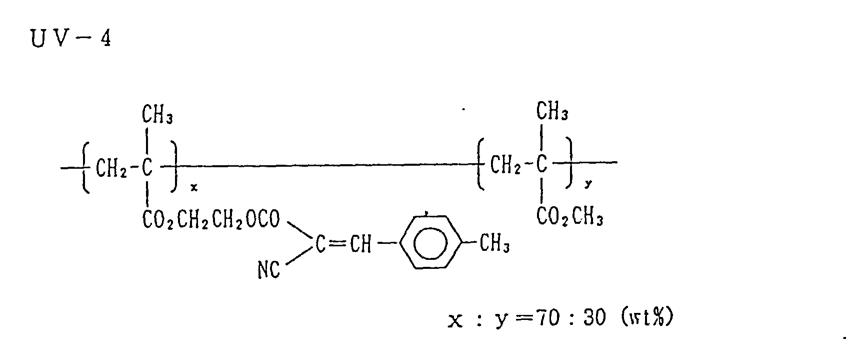

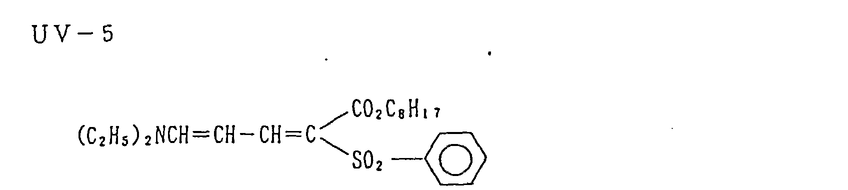

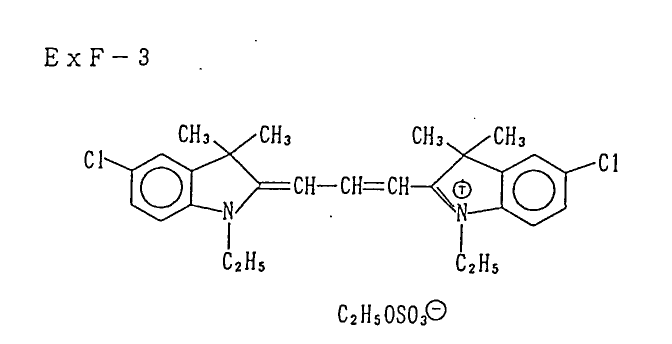

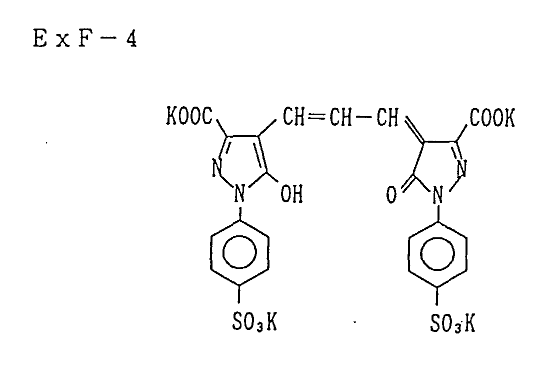

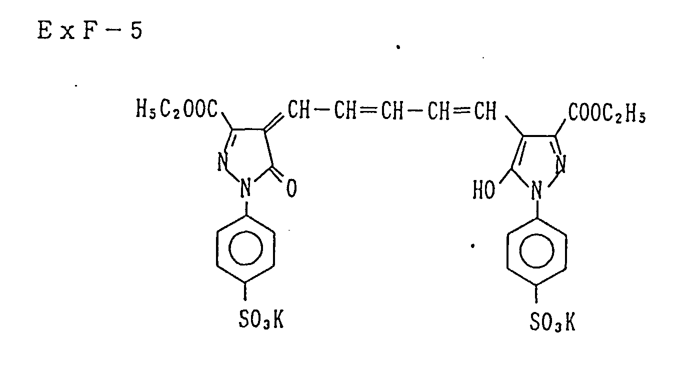

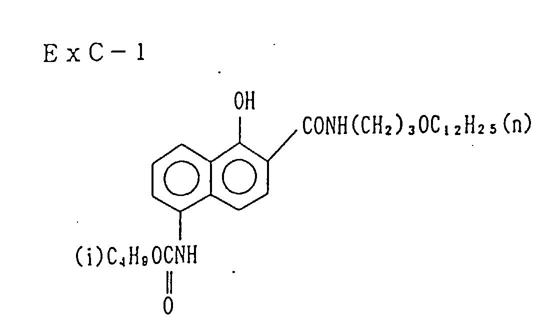

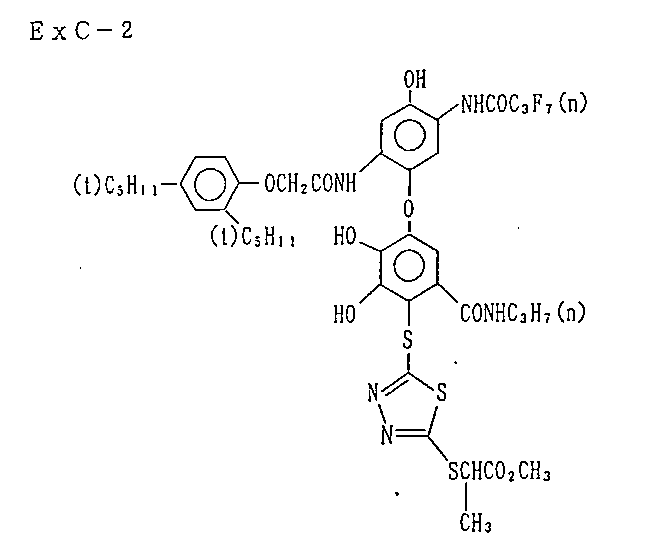

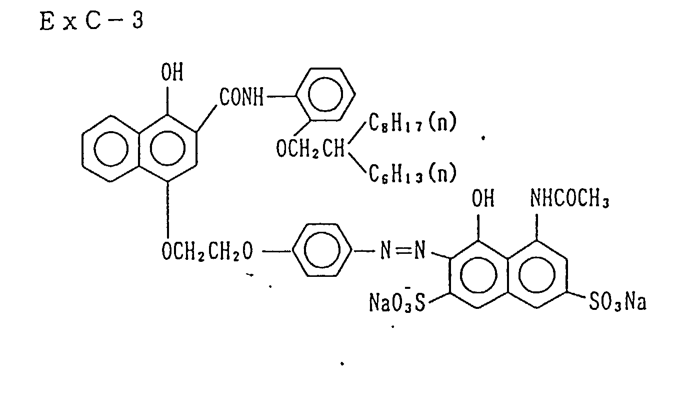

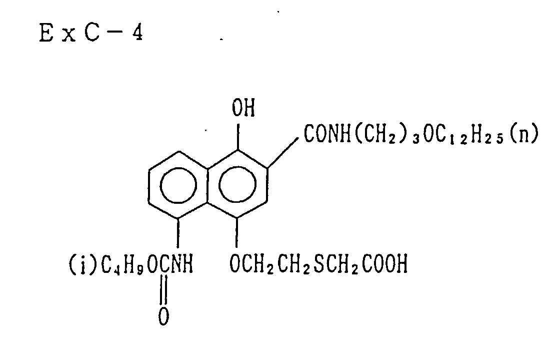

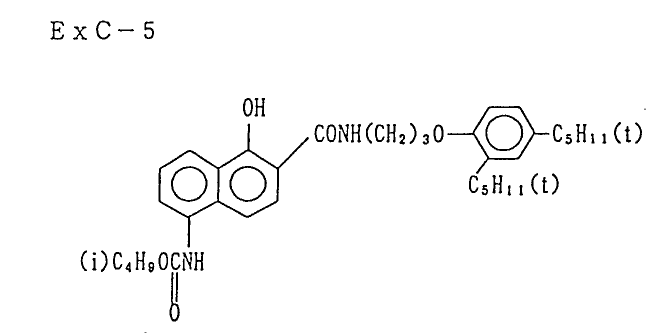

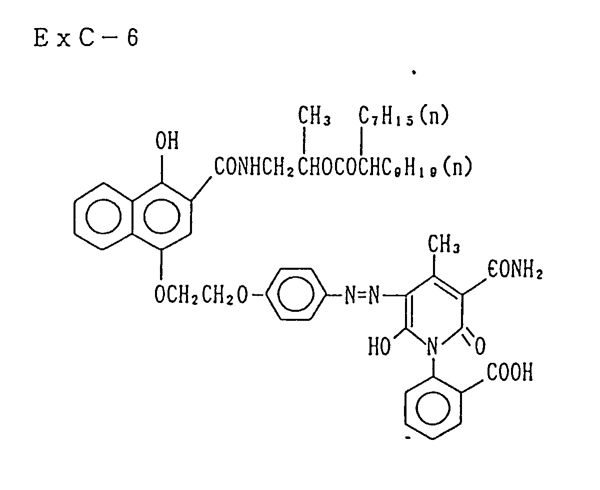

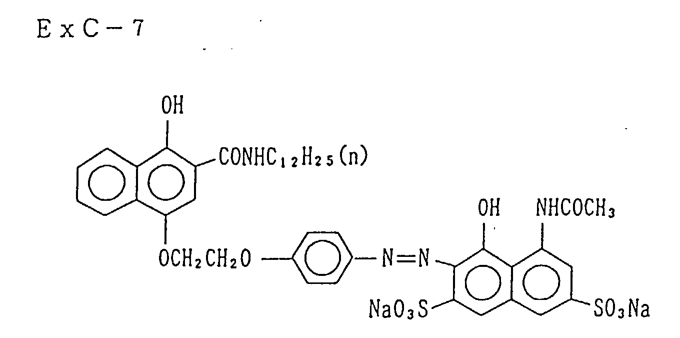

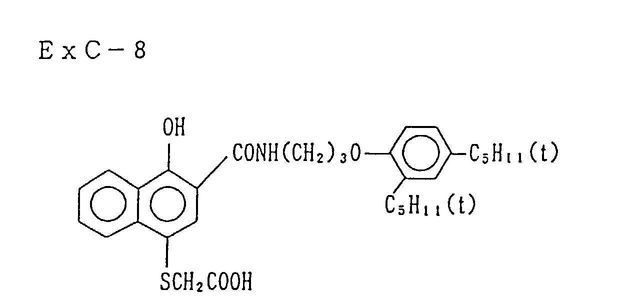

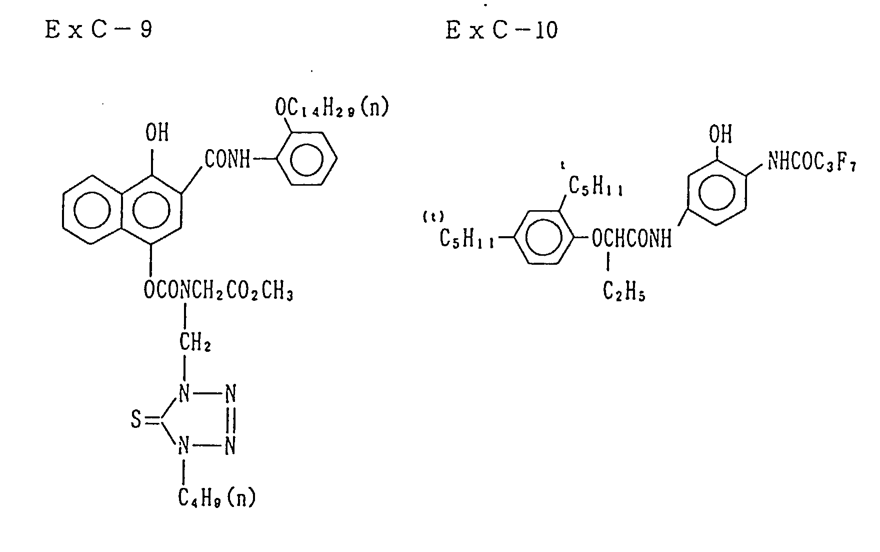

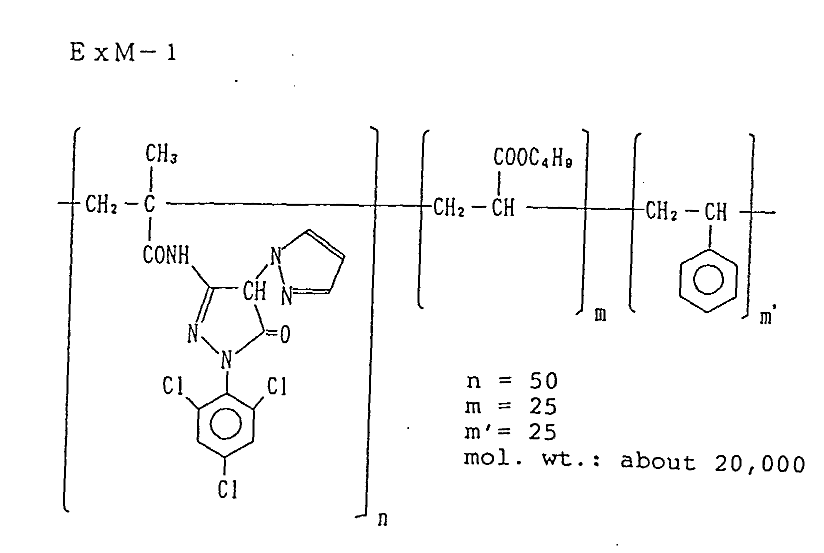

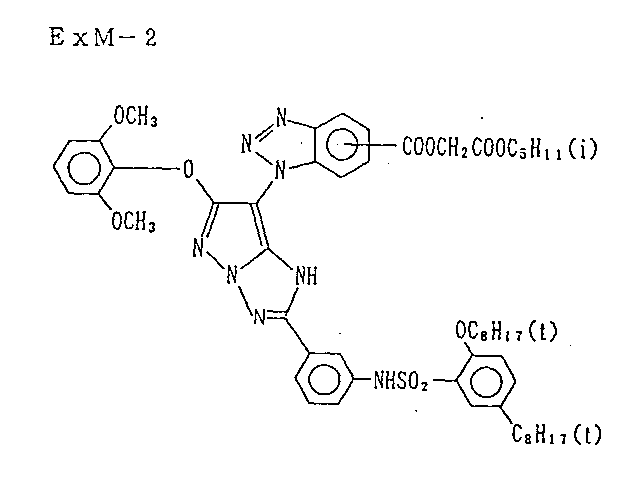

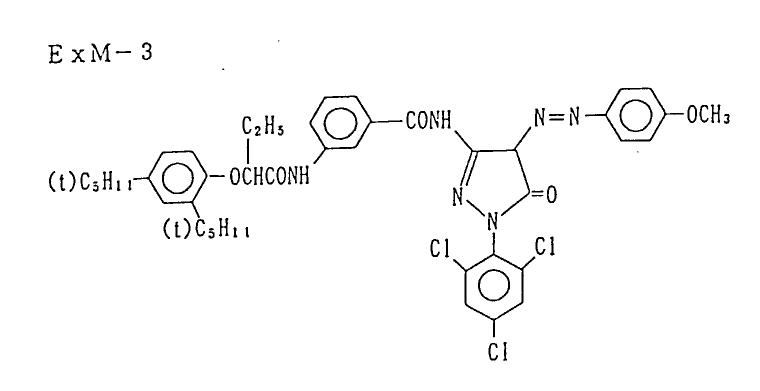

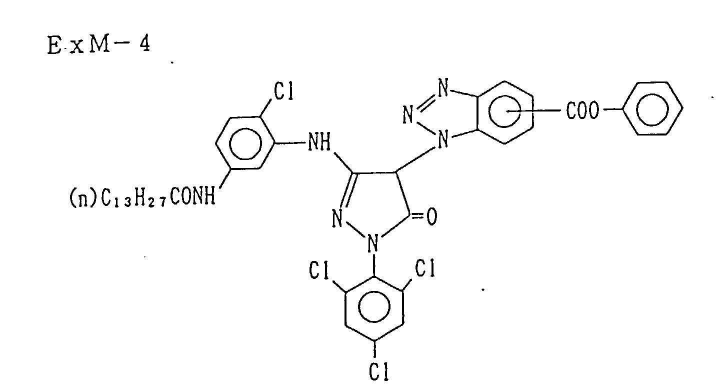

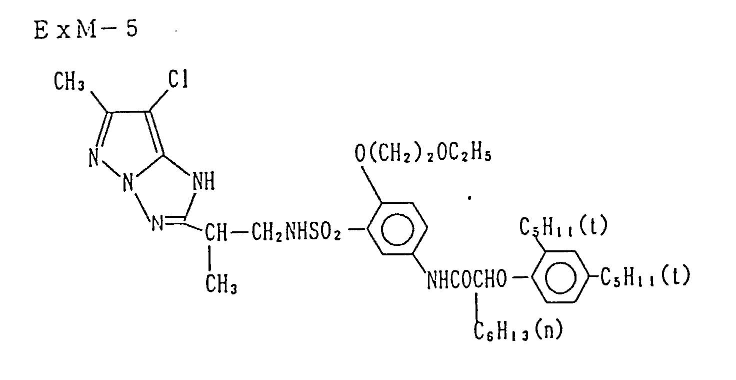

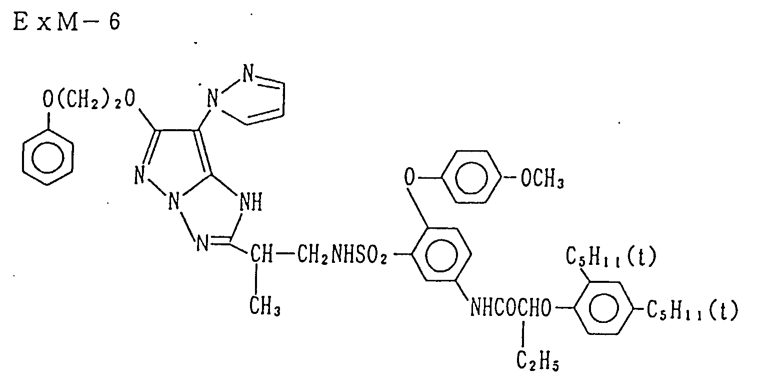

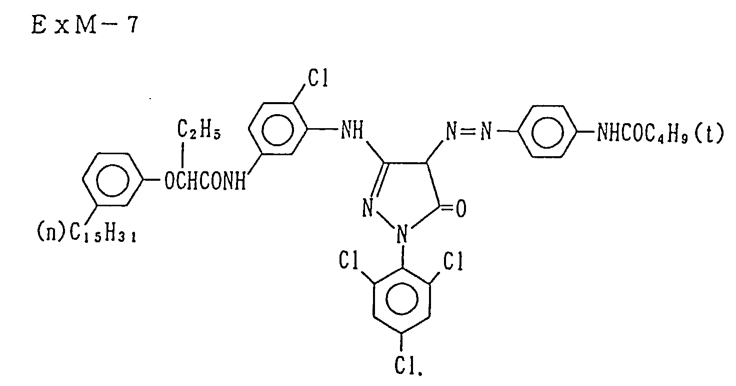

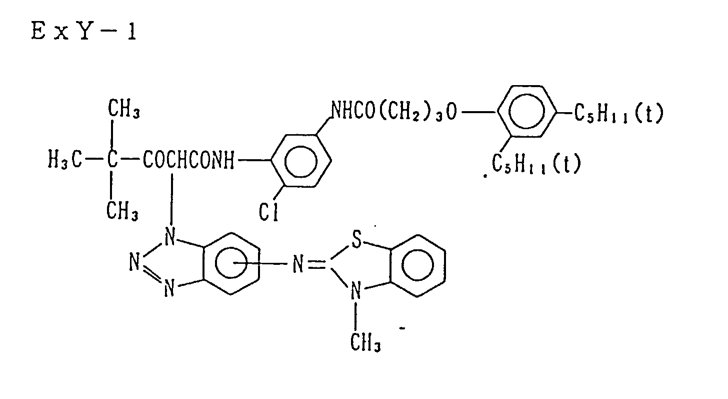

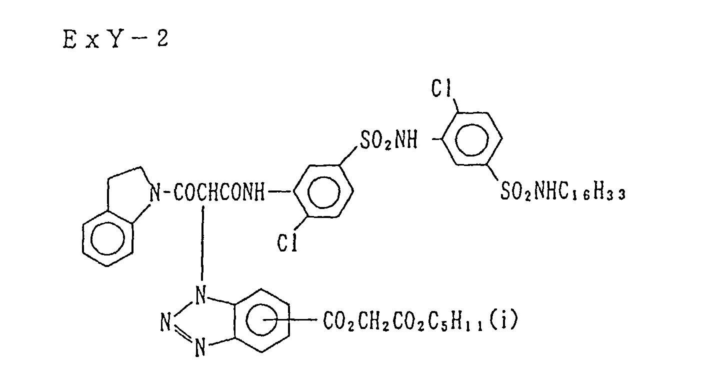

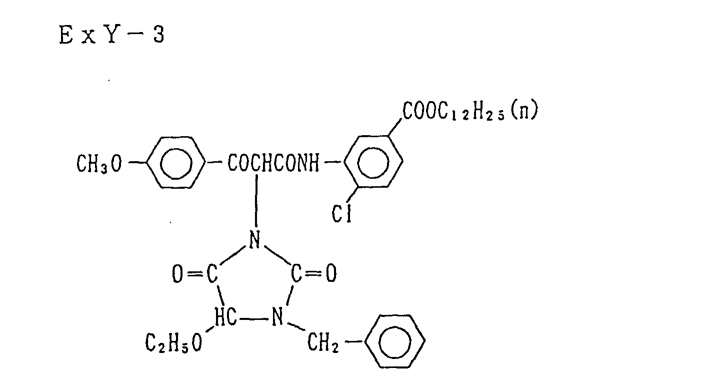

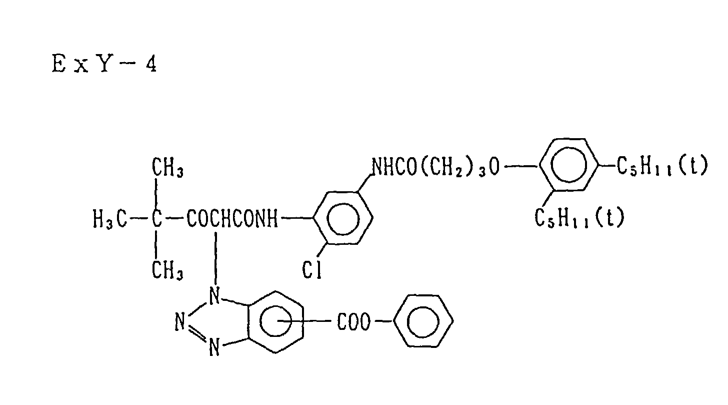

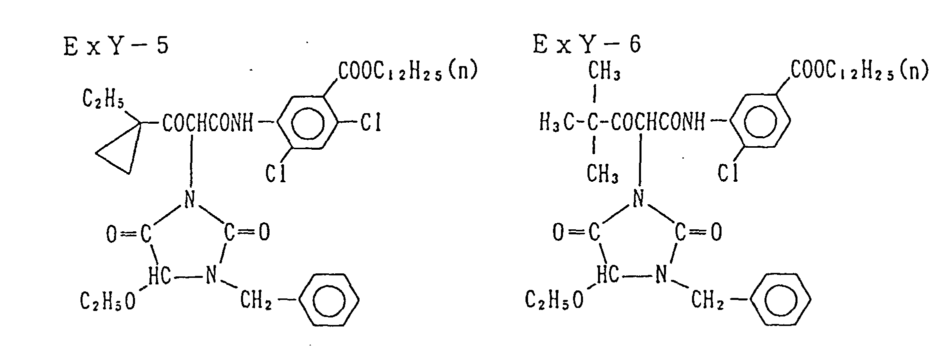

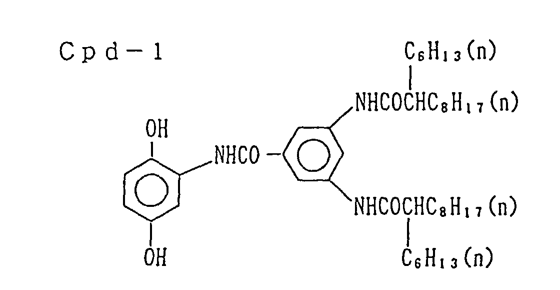

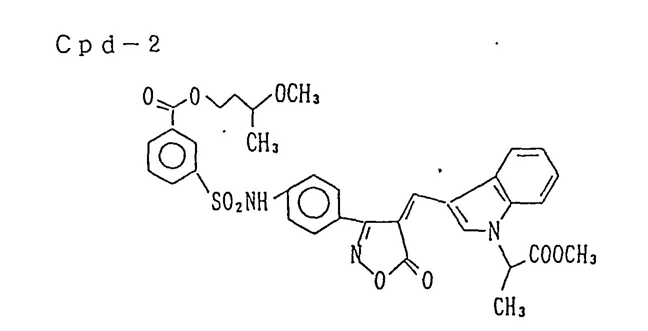

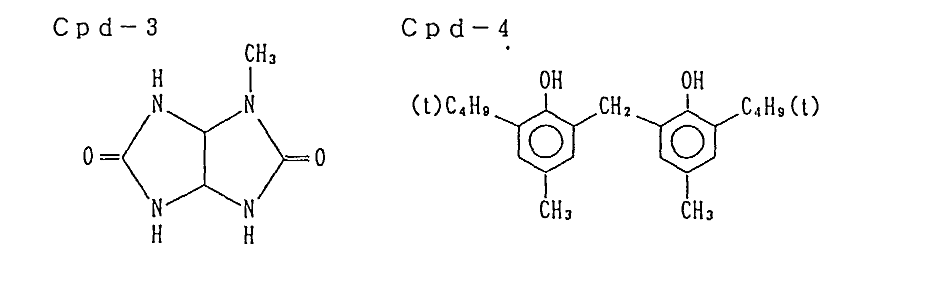

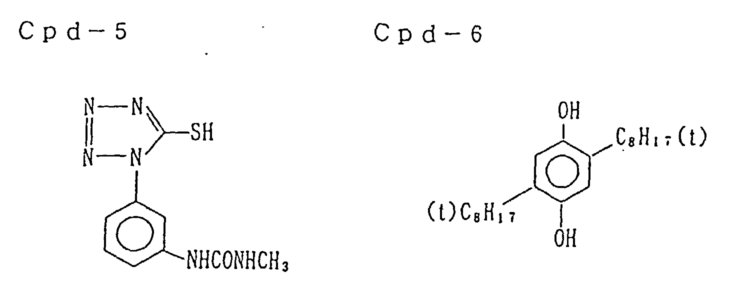

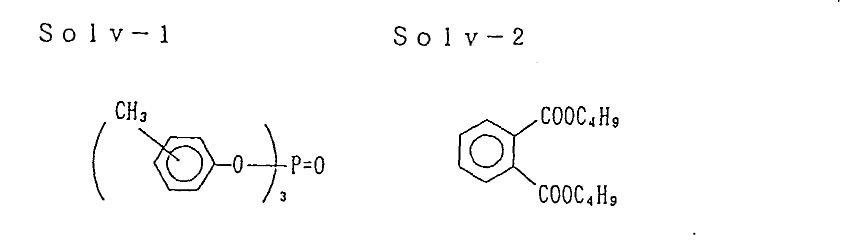

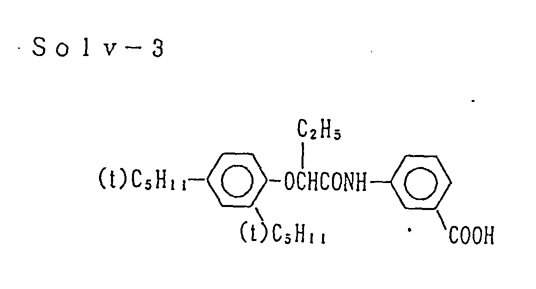

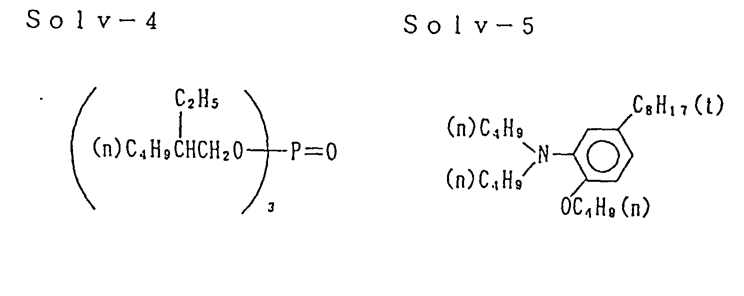

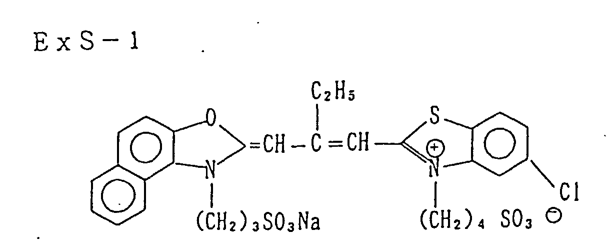

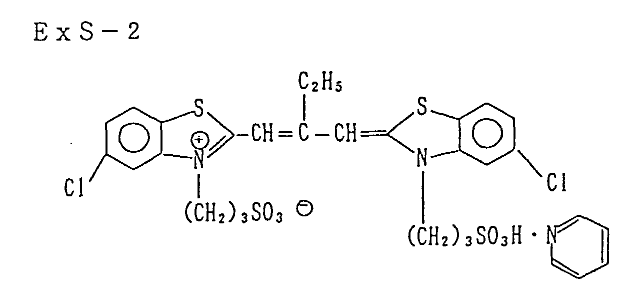

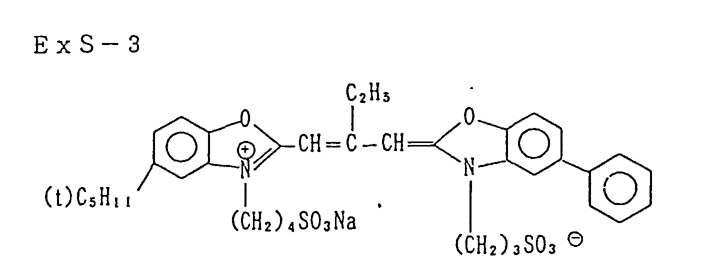

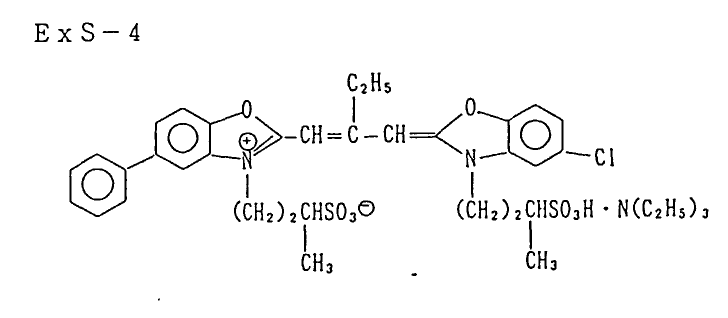

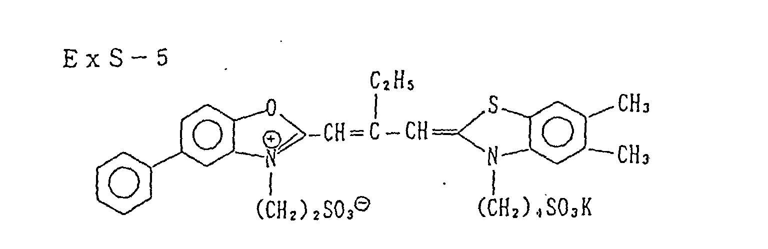

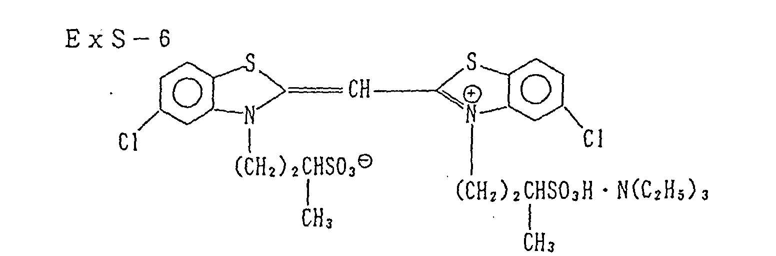

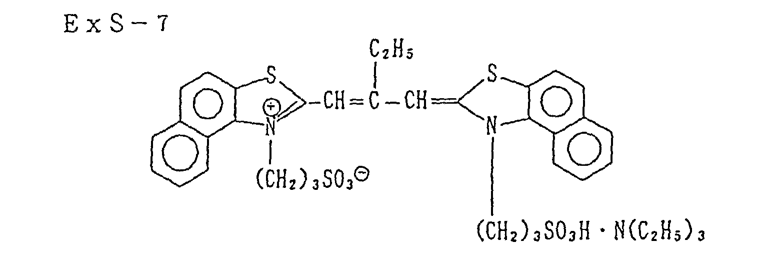

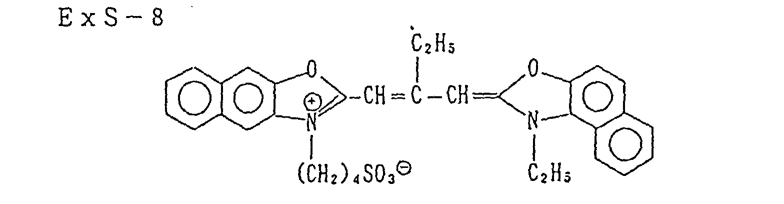

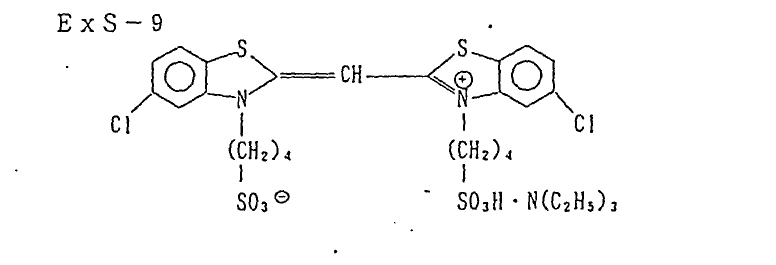

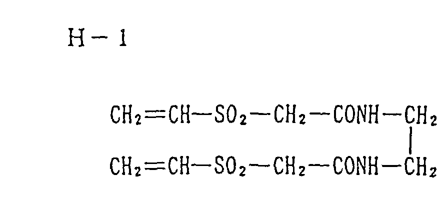

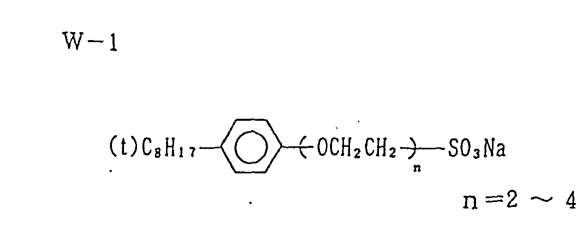

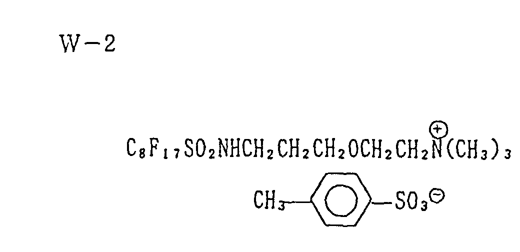

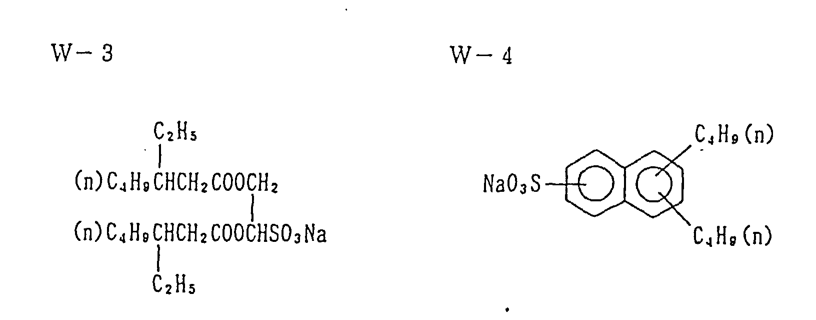

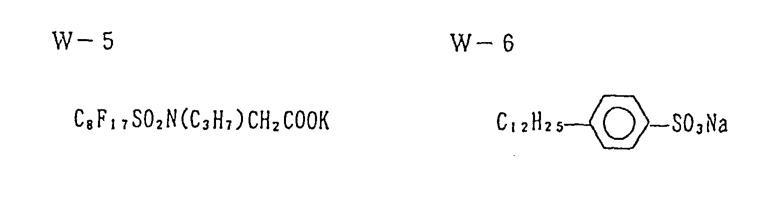

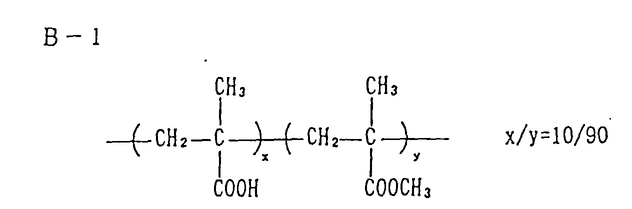

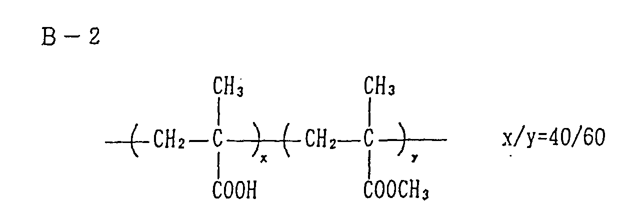

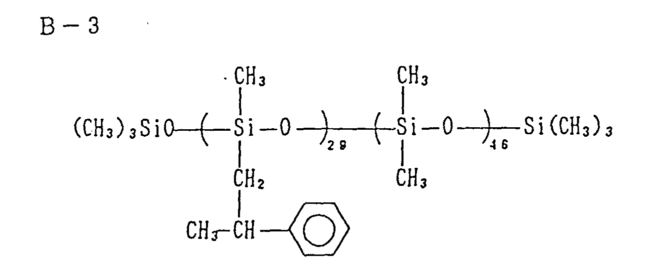

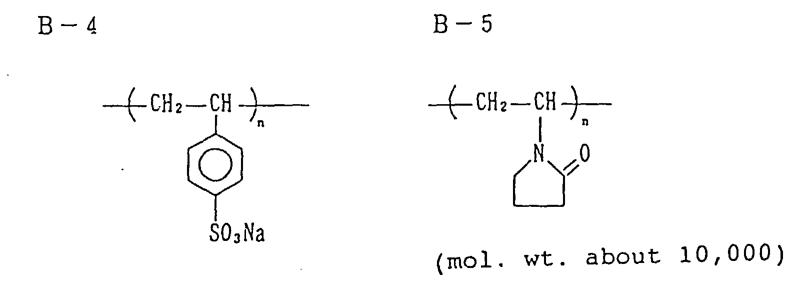

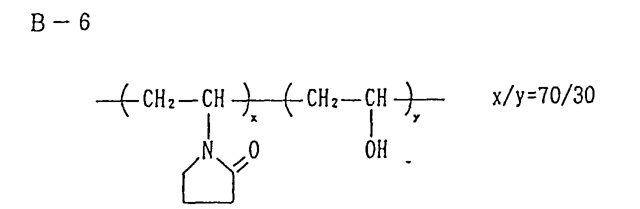

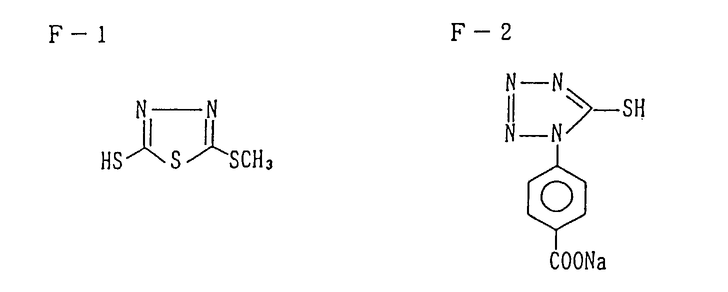

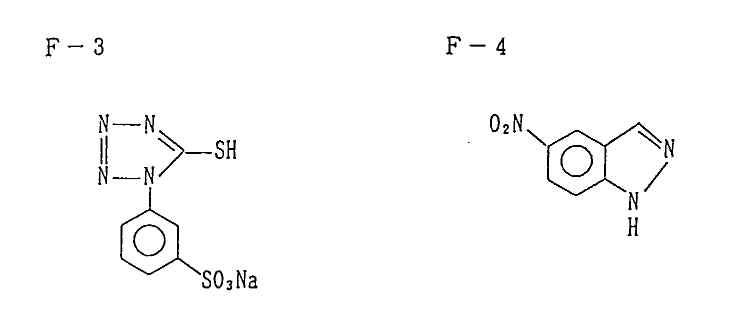

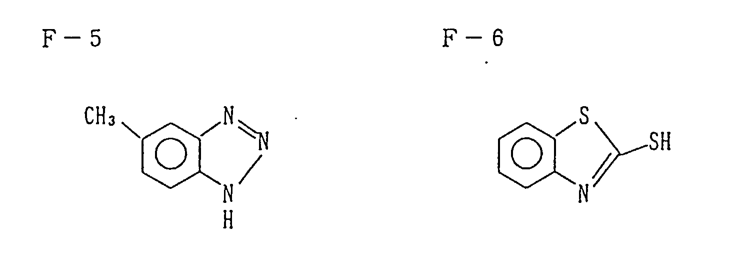

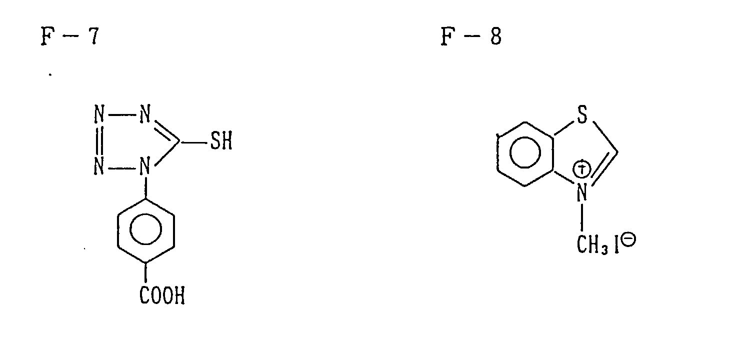

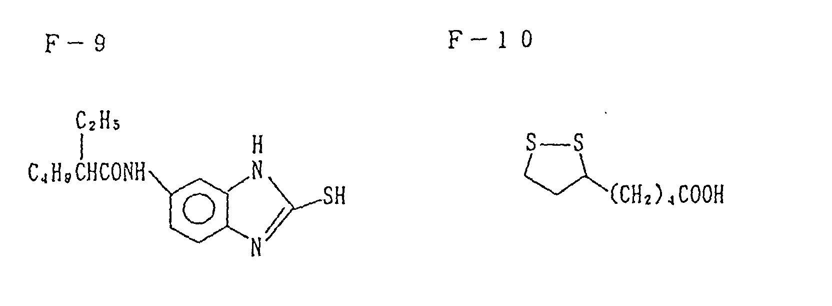

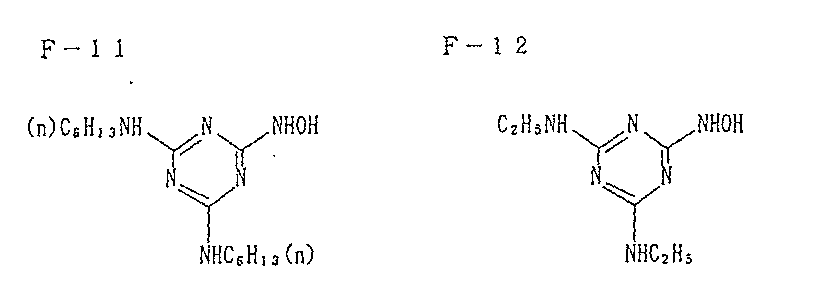

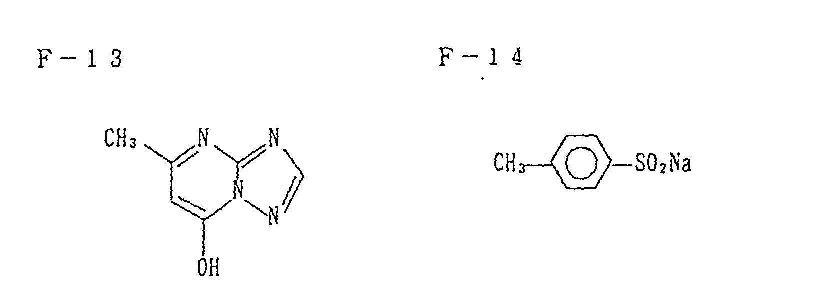

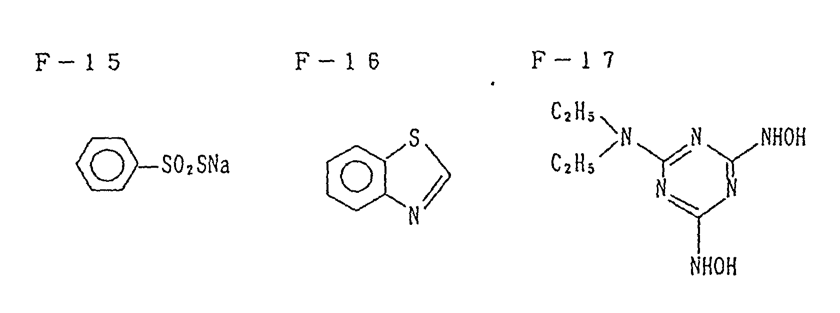

Compounds represented by formula (I) or comparative compounds were added to the fourth layer of Sample 101 in coemulsified state with the couplers of the fourth layer so as to provide the addition amount of 5 × 10⁻² mol per mol of Ag in the fourth layer. Samples 102 to 116 as indicated in Table 2 were prepared in the same manner as the preparation of Sample 101 except for the above. In the case where there are shown two compounds, they were mixed in mol ratio of 1/1 and the total amount was 5 × 10⁻² mol per mol of Ag.

The evaluation of the fluctuation of the photographic property after photographing until processing was carried out as follows.

After being subjected to sensitometry exposure, these samples were stored for 3 days under the forced aged conditions of 60°C, 7.0% RH and 50°C, 80% RH, then color development processed at 38°C as follows. Densities of the processed samples were measured using a blue filter and a red filter. The difference in sensitivities of these samples and samples immediately development processed after exposure was evaluated.

The above color photographic materials were processed, after exposure, according to the following processing step.

| Processing Step | | |
|---|---|---|
| Step | Processing Time | Processing Temperature (°C) |
| Color Development | 3 min 15 sec | 38 |
| Bleaching | 3 min 00 sec | 38 |
| Washing | 30 sec | 24 |
| Fixing | 3 min 00 sec | 38 |
| Washing (1) | 30 sec | 24 |
| Washing (2) | 30 sec | 24 |
| Stabilization | 30 sec | 38 |
| Drying | 4 min 20 sec | 55 |

The composition of each processing solution is as follows.

| Color Developing Solution | |
|---|---|
| | (unit: g) |
| Diethylenetriaminepentaacetic Acid | 1.0 |
| 1-Hydroxyethylidene-1,1-diphosphonic Acid | 3.0 |
| Sodium Sulfite | 4.0 |
| Potassium Carbonate | 30.0 |
| Potassium Bromide | 1.4 |
| Potassium Iodide | 1.5 mg |
| Hydroxylamine Sulfate | 2.4 |
| 4-[N-Ethyl-N-β-hydroxyethylamino]-2-methylaniline Sulfate | 4.5 |
| Water to make | 1.0 ℓ |
| pH | 10.05 |

| Bleaching Solution | |
|---|---|
| | (unit: g) |
| Sodium Ethylenediaminetetraacetato Ferrate Trihydrate | 100.0 |
| Disodium Ethylenediaminetetraacetate | 10.0 |
| 3-Mercapto-1,2,4-triazole | 0.03 |
| Ammonium Bromide | 140.0 |
| Ammonium Nitrate | 30.0 |
| Aqueous Ammonia (27%) | 6.5 ml |
| Water to make | 1.0 ℓ |
| pH | 6.0 |

| Fixing Solution | |
|---|---|
| | (unit: g) |
| Disodium Ethylenediaminetetraacetate | 0.5 |
| Ammonium Sulfite | 20.0 |
| Aqueous Solution of Ammonium Thiosulfate (700 g/liter) | 295.0 |
| Acetic Acid (90%) | 3.3 |
| Water to make | 1.0 ℓ |
| pH | 6.7 |

| Stabilizing Solution | |
|---|---|
| | (unit: g) |
| p-Nonylphenoxypolyglycidol (average polymerization degree of glycidol: 10) | 0.2 |
| Ethylenediaminetetraacetic Acid | 0.05 |
| 1,2,4-Triazole | 1.3 |
| 1,4-Bis(1,2,4-triazol-l-ylmethyl)-piperazine | 0.75 |
| Hydroxyacetic Acid | 0.02 |
| Hydroxyethyl Cellulose (SP-2000, Daicel Chemical Industries HEC) | 0.1 |
| 1,2-Benzisothiazolin-3-one | 0.05 |
| Water to make | 1.0 ℓ |
| pH | 8.5 |

The logarithmic value of the reciprocal of the exposure amount required to provide an optical density of fog + 1.0 was taken as the photographic sensitivity. The fluctuation of the photographic performance between the time after photographing until processing was expressed as a relative sensitivity of the sample stored, after exposure, under the forced aged conditions to that of the sample processed immediately after exposure (difference in the logarithmic values). The value nearer to zero means less fluctuation of the sensitivity due to the storage of the photographic material.

The evaluation was carried out for the fluctuation of the photographic performance with respect to the red-sensitive layer containing the compound of formula (I). In the sample containing the compound of formula (I), the fluctuation of photographic performance was less compared with the sample not containing the compound (Sample 101).

When the compound represented by formula (I) was added, there were no influences to other photographic performances.

### EXAMPLE 2

Samples 201 to 210 were prepared using the compounds of the present invention in the same manner as in Example 1. Evaluation of the aged fluctuation in sensitivity was conducted.

The evaluation was carried out for the fluctuation of the photographic property with respect to both of the red-sensitive layer containing the compound of formula (I) and the blue-sensitive layer not containing the compound of formula (I). In the sample containing the compound of formula (I) or the comparative compound, the fluctuation of photographic property of the blue-sensitive layer based on the sample not containing either compound (Sample 101) means the diffusion of the compound from the red-sensitive layer (addition layer) to the blue-sensitive layer (non-addition layer) and is not desired.

The results obtained are shown in Table 3.

**TABLE 3**

| | | Aged Fluctuation of Photographic Property after Photographing until Processing | |
|---|---|---|---|
| Sample No. | Compound | Red-Sensitive Layer (60°C, 70%) | Blue-Sensitive Layer (60°C, 70%) |
| 201 (Comparison) | - | 0.70 | -0.03 |
| 202 ( " ) | 55 | 0.05 | -0.03 |
| 203 ( " ) | 56 | 0.04 | -0.03 |
| 204 ( " ) | 57 | 0.05 | -0.03 |
| 205 ( " ) | 58 | 0.05 | -0.03 |
| 206 ( " ) | 63 | 0.07 | -0.03 |
| 207 ( " ) | 66 | 0.12 | -0.03 |
| 208 ( " ) | 68 | 0.05 | -0.03 |
| 209 ( " ) | 82 | 0.11 | -0.03 |
| 210 ( " ) | 57, 58 | 0.05 | -0.03 |

In the sample containing the compound represented by formula (II) of the present invention, the layer containing the compound (the red-sensitive layer) underwent less fluctuation of sensitivity with the passage of time after photographing until processing and did not adversely affect the sensitivity with the lapse of time of the layer not containing the compound of the present invention (the blue-sensitive layer).

In addition, the compound represented by formula (II) of the present invention did not adversely affect other photographic performances and preferred results could be obtained.

### EXAMPLE 3

Compounds of the present invention other than those indicated in Table 2 in Example 1 and Table 3 in Example 2, that is, Compounds 59 to 62, 64, 65, 67, 69 to 81, 83 to 93, 97 and 99 to 110 were evaluated similarly. They showed desirable effect to reduce the fluctuation of the photographic property after photographing with the lapse of time.

### EXAMPLE 4

When the compound of the present invention was used in a green-sensitive layer or a blue-sensitive layer, the storage stability of the layer containing the compound was improved similarly in Example 1, and desired effect could be obtained.

### EXAMPLE 5

Sample 301 (not in accordance with the invention) shown below was prepared instead of Sample 101 of Example 1.

Samples 402 to 410 corresponding to 202 to 210 were prepared according to the method of preparing Sample 301 except that compounds represented by formula (I) or comparative compounds were added to the fourth layer of Sample 301 so that the compounds were coemulsified with the couplers of this layer and the amount became 5 × 10⁻² mol per mol of the Ag in this layer.

Further, where two compounds are indicated, they were mixed in mol ratio of 1/1 so that the total mol number became 5 × 10⁻² mol per mol of the AgX.

Evaluation of the compounds of the present invention was conducted using these samples, they showed desirable performance similarly as in Examples 1 and 2.

### 1) Support

The support which was used in Example 5 was prepared as follows.

100 weight parts of polyethylene-2,6-naphthalate polymer and 2 weight parts of Tinuvin P. 326 (product of Ciba-Geigy), as an ultraviolet absorber, were dried, then melted at 300°C, subsequently, extruded through a T-type die, and stretched 3.3 times in a lengthwise direction at 140°C and then 3.3 times in a width direction at 130°C, and further fixed for 6 seconds at 250°C and PEN film having a thickness of 90 µm was obtained. Appropriate amounts of a blue dye, magenta dye and yellow dye were added to this PEN film (I-1, I-4, I-6, I-24, I-26, I-27, II-5 disclosed in Kokai-Giho, Kogi No. 94-6023). Further, a part of the film was wound on to a stainless steel spool having a diameter of 20 cm and provided heat history at 110°C for 48 hours to obtain a support reluctant to get curling habit.

### 2) Coating of a subbing layer

A coating solution for a subbing layer having the following composition was coated on each side of the above support after both surfaces of which were corona discharged, UV discharged, and further glow discharged and flame discharged. The subbing layer was provided on the hotter side at the time of stretching. The corona discharge treatment was carried out using solid state corona processor model 6 KVA available from Pillar Co. which can treat the support of 30 cm wide at a rate of 20 m/min. At that time, the treatment of 0.375 KV·A·min/m² was conducted to the support from the reading of the electric current and voltage. The discharge frequency at the treatment time was 9.6 KHz, gap clearance between the electrode and the induction roll was 1.6 mm. UV discharge treatment was conducted heating at 75°C. Further, glow discharge treatment was conducted at 3,000 W for 30 second irradiation using a cylindrical electrode.

| | |
|---|---|
| Gelatin | 3 g |
| Distilled Water | 25 ml |
| Sodium-α-sulfodi-2-ethylhexylsuccinate | 0.05 g |
| Formaldehyde | 0.02 g |
| Salicylic Acid | 0.1 g |
| Diacetyl Cellulose | 0.5 g |
| p-Chlorophenol | 0.5 g |
| Resorcin | 0.5 g |
| Cresol | 0.5 g |
| (CH₂=CHSO₂CH₂CH₂NHCO)₂CH₂ | 0.02 g |
| Trimethylolpropane addition product of 3 time mol of aziridine | 0.02 g |
| Trimethylolpropane addition product of 3 time mol of toluenediisocyanate | 0.02 g |
| Methanol | 15 ml |
| Acetone | 85 ml |
| Formaldehyde | 0.01 g |
| Acetic Acid | 0.01 g |
| Concentrated Hydrochloric Acid | 0.01 g |

### 3) Coating of a backing layer

An antistatic layer, a magnetic recording layer and a sliding layer each having the following composition were coated as backing layers on one side of the above undercoated support.

### 3-1) Coating of an antistatic layer

### 3-1-1) Preparation of electrically conductive fine grain dispersion solution (a composite dispersion solution of stannic oxide-antimony oxide)

230 weight parts of stannic chloride hydrate and 23 weight parts of antimony trichloride were dissolved in 3,000 weight parts of ethanol and homogeneous solution was obtained. A 1 N aqueous sodium hydroxide solution was dropwise added to the above solution until the pH of the solution reached 3, thereby the coprecipitate of colloidal stannic oxide and antimony oxide was obtained. The thus-obtained coprecipitate was allowed to stand at 50°C for 24 hours and red brown colloidal precipitate was obtained.

The red brown colloidal precipitate was isolated by a centrifugal separator. Water was added to the precipitate and washed by centrifugation to remove excessive ion. The excessive ion was removed by repeating this operation three times.

200 weight parts of the colloidal precipitate from which the excessive ion was removed was again dispersed in 1,500 weight parts of water, atomized in a kiln heated to 650°C, thereby a bluish fine grain powder of a composite of stannic oxide-antimony oxide having an average grain size of 0.005 µm was obtained. The resistivity of this fine grain powder was 5 Ω•cm.

The pH of the mixed solution comprising 40 weight parts of the above fine grain powder and 60 weight parts of water was adjusted to 7.0. This mixed solution was dispersed coarsely by a disperser, then dispersed using a horizontal sand mill (Dyno Mill, manufactured by WILLYA. BACHOFENAG) until the residence time reached 30 minutes, thus the objective product was prepared. The average grain size of the secondary agglomerate at that time was about 0.04 µm. 3-1-2) Coating of an electrically conductive layer

An electrically conductive layer having the formulation shown below was coated so as to provide a dry film thickness of 0.2 µm and dried at 115°C for 60 seconds.

| | |
|---|---|
| Electrically Conductive Fine Grain Dispersion Solution Prepared in 3-1-1) | 20 weight parts |
| Gelatin | 2 weight part |
| Water | 27 weight parts |
| Methanol | 60 weight parts |
| p-Chlorophenol | 0.5 weight parts |
| Resorcin | 2 weight parts |
| Polyoxyethylenenonylphenyl Ether | 0.01 weight parts |

The resistance of the thus obtained electrically conductive film was 10^{8.0} (100 V), which had an excellent antistatic performance.

### 3-2) Coating of a magnetic recording layer

220 g of water and 150 g of silane coupling agent of poly(polymerization degree: 16)oxyethylenepropyl trimethoxysilane were added to 1,100 g of magnetic substance Co-adhered γ-Fe₂O₃ (acicular, major axis: 0.14 µm, minor axis: 0.03 µm, specific surface area: 41 m²/g, saturation magnetization: 89 emu/g, the surface comprised aluminum oxide and silicon oxide and each was surface treated with 2 wt% of Fe₂O₃, coercive force: 930 Oe, Fe⁺²/Fe⁺³ ratio: 6/94) and they were well kneaded in an open kneader for 3 hours. The thus coarsely dispersed viscous liquid was dried at 70°C for a whole day and night and, after removal of water, heated at 110°C for 1 hour and the surface-treated magnetic grains were obtained.

The above grains were kneaded again in an open kneader according to the following formulation.

| | |
|---|---|
| The Above Surface-Treated Magnetic Grains | 1,000 g |
| Diacetyl Cellulose | 17 g |
| Methyl Ethyl Ketone | 100 g |
| Cyclohexanone | 100 g |

The above product was finely dispersed using a sand mill (1/4 G) at 200 rpm for 4 hours according to the following formulation.

| | |
|---|---|
| The Above Kneaded Product | 100 g |
| Diacetyl Cellulose | 60 g |
| Methyl Ethyl Ketone | 300 g |
| Cyclohexanone | 300 g |

Further, diacetyl cellulose and trimethylolpropane addition product of 3 time mol of toluenediisocyanate as a hardening agent were added in the amount of 20 wt% based on the binder. The product was diluted with an equal amount of methyl ethyl ketone and cyclohexanone so as to obtain the solution having the viscosity of 80 cp. The solution was coated on the above electrically conductive layer with a bar coater to provide a film thickness of 1.2 µm. The amount of the magnetic substance was 0.6 g/m². Silica grains (0.3 µm) and abrasive aluminum oxide (0.5 µm) were added as a matting agent each in an amount of 10 mg/m². Drying was conducted at 115°C for 6 minutes (the rollers in the drying zone and transporting devices were all set at 115°C).

When using a blue filter at the status M of an X-light, the increment of color density of D^{B} of the magnetic recording layer was about 0.1. The saturation magnetization moment of the magnetic recording layer was 4.2 emu/m², coercive force was 923 Oe, and angular ratio was 65%.

### 3-3) Preparation of a sliding layer

The coating solution of the following formulation was coated in the solid coating amount of the compound shown below and dried at 110°C for 5 minutes to obtain a sliding layer.

| | |
|---|---|
| Diacetyl Cellulose | 25 mg/m ² |
| C₆H₁₃CH(OH)C₁₀H₂₀COOC₄₀H₈₁ (Compound a) | 6 mg/m ² |
| C₅₀H₁₀₁O(CH₂CH₂O)₁₆H (Compound b) | 9 mg/m ² |

Moreover, Compound a/Compound b (6/9) were heated at 105°C and dissolved in a solvent of xylene and propyleneglycol monomethyl ether (volume ratio: 1/1), and this solution was poured to propyleneglycol monomethyl ether (25°C) of 10 times the volume and finely dispersed. This fine dispersion solution was further diluted with acetone of 5 times the volume, again dispersed using a high pressure homogenizer (200 atm) and the thus-obtained dispersion (average grain size: 0.01 µm) was added.

The sliding layer obtained had excellent characteristics such as a dynamic friction coefficient of 0.06 (stainless steel ball of 5 mmφ, load: 100 g, speed: 6 cm/min) and a static friction coefficient of 0.07 (by a clip method). The sliding characteristic with the emulsion surface described below showed a dynamic friction coefficient of 0.12.

### 4) Coating of a photographic material

Each layer having the following composition was multilayer coated on the opposite side of the backing layer obtained above, thus a color negative film was prepared. This was designated as Sample 301.

The main components for use in each layer are classified as follows:
- ExC:: Cyan Coupler
- ExM:: Magenta Coupler
- ExY:: Yellow Coupler
- ExS:: Sensitizing Dye
- UV:: Ultraviolet Absorbing Agent
- HBS:: High Boiling Point Organic Solvent
- H:: Hardening Agent for Gelatin

The numeral corresponding to each component indicates the coated weight in unit of g/m², and the coated weight of silver halide is shown as the calculated weight of silver. Further, in the case of a sensitizing dye, the coated weight is indicated in unit of mol per mol of the silver halide in the same layer.

### Sample 201

| First Layer: Antihalation Layer | |
|---|---|
| Black Colloidal Silver | 0.09 as silver |
| Gelatin | 1.60 |
| ExM-1 | 0.12 |
| ExF-1 | 2.0 × 10⁻³ |
| Solid Dispersion Dye ExF-2 | 0.030 |
| Solid Dispersion Dye ExF-3 | 0.040 |
| HBS-1 | 0.15 |
| HBS-2 | 0.02 |

| Second Layer: Interlayer | |
|---|---|
| Silver Iodobromide Emulsion M | 0.065 as silver |
| ExC-2 | 0.04 |
| Polyethyl Acrylate Latex | 0.20 |
| Gelatin | 1.04 |

| Third Layer: Low Sensitivity Red-Sensitive Emulsion Layer | |
|---|---|
| Silver Iodobromide Emulsion A | 0.25 as silver |
| Silver Iodobromide Emulsion B | 0.25 as silver |
| ExS-1 | 6.9 × 10⁻⁵ |
| ExS-2 | 1.8 × 10⁻⁵ |
| ExS-3 | 3.1 × 10⁻⁴ |
| ExC-1 | 0.17 |
| ExC-3 | 0.030 |
| ExC-4 | 0.10 |
| ExC-5 | 0.020 |
| ExC-6 | 0.010 |
| Cpd-2 | 0.025 |
| HBS-1 | 0.10 |
| Gelatin | 0.87 |

| Fourth Layer: Middle Sensitivity Red-Sensitive Emulsion Layer | |
|---|---|
| Silver Iodobromide Emulsion C | 0.70 as silver |
| ExS-1 | 3.5 × 10⁻⁴ |
| ExS-2 | 1.6 × 10⁻⁵ |
| ExS-3 | 5.1 × 10⁻⁴ |
| ExC-1 | 0.13 |
| ExC-2 | 0.060 |
| ExC-3 | 0.0070 |
| ExC-4 | 0.090 |
| ExC-5 | 0.015 |
| ExC-6 | 0.0070 |
| Cpd-2 | 0.023 |
| HBS-1 | 0.10 |
| Gelatin | 0.75 |

| Fifth Layer: High Sensitivity Red-Sensitive Emulsion Layer | |
|---|---|
| Silver Iodobromide Emulsion D | 1.40 as silver |
| ExS-1 | 2.4 × 10⁻⁴ |
| ExS-2 | 1.0 × 10⁻⁴ |
| ExS-3 | 3.4 × 10⁻⁴ |
| ExC-1 | 0.10 |
| ExC-3 | 0.045 |
| ExC-6 | 0.020 |
| ExC-7 | 0.010 |
| Cpd-2 | 0.050 |
| HBS-1 | 0.22 |
| HBS-2 | 0.050 |
| Gelatin | 1.10 |

| Sixth Layer: Interlayer | |
|---|---|
| Cpd-1 | 0.090 |
| Solid Dispersion Dye ExF-4 | 0.030 |
| HBS-1 | 0.050 |
| Polyethyl Acrylate Latex | 0.15 |
| Gelatin | 1.10 |

| Seventh Layer: Low Sensitivity Green-Sensitive Emulsion Layer | |
|---|---|
| Silver Iodobromide Emulsion E | 0.15 as silver |
| Silver Iodobromide Emulsion F | 0.10 as silver |
| Silver Iodobromide Emulsion G | 0.10 as silver |
| ExS-4 | 3.0 × 10⁻⁵ |
| ExS-5 | 2.1 × 10⁻⁴ |
| ExS-6 | 8.0 × 10⁻⁴ |
| ExM-2 | 0.33 |
| ExM-3 | 0.086 |
| ExY-1 | 0.015 |
| HBS-1 | 0.30 |
| HBS-3 | 0.010 |
| Gelatin | 0.73 |

| Eighth Layer: Middle Sensitivity Green-Sensitive Emulsion Layer | |
|---|---|
| Silver Iodobromide Emulsion H | 0.80 as silver |
| ExS-4 | 3.2 × 10⁻⁵ |
| ExS-5 | 2.2 × 10⁻⁴ |
| ExS-6 | 8.4 × 10⁻⁴ |
| ExC-8 | 0.010 |
| ExM-2 | 0.10 |
| ExM-3 | 0.025 |
| ExY-1 | 0.018 |
| ExY-4 | 0.010 |
| ExY-5 | 0.040 |
| HBS-1 | 0.13 |
| HBS-3 | 4.0 × 10⁻³ |
| Gelatin | 0.80 |

| Ninth Layer: High Sensitivity Green-Sensitive Emulsion Layer | |
|---|---|
| Silver Iodobromide Emulsion I | 1.25 as silver |
| ExS-4 | 3.7 × 10⁻⁵ |
| ExS-5 | 8.1 × 10⁻⁵ |
| ExS-6 | 3.2 × 10⁻⁴ |
| ExC-1 | 0.005 |
| ExC-6 | 0.005 |
| ExM-1 | 0.020 |
| ExM-4 | 0.025 |
| ExM-5 | 0.040 |
| Cpd-3 | 0.040 |
| HBS-1 | 0.25 |
| Polyethyl Acrylate Latex | 0.15 |
| Gelatin | 1.33 |

| Tenth Layer: Yellow Filter Layer | |
|---|---|
| Yellow Colloidal Silver | 0.015 as silver |
| Cpd-1 | 0.16 |
| Solid Dispersion Dye ExF-5 | 0.060 |
| Solid Dispersion Dye ExF-6 | 0.060 |
| Oil-Soluble Dye ExF-7 | 0.010 |
| HBS-1 | 0.60 |
| Gelatin | 0.60 |

| Eleventh Layer: Low Sensitivity Blue-Sensitive Emulsion Layer | |
|---|---|
| Silver Iodobromide Emulsion J | 0.09 as silver |
| Silver Iodobromide Emulsion K | 0.09 as silver |
| ExS-7 | 8.6 × 10⁻⁴ |
| ExC-8 | 7.0 × 10⁻³ |
| ExY-1 | 0.050 |
| ExY-2 | 0.22 |
| ExY-3 | 0.50 |
| ExY-4 | 0.020 |
| Cpd-2 | 0.10 |
| Cpd-3 | 4.0 × 10⁻³ |
| HBS-1 | 0.28 |
| Gelatin | 1.20 |

| Twelfth Layer: High Sensitivity Blue-Sensitive Emulsion Layer | |
|---|---|
| Silver Iodobromide Emulsion L | 1.00 as silver |
| ExS-7 | 4.0 × 10⁻⁴ |
| ExY-2 | 0.10 |
| ExY-3 | 0.10 |
| ExY-4 | 0.010 |
| Cpd-2 | 0.10 |
| Cpd-3 | 1.0 × 10⁻³ |
| HBS-1 | 0.070 |
| Gelatin | 0.70 |

| Thirteenth Layer: First Protective Layer | |
|---|---|
| UV-1 | 0.19 |
| UV-2 | 0.075 |
| UV-3 | 0.065 |
| HBS-1 | 5.0 × 10⁻² |
| HBS-4 | 5.0 × 10⁻² |
| ExF-8 | 2.1 × 10⁻³ |
| ExF-9 | 6.3 x 10⁻³ |
| Gelatin | 1.8 |

| Fourteenth Layer: Second Protective Layer | |
|---|---|
| Silver Iodobromide Emulsion M | 0.10 as silver |
| H-1 | 0.40 |
| B-1 (diameter: 1.7 µm) | 5.0 × 10⁻² |
| B-2 (diameter: 1.7 µm) | 0.15 |
| B-3 | 0.05 |
| S-1 | 0.20 |
| Gelatin | 70 |

Further, W-1 to W-3, B-4 to B-6, F-1 to F-17, and iron salts, lead salts, gold salts, platinum salts, palladium salts, iridium salts and rhodium salts were appropriately included in each layer to improve storage stability, processing property, pressure resistance, fungicidal and biocidal properties, antistatic property and coating property.

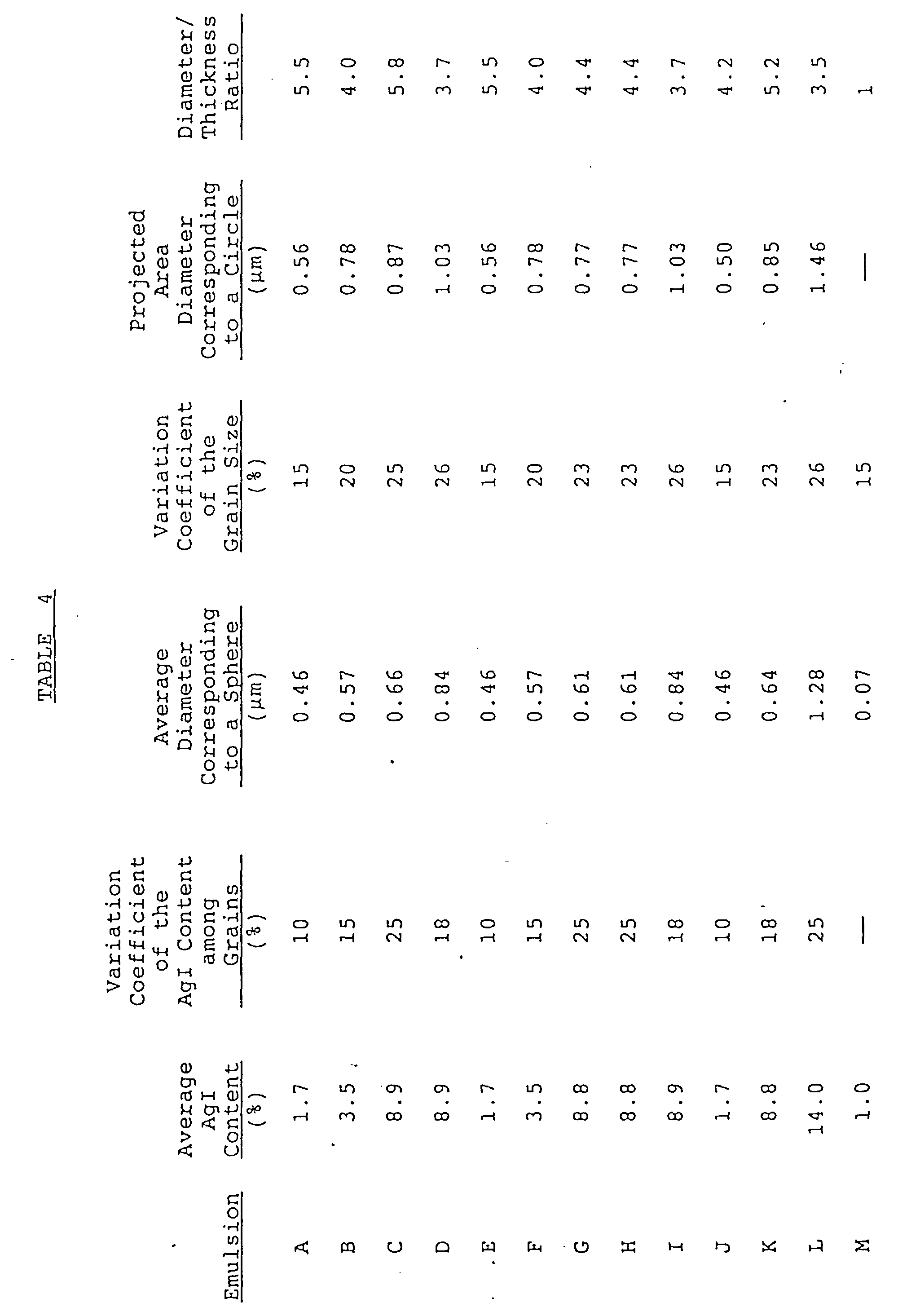

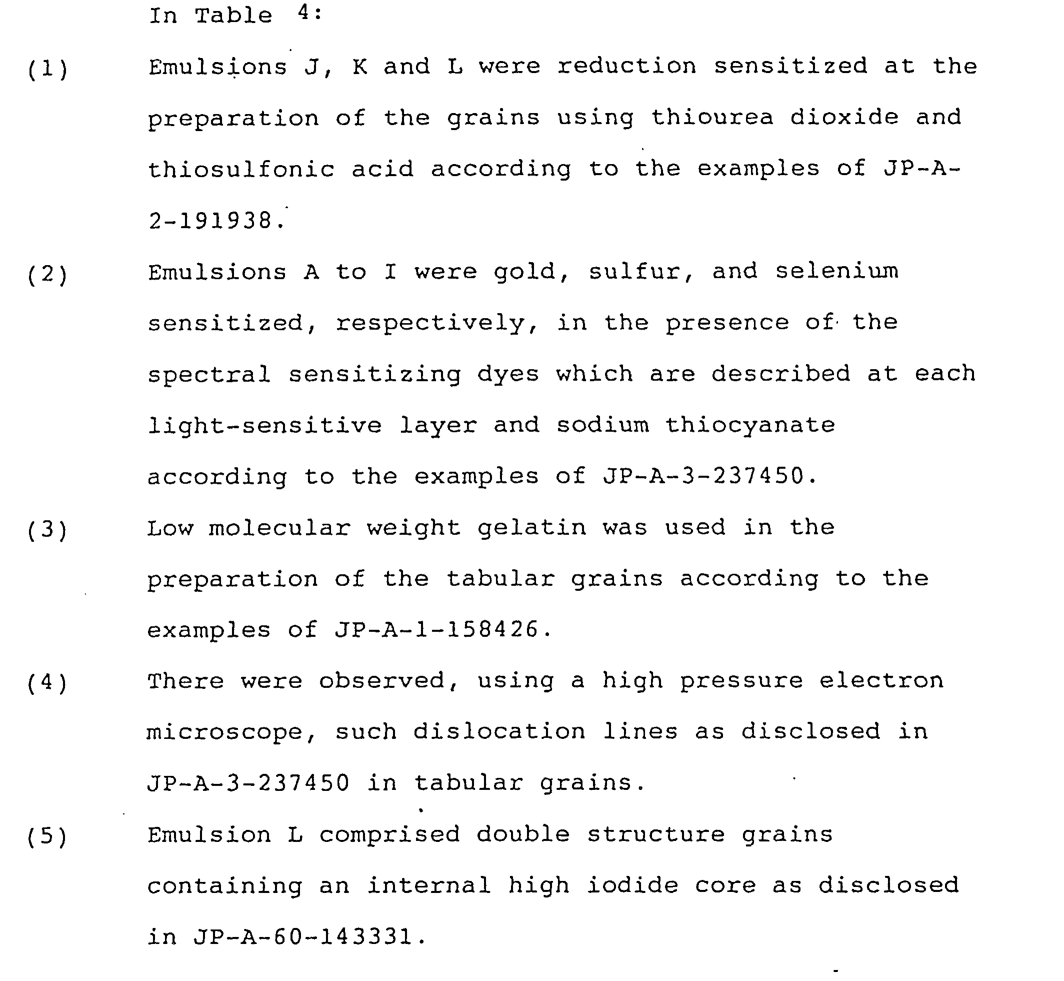

### Preparation of Organic Solid Dispersion of Dye

The ExF-2 shown below was dispersed according to the following method. That is, 21.7 ml of water, 3 ml of a 5% aqueous solution of sodium p-octylphenoxyethoxyethoxyethanesulfonate, and 0.5 g of a 5% aqueous solution of p-octylphenoxypolyoxyethylene ether (polymerization degree: 10) were placed in a pot mill having a capacity of 700 ml, and 5.0 g of Dye ExF-2 and 500 ml of zirconium oxide beads (diameter: 1 mm) were added thereto and the content was dispersed for 2 hours. The vibrating ball mill which was used was BO type ball mill manufactured by Chuo Koki. The content was taken out after dispersion and added to 8 g of a 12.5% aqueous gelatin solution and the beads was removed by filtration and the gelatin dispersion of the dye was obtained. The average grain size of fine grains of the dye was 0.44 µm.

Solid dispersions of ExF-3, ExF-4 and ExF-6 were obtained in the same manner. The average grain size of fine grains of the dye were 0.24 µm, 0.45 µm and 0.52 µm, respectively. ExF-5 was dispersed according to the microprecipitation dispersion method disclosed in Example 1 of EP 549489A. The average grain size was 0.06 µm. 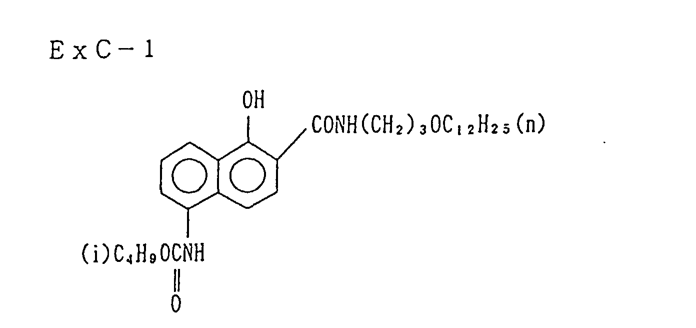 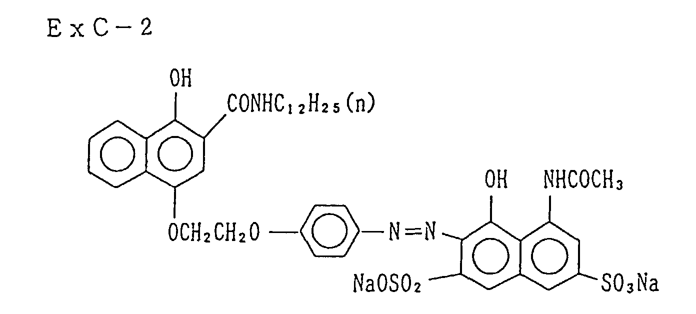 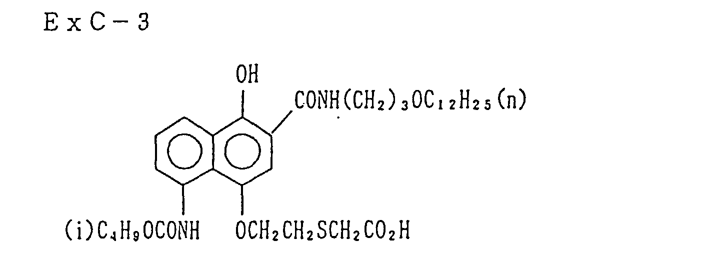 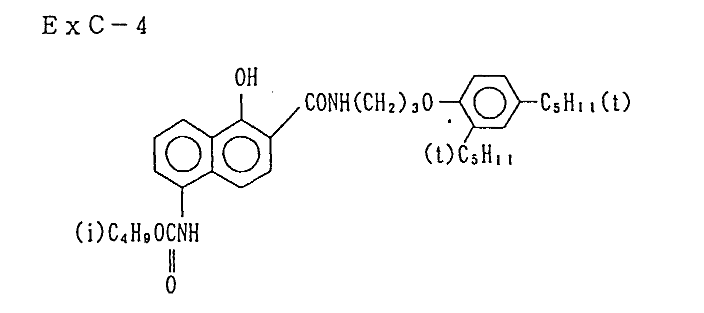 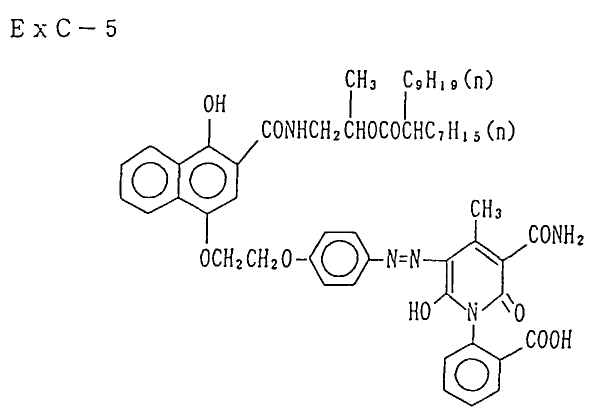 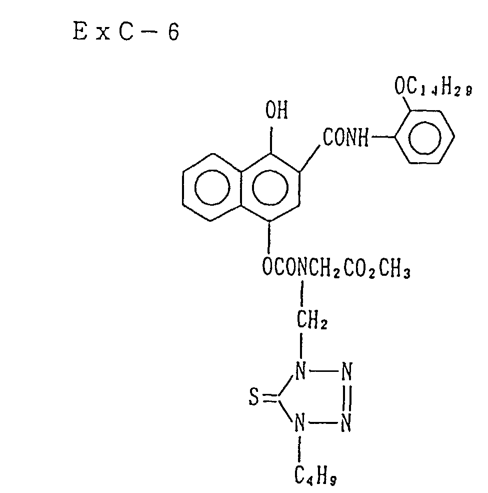 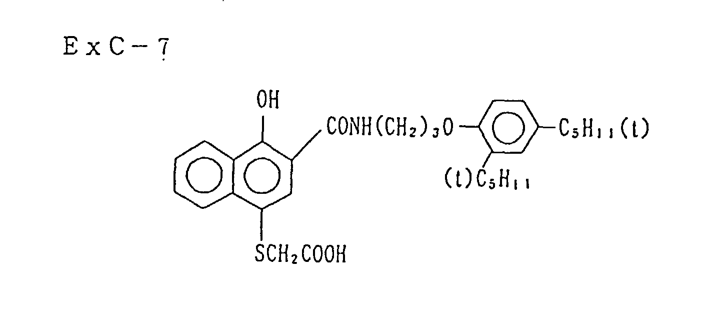 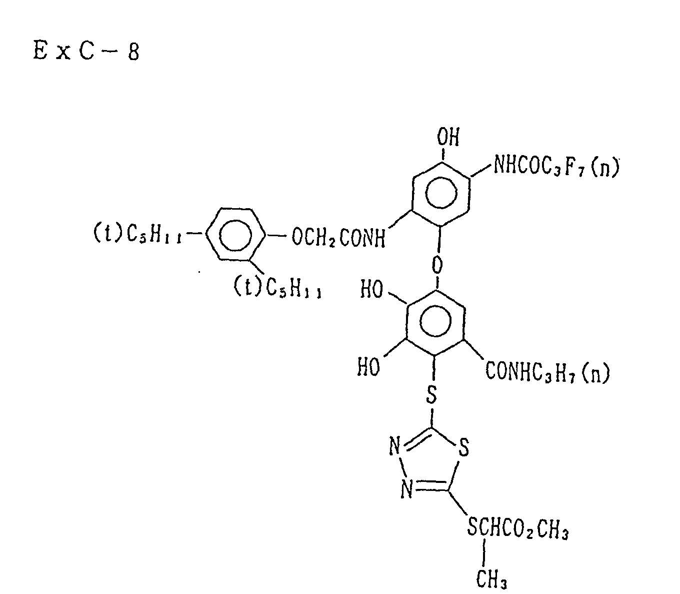 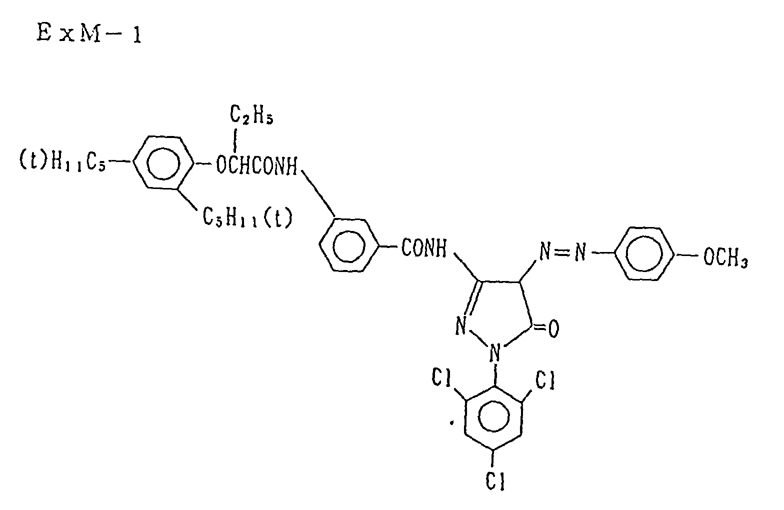 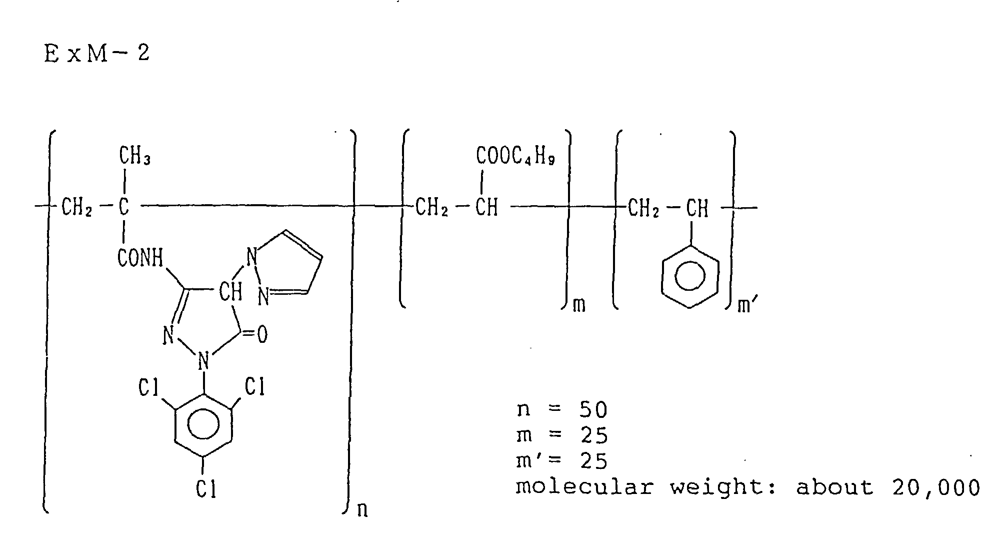 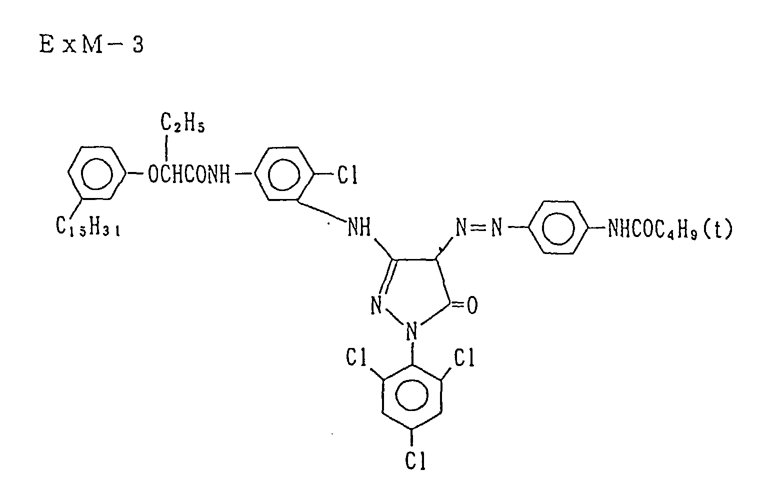 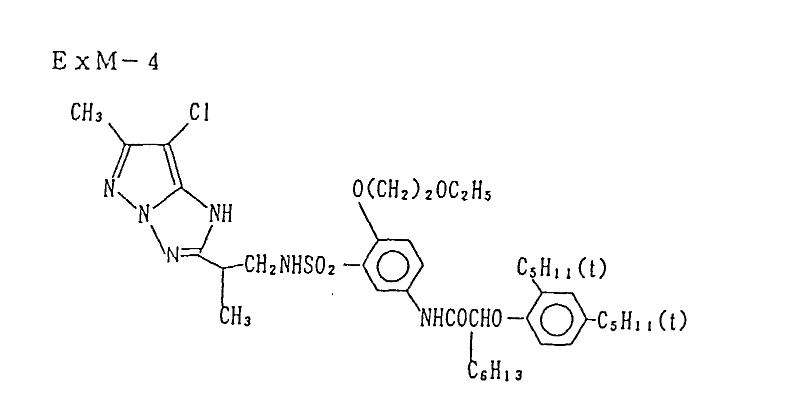 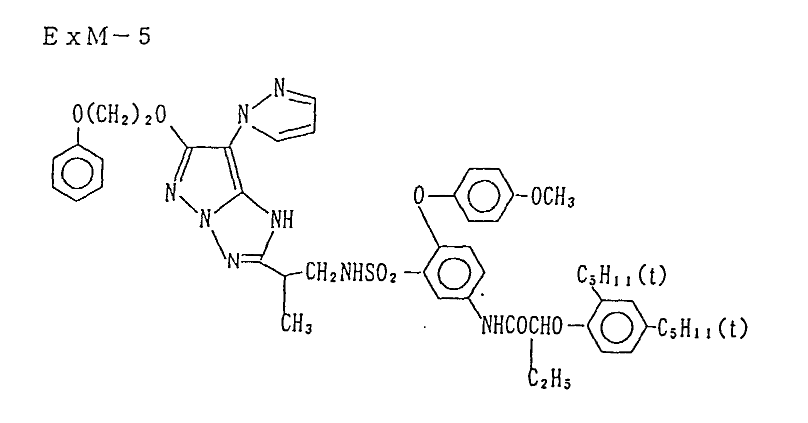 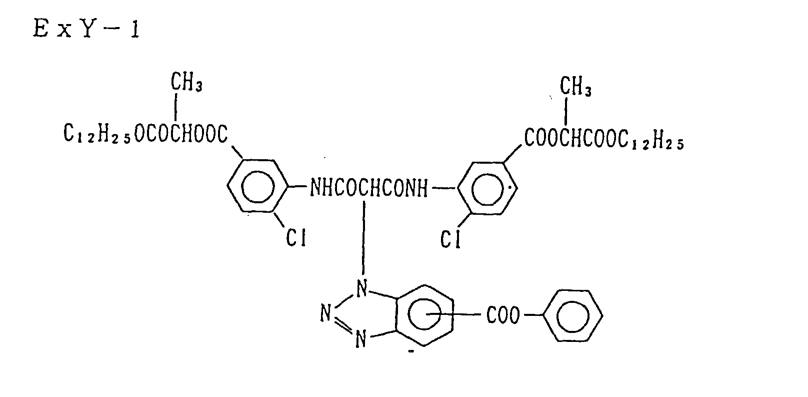 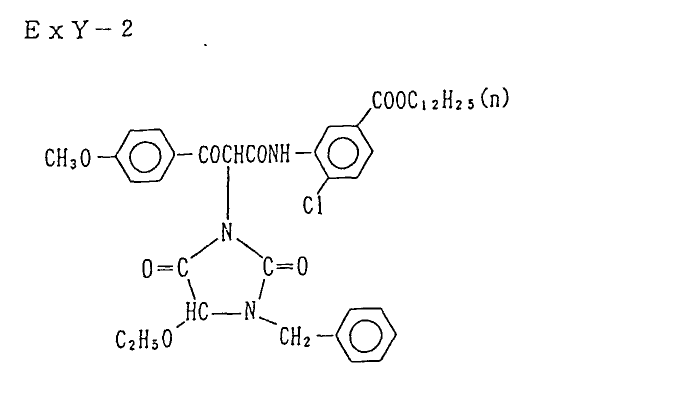 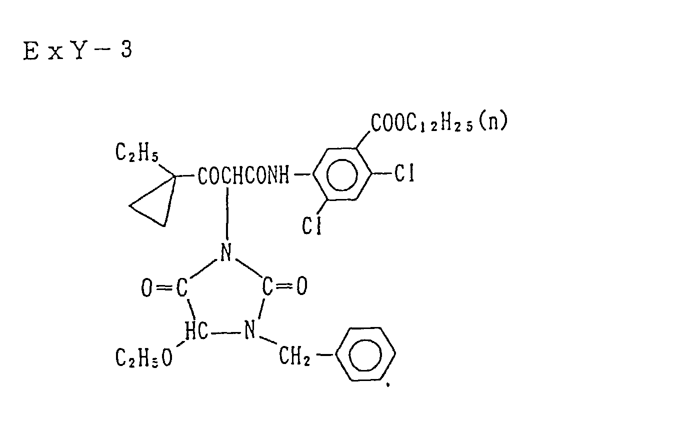 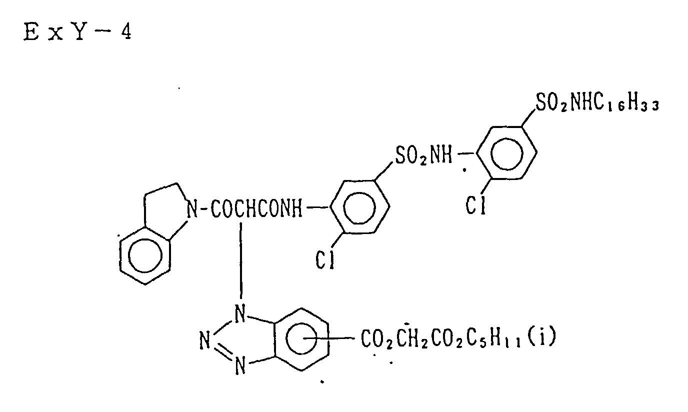 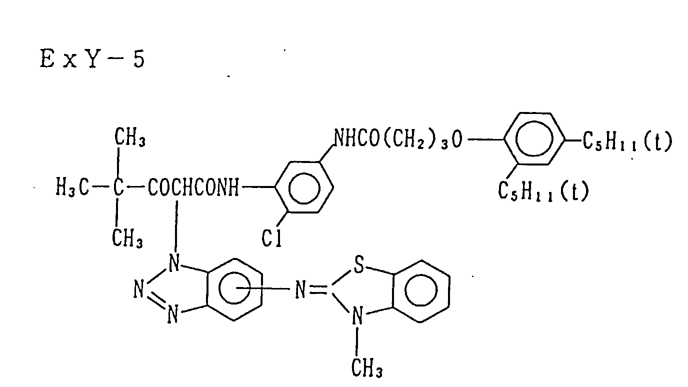 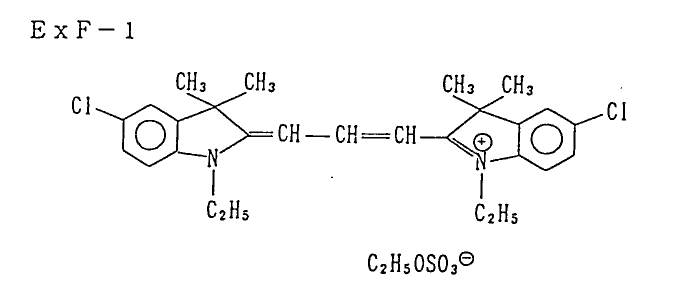 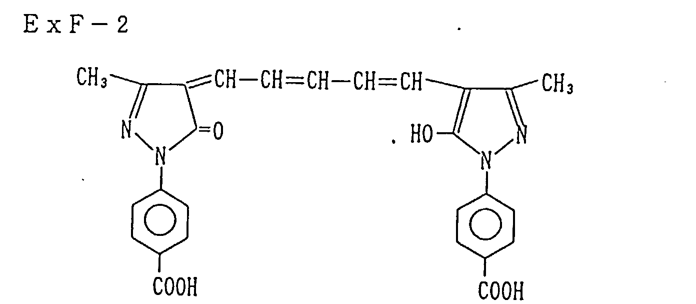 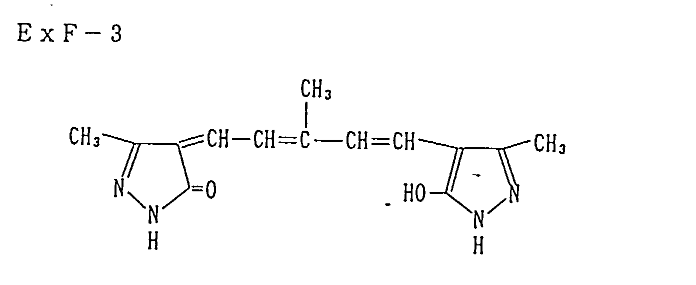 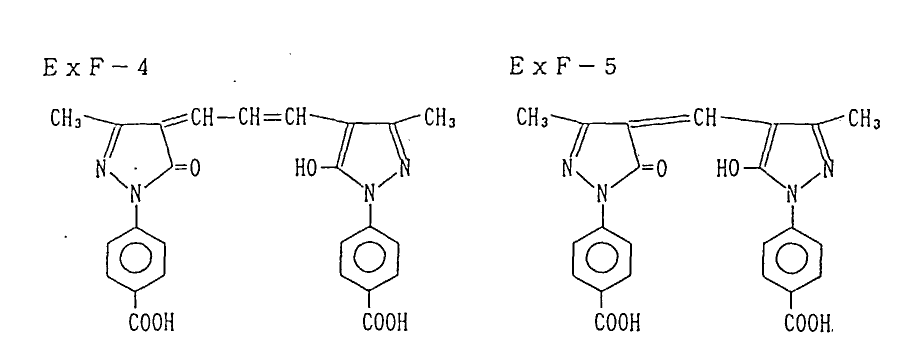 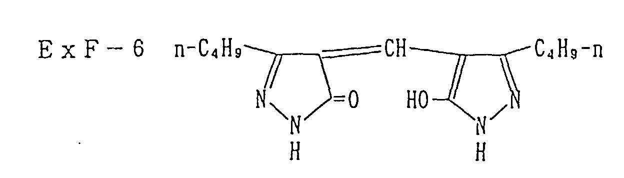 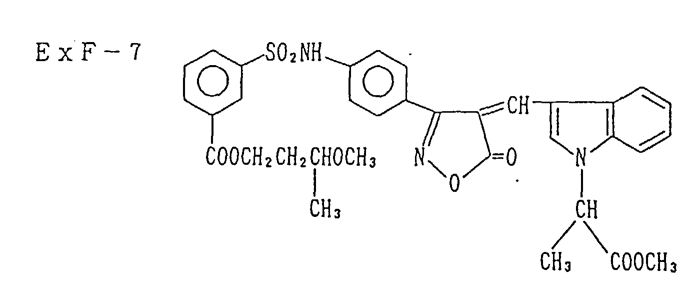 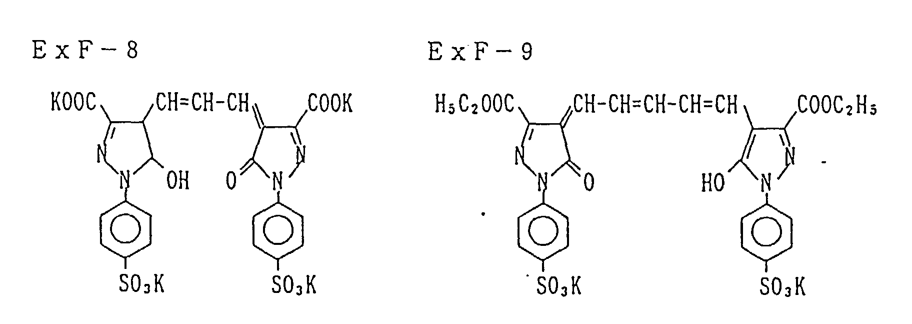 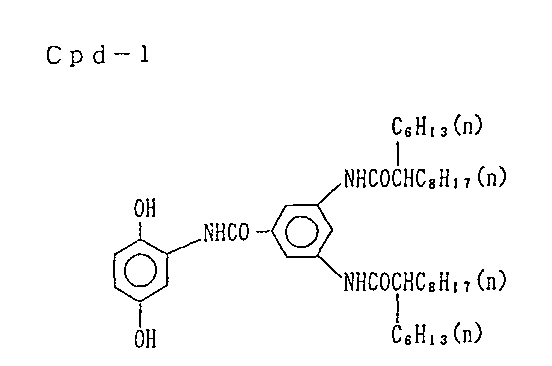 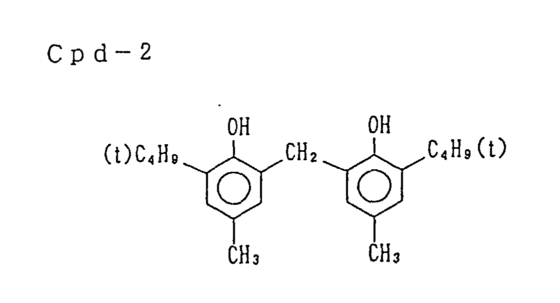 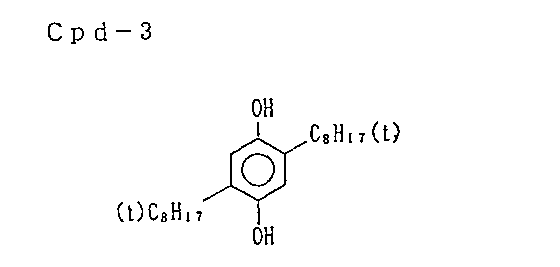 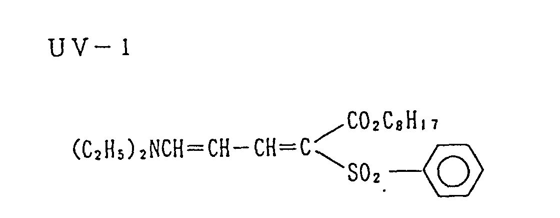 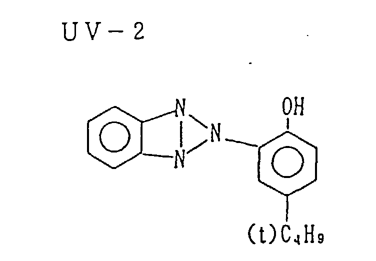 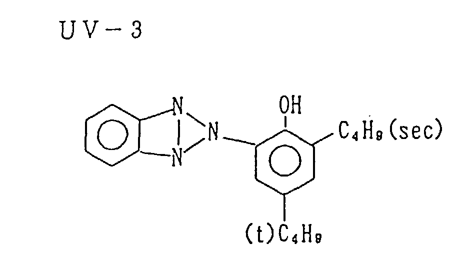
- HBS-1: Tricresyl Phosphate
- HBS-2: Di-n-butyl Phthalate

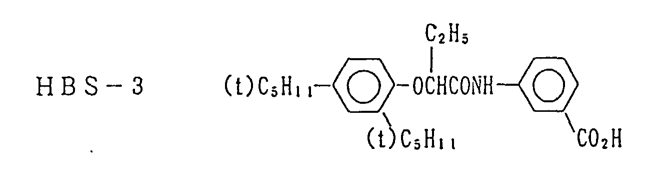

- HBS-4: Tri(2-ethylhexyl) Phosphate

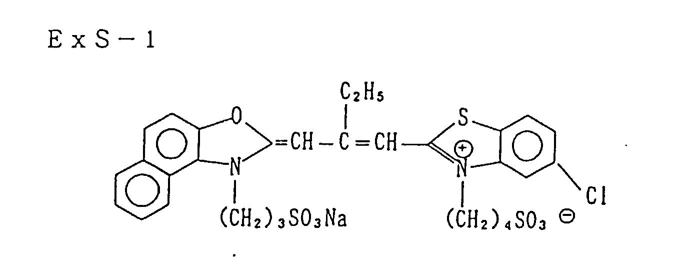

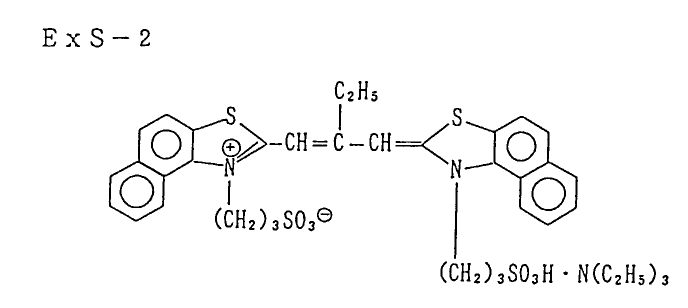

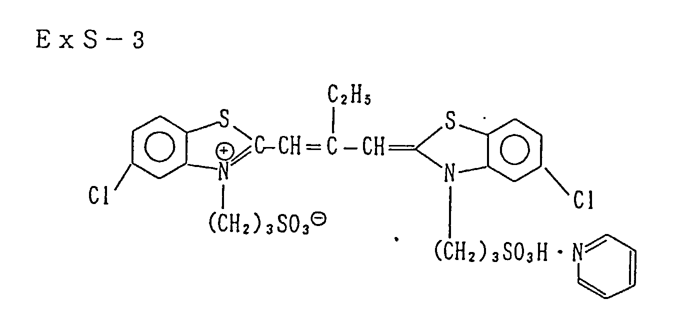

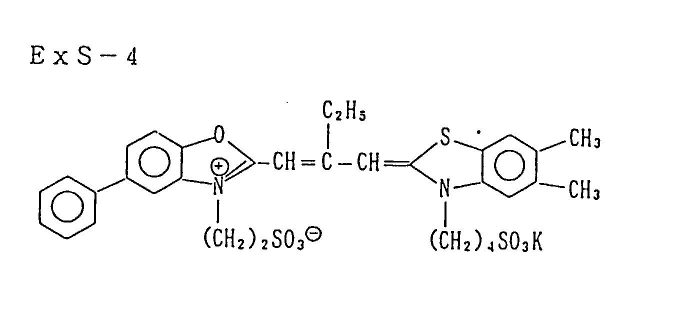

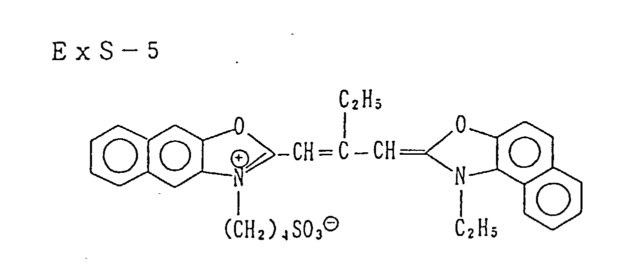

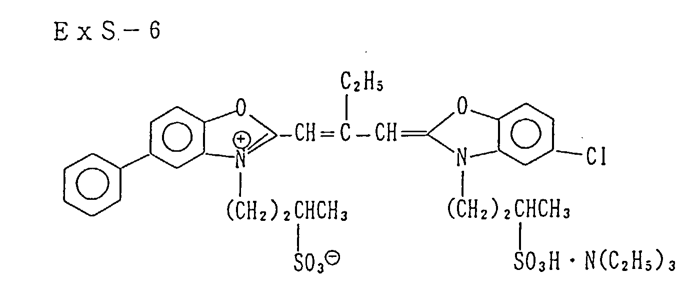

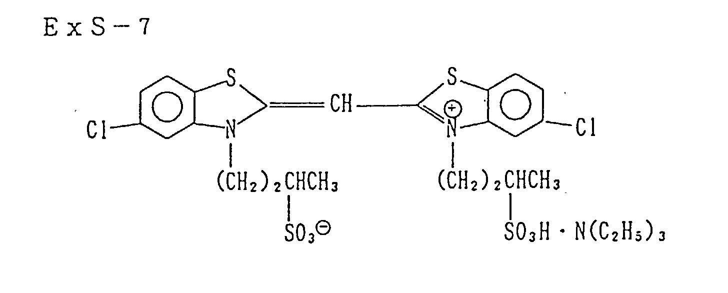

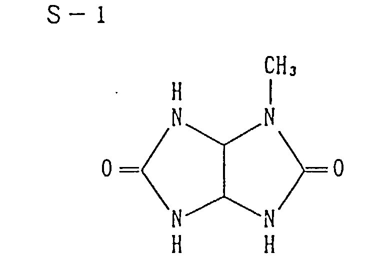

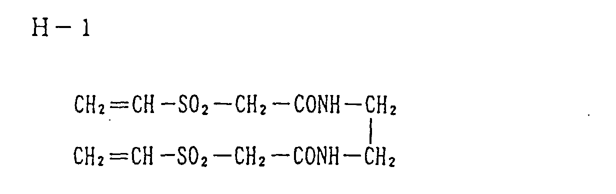

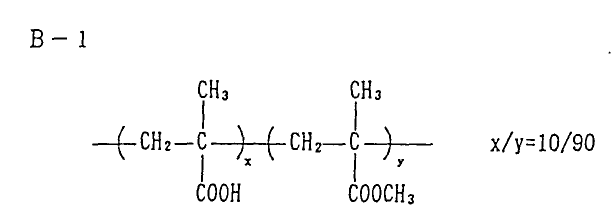

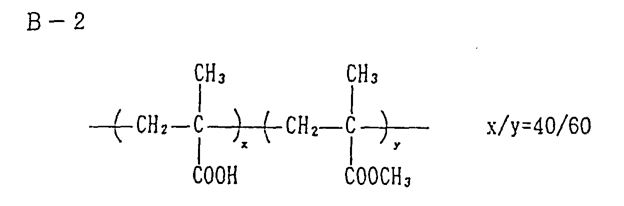

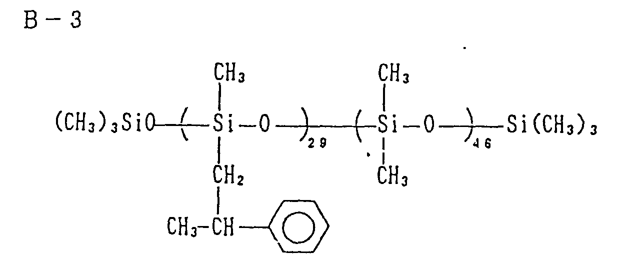

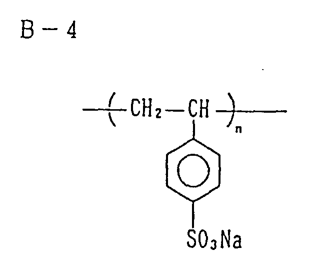

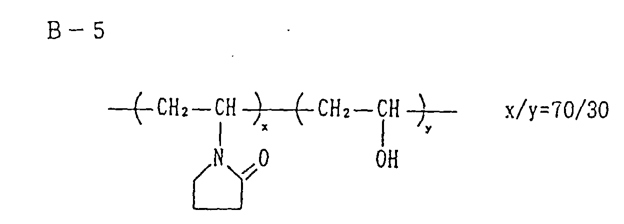

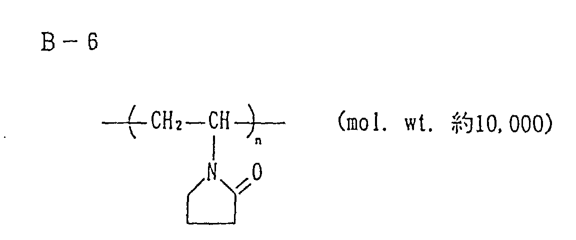

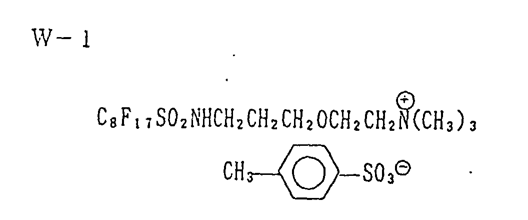

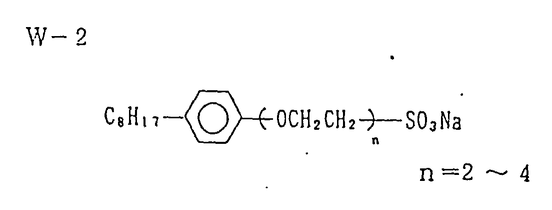

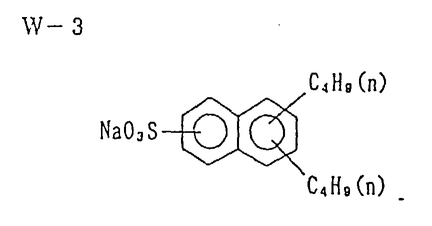

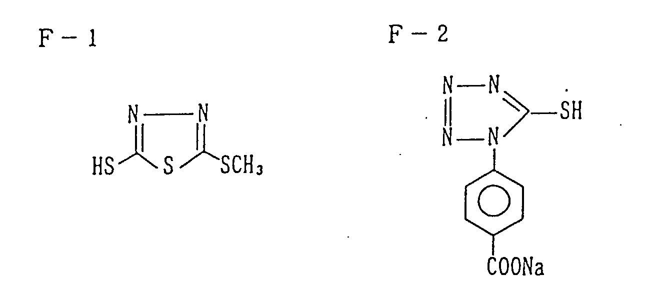

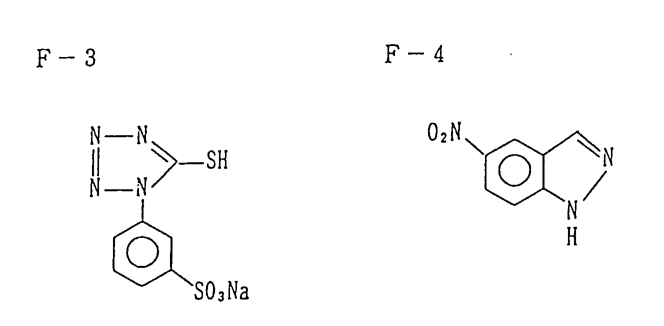

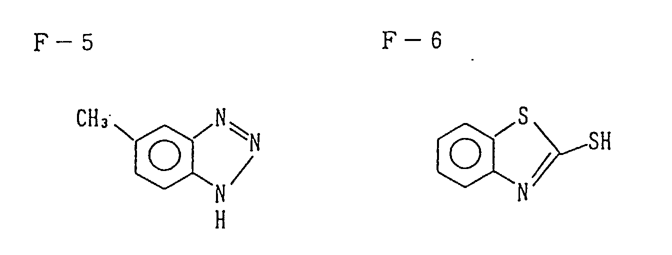

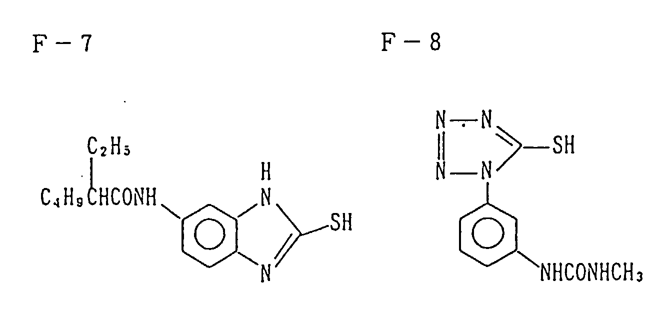

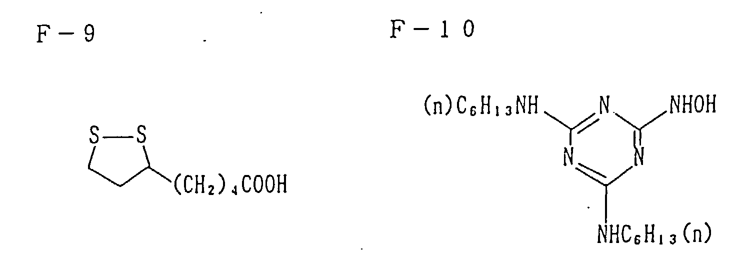

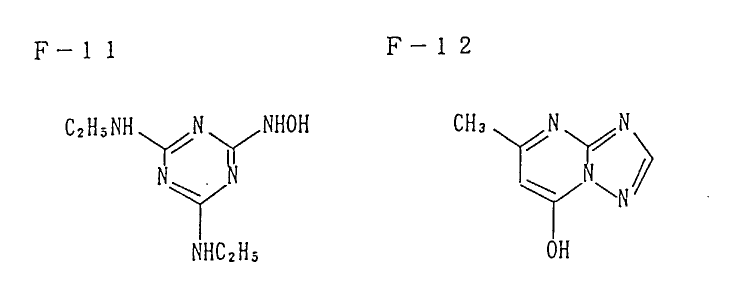

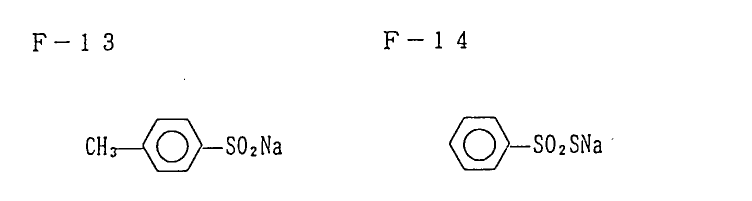

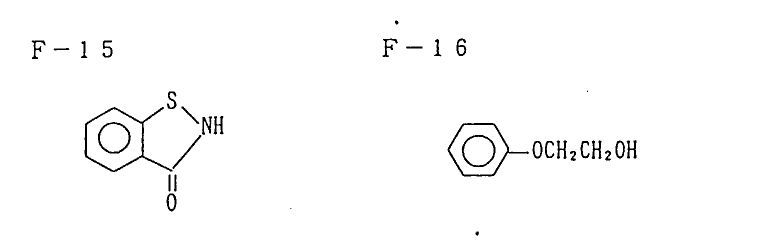

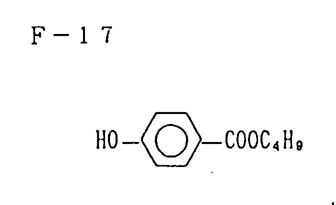

### EXAMPLE 6

A sample was prepared by adding Compounds 4, 27, 28, 58 and 107 of the present invention respectively to the fourth, fifth and sixth layers of Sample 101 in Example 1 of JP-A-6-118533 in an amount of 5 × 10⁻² mol per mol of Ag. After exposure, this sample was put under the forced aged conditions of 50°C, 60% RH for seven days. The sample was after then subjected to the color reversal process disclosed on pages 37 and 38 of JP-A-6-118533 and evaluated. The sample containing the compound of the present invention showed desired performance such as less increase of sensitivity and less reduction of the maximum color density when being left after exposure compared with the compound not containing the compound of the present invention.

### EXAMPLE 7

### Preparation of Emulsion A

A vessel in which 1 liter of water, 25 g of potassium bromide, 15 g of potassium iodide, 1.9 g of potassium thiocyanate and 24 g of gelatin was maintained at 60°C, and an aqueous solution of silver nitrate and an aqueous solution of potassium bromide were added thereto by a double jet method with vigorously stirring according to an ordinary ammonia method to thereby obtain a comparatively amorphous thick plate-shape silver iodobromide emulsion having an iodide content of 10 mol% and an average grain size of 1.0 µm. The temperature was then lowered to 35°C and soluble salts were removed by coagulating sedimentation, then the temperature was raised to 40°C, 82 g of gelatin was added thereto, and the pH and pAg were adjusted to 6.40 and 8.80, respectively, with sodium hydroxide and sodium bromide.

After the temperature was raised to 61°C, 0.95 g of 2-phenoxyethanol was added, and further 213 mg of Sensitizing Dye-A shown below was added. After 10 minutes, 1.2 mg of sodium thiosulfate pentahydrate, 28 mg of potassium thiocyanate and 0.4 mg of chloroauric acid were added, and after 65 minutes the emulsion was solidified by cooling quickly.

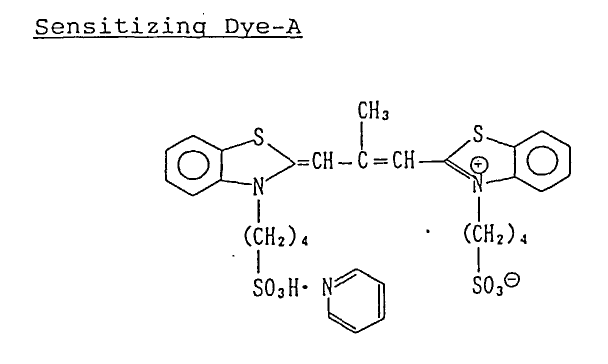

### Preparation of Emulsion B

A vessel in which 1 liter of water, 25 g of potassium bromide, 9 g of potassium iodide, 7.6 g of potassium thiocyanate and 24 g of gelatin was maintained at 40°C, and an aqueous solution of silver nitrate and an aqueous solution of potassium bromide were added thereto by a double jet method with vigorously stirring according to an ordinary ammonia method to thereby obtain a comparatively amorphous thick plate-shape silver iodobromide emulsion having an iodide content of 6 mol% and an average grain size of 0.6 µm. The temperature was then lowered to 35°C and soluble salts were removed by precipitation method, then the temperature was raised to 40°C, 110 g of gelatin was added thereto, and the pH and pAg were adjusted to 6.60 and 8.90, respectively, with sodium hydroxide sodium bromide.

After the temperature was raised to 56°C, 0.8 mg of chloroauric acid, 9 mg of potassium thiocyanate and 4 mg of sodium sulfate were added. After 55 minutes, 180 mg of Sensitizing Dye-A was added, and after 65 minutes the emulsion was solidified by cooling quickly.

### Preparation of a Coated Sample

Sample 601 was prepared according to the method disclosed in JP-A-62-115035. The emulsion coating surface of the triacetyl cellulose support was previously undercoated, and the following coating solution was coated on the back surface thereof.

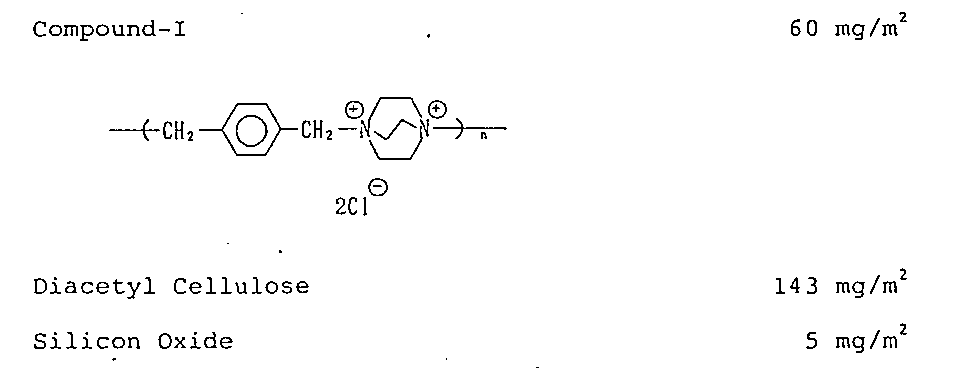

The following layers were coated to prepare Sample 601.

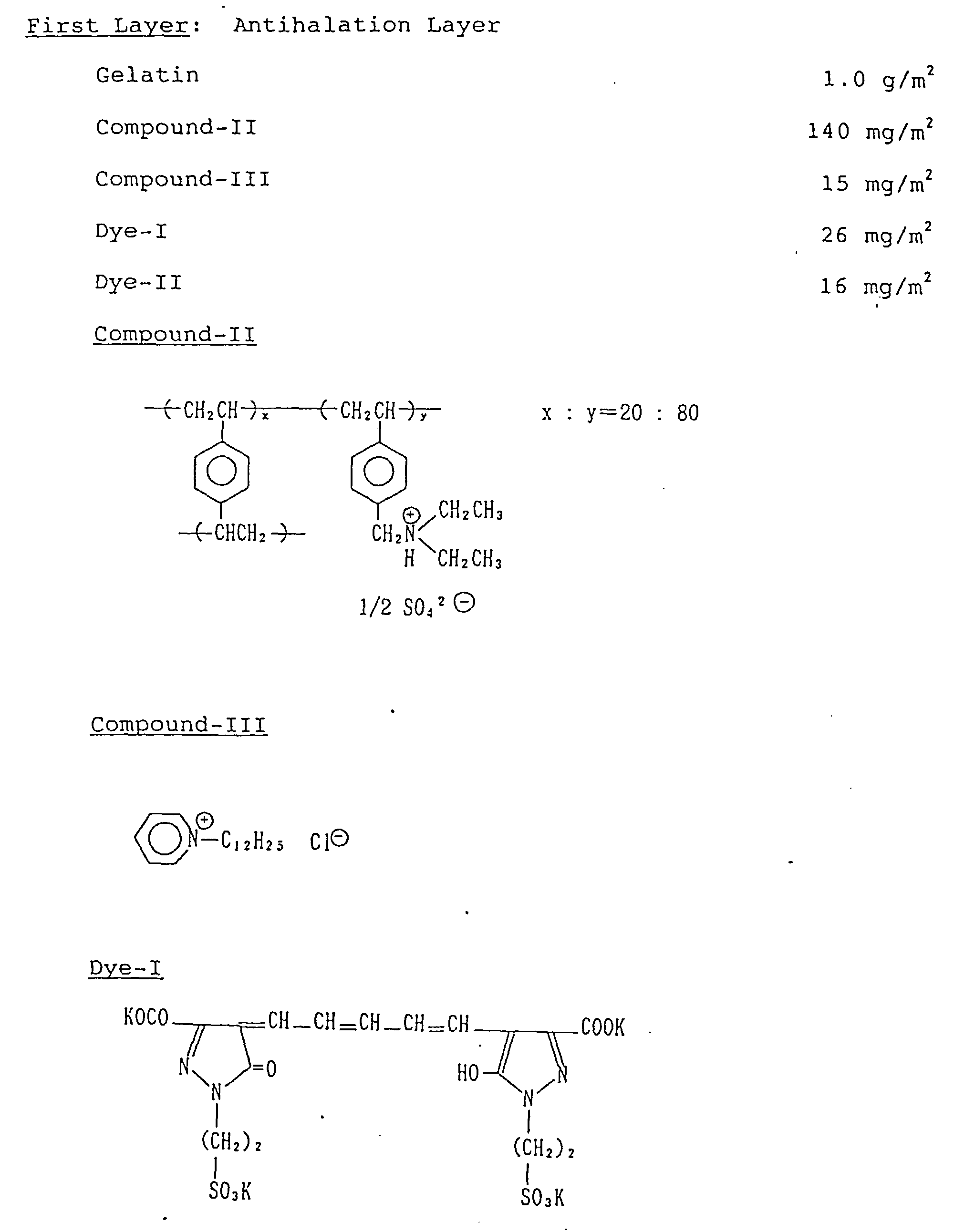

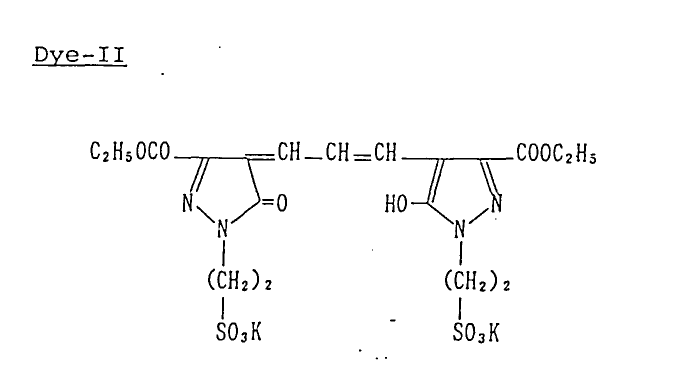

| Second Layer: Interlayer | |
|---|---|
| Gelatin | 0.4 g/m² |
| Polypotassium p-Vinylbenzenesulfonate | 5 mg/m² |

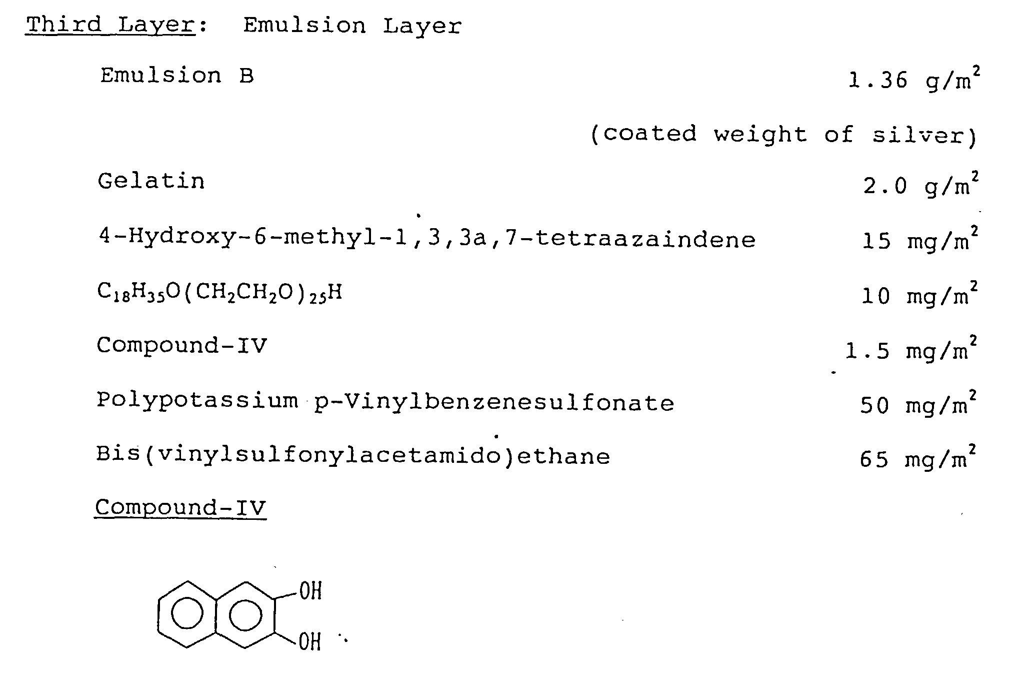

| Fourth Layer: Emulsion Layer | |
|---|---|
| Emulsion A | 4.2 g/m² |
| | coated weight of silver |
| Gelatin | 6.5 g/m² |
| Dextran (average molecular weight: 150,000) | 1.2 g/m² |
| 4-Hydroxy-6-methyl-1,3,3a,7-tetraazaindene | 41 mg/m² |
| C₁₈H₃₅O(CH₂CH₂O)₂₅H | 23 mg/m² |
| Trimethylolpropane | 500 mg/m² |
| Polypotassium p-Vinylbenzenesulfonate | 88 mg/m² |
| Polyacrylic Acid | 54 mg/m² |

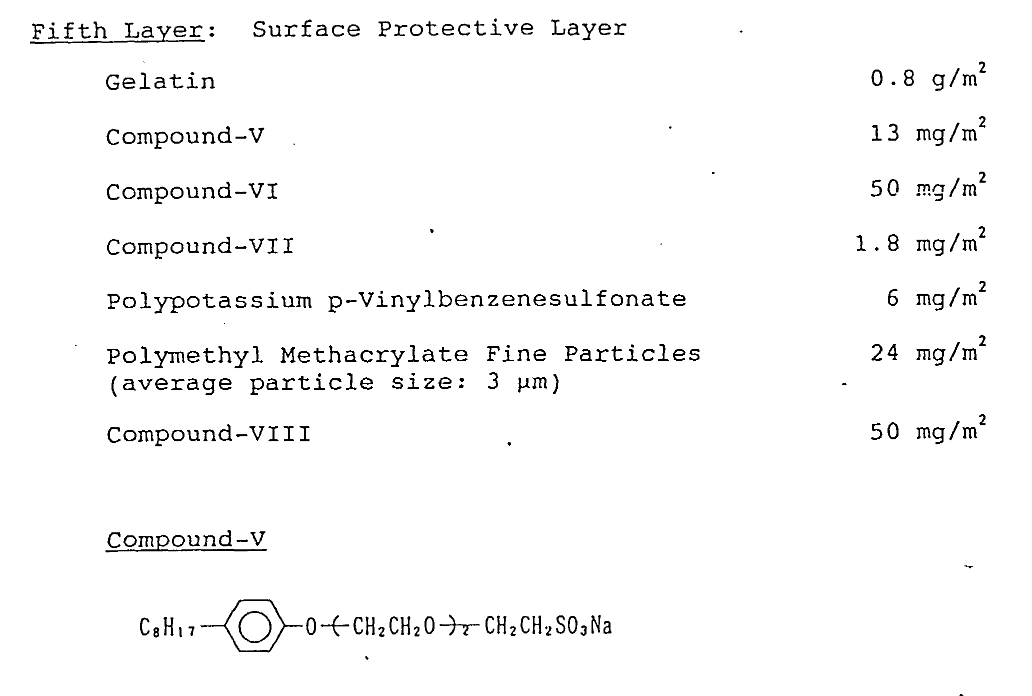

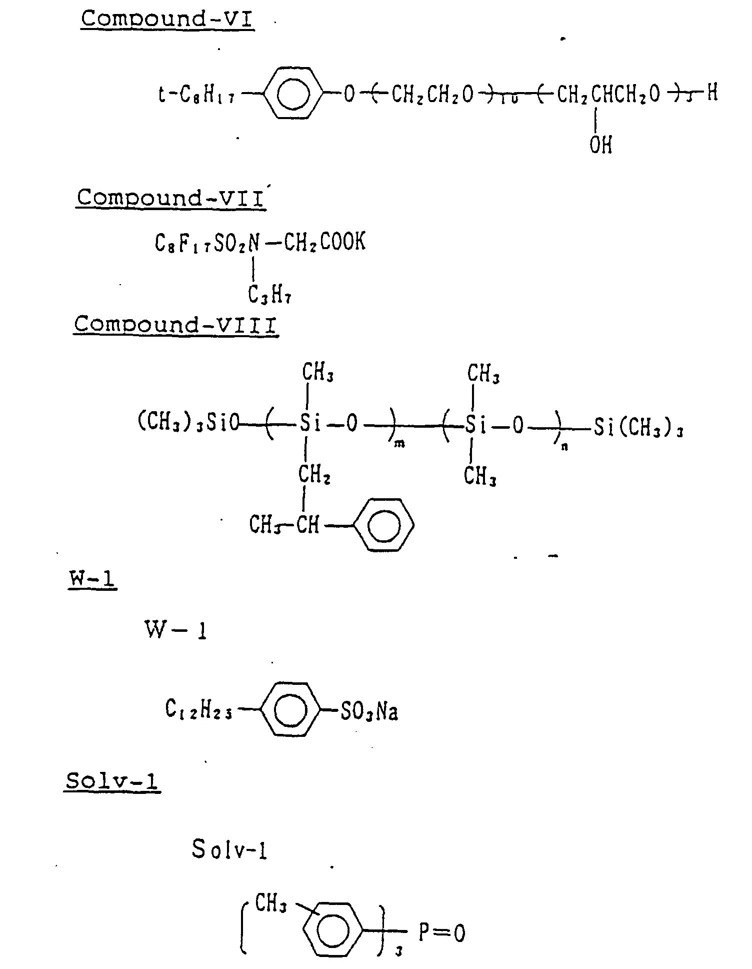

18.9 g of Compound 58 of the present invention, 19.0 g of poly-t-butyl acrylamide (molecular weight: 100,000), 9.5 g of a high boiling point organic solvent (Solv-1), 38.0 g of a surfactant (W-1) were added to 300 cc of acetic acid and dissolved by heating. This solution was added to 10% aqueous gelatin solution and dispersed in an emulsified state by a home mixer for 10 minutes.

This emulsion was added to the third and fourth layers of Sample 601 so that the amount of Compound 58 of the present invention was 0.1 mol per mol of Ag, thus Sample 603 was prepared. That which excluded Compound 58 of the present invention from the emulsion of Sample 603 was designated Sample 602, and that which used Compound 57 in place of Compound 58 of the present invention was designated Sample 604.

These samples were stored under 30°C, 65% RH conditions for 14 days after coating. Each sample was subjected to the following test.

### (1) Measurement of sensitivity

Each sample was exposed for 1/100 sec through an optical wedge. A tungsten light source having color temperature of 2,854°K was modified by a color temperature modifying filter to 5,400°K and optical wedge exposure was carried out using this light source.

Each sample was development, fixing, washing and drying processed using an automatic processor. Each sample was measured for a certain density higher than fog density (optical density of 0.5) and the sensitivity and fog were obtained.

Developing conditions were as follows.

| | Processing Solution | Temperature (°C) | Time (sec) |
|---|---|---|---|
| Development | HPD | 26.5 | 55 |
| Fixing | Super Fujix DP2 | 26.5 | 76 |
| Washing | running water | 20 | 95 |
| Drying | | 50 | 69 |

The stability of the photographic property with the lapse of time when the photographic material was left as it was after photographing was evaluated according to the following method.

Samples 601 to 604 were put under the conditions of 40°C, 60% RH for 14 days after being exposed in the above described manner. They were processed and measured in the above manner. The sensitivity was expressed as a relative value to the sensitivity of the sample processed immediately after exposure. The value nearer to zero means less fluctuation of the photographic property with the lapse of time and desired.

**TABLE 5**

| Sample No. | Compound | Stability with the Lapse of Time after Photographing | |
|---|---|---|---|
| | | Change of Sensitivity | Increase of Fog |
| 601 (Comparison) | - | +0.08 | 0.05 |
| 602 (Comparison) | - | +0.09 | 0.05 |
| 603 (Invention) | 58 | +0.01 | 0.02 |
| 604 (Invention) | 57 | +0.02 | 0.02 |

It is apparently understood from Table 5 that the stability with the lapse of time after photographing of the photographic material containing the compound of the present invention is extremely improved.

## Claims

1. A compound of formula (II)': 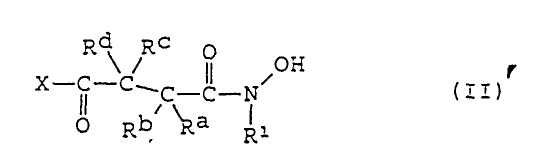 wherein R¹ is a substituted or unsubstituted alkyl group having from 1 to 6 carbon atoms; R^{a}, R^{b}, R^{c} and R^{d} are each independently a hydrogen atom, a substituted or unsubstituted alkyl group having from 1 to 18 carbon atoms, or a substituted or unsubstituted alkenyl group having from 2 to 18 carbon atoms; X is -OR⁶ or -N(R⁶) (R⁷); R⁶ is a substituted or unsubstituted alkyl group having from 6 to 22 carbon atoms, or a substituted or unsubstituted alkenyl group having from 6 to 22 carbon atoms, and R⁷ is an alkyl group having from 6 to 18 carbon atoms, provided that when all of R^{a}, R^{b}, R^{c} and R^{d} are hydrogen atoms, R⁶ and R⁷, if present, are each a substituted or unsubstituted alkyl group having 14 or more carbon atoms; and that the compound of formula (II)' is not N-methyl-3-(dioctadecylcarbamoyl) propanohydroxamic acid.

2. A compound according to Claim 1, wherein R^{a}, R^{b}, R^{c}, and R^{d} are all hydrogen atoms, R⁶ is a myristyl group, a palmityl group or a stearyl group, and R¹ is a methyl group, an ethyl group or an n-hexyl group.

3. A compound according to Claim 1, having the structural formula: 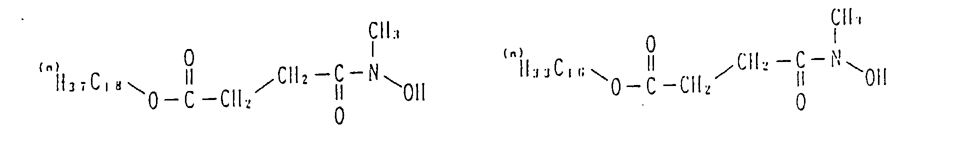 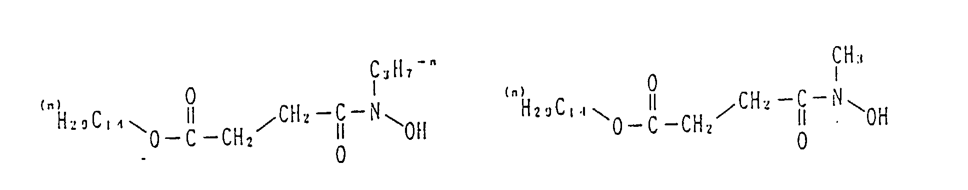 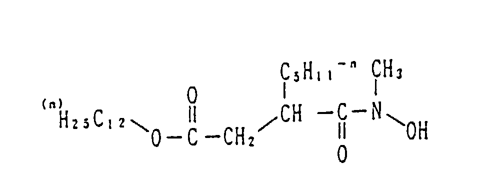 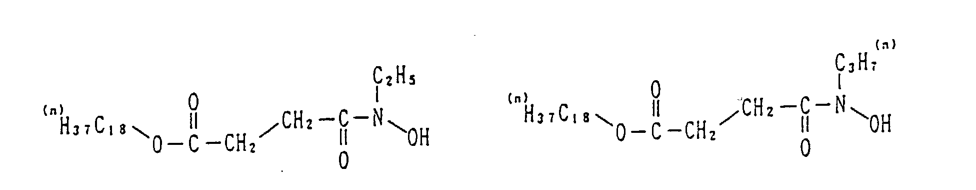 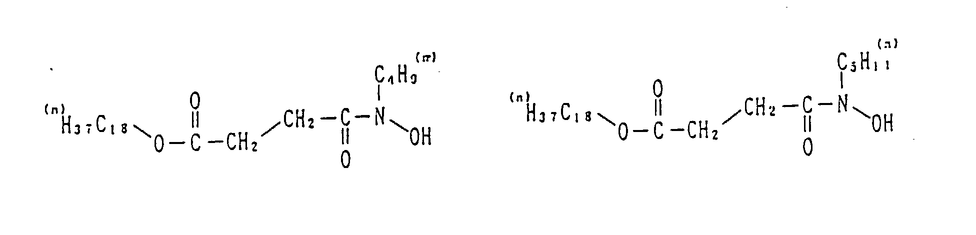 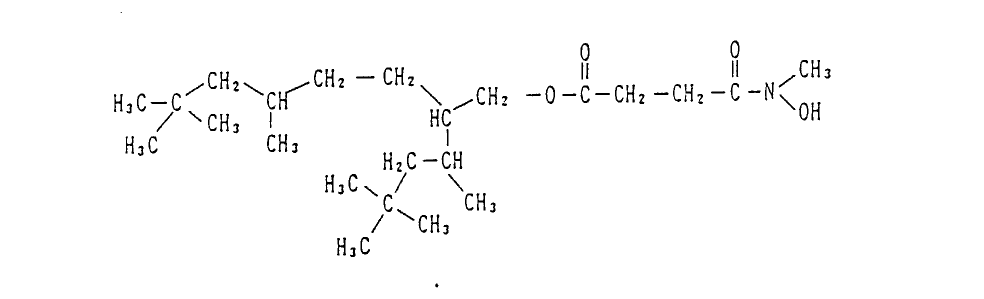 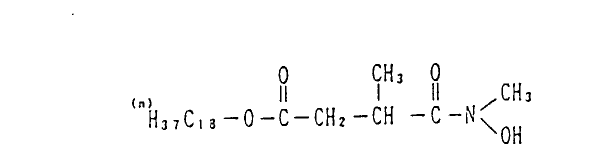 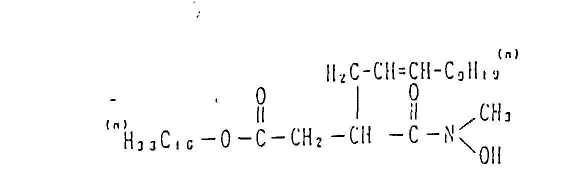 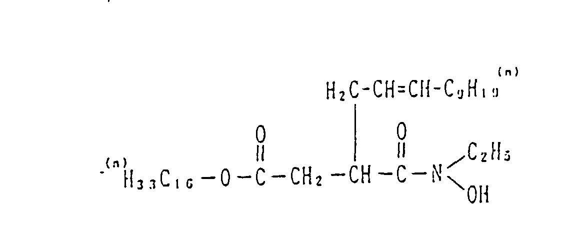 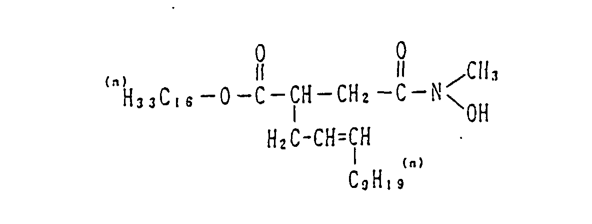 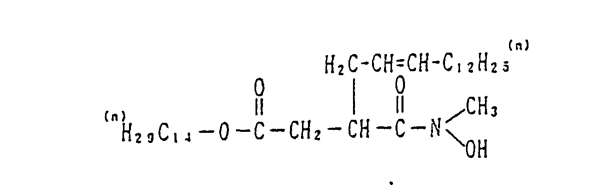 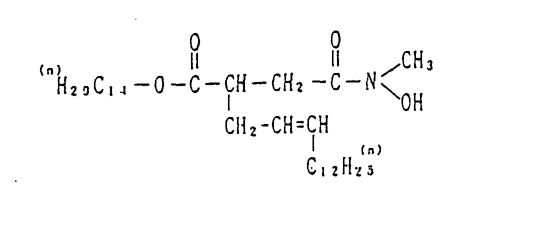 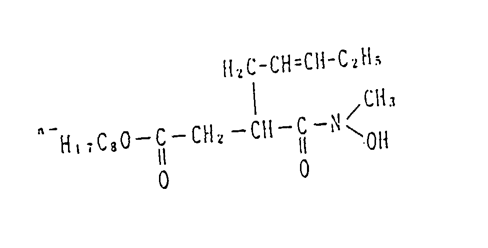 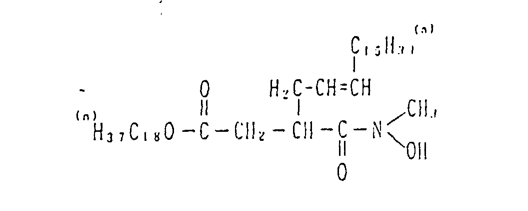 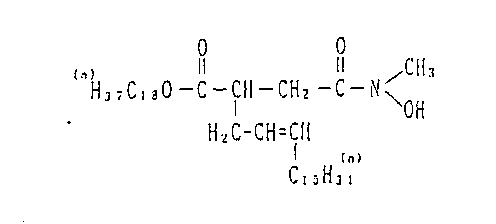 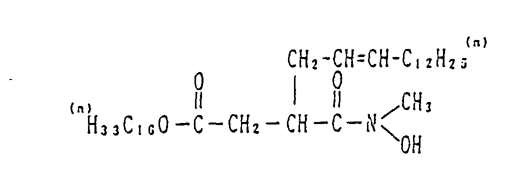 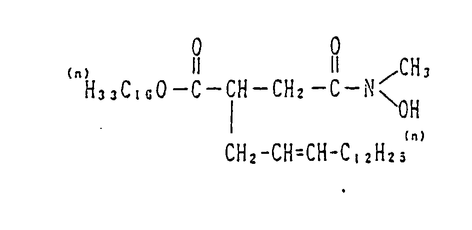 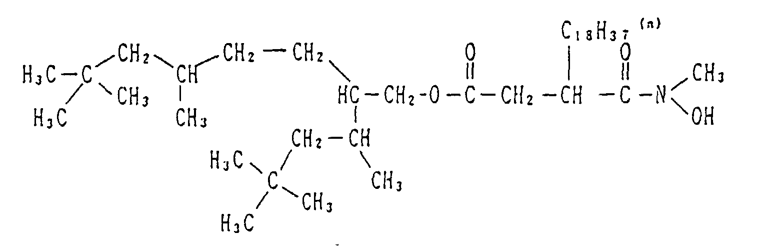 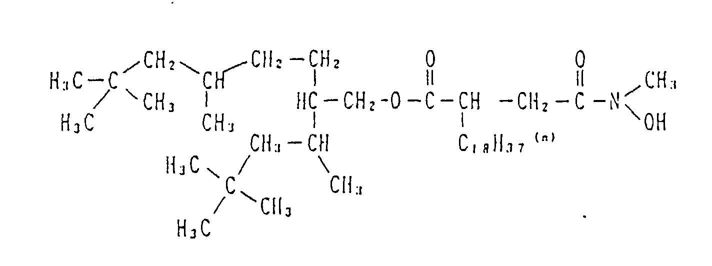 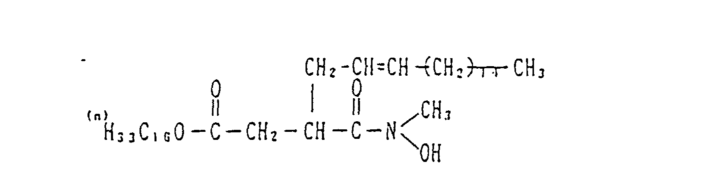 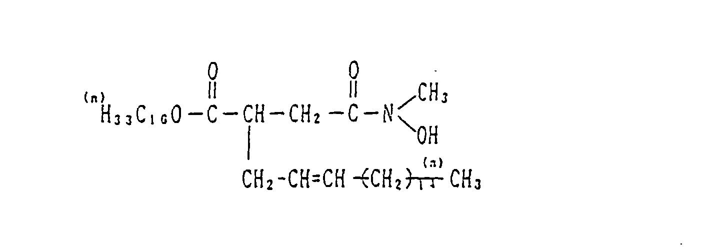 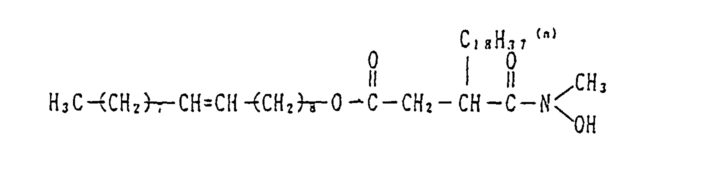 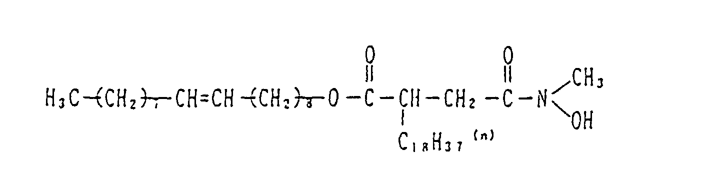 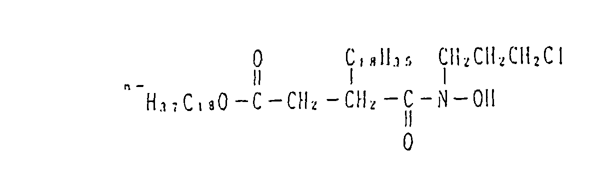 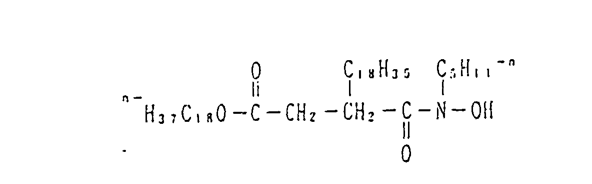 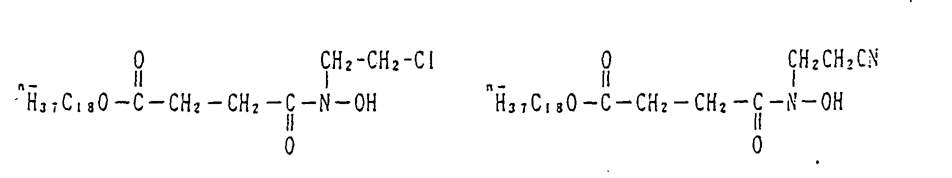 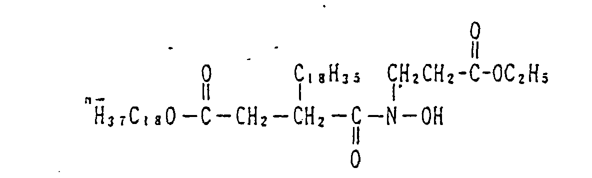 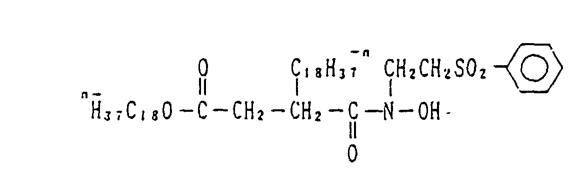 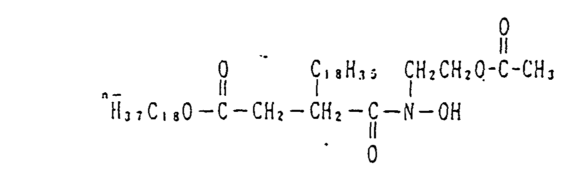 or

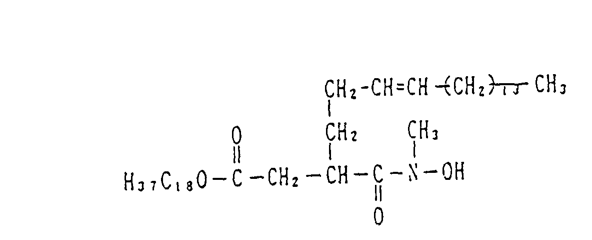

4. A silver halide photographic material comprising a support having thereon at least one light-sensitive silver halide emulsion layer, wherein said silver halide photographic material contains a compound of formula (II) : 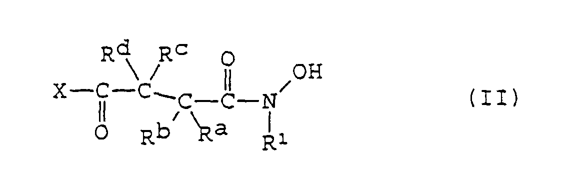 wherein R¹ is a substituted or unsubstituted alkyl group having from 1 to 6 carbon atoms; R^{a}, R^{b}, R^{c} and R^{d} are each independently a hydrogen atom, a substituted or unsubstituted alkyl group having from 1 to 18 carbon atoms, or a substituted or unsubstituted alkenyl group having from 2 to 18 carbon atoms; X is -OR⁶ or -N(R⁶) (R⁷); and R⁶ and R⁷, which may be the same or different, are each a hydrogen atom, a substituted or unsubstituted alkyl group having from 1 to 22 carbon atoms, a substituted or unsubstituted alkenyl group having from 2 to 22 carbon atoms, or an aryl group having from 6 to 22 carbon atoms.

5. Use of a compound of formula (II) for improving the storage stability of a latent image of a silver halide photographic material: 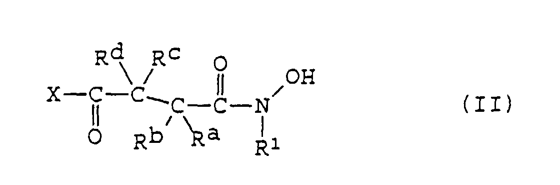 wherein R¹ is a substituted or unsubstituted alkyl group having from 1 to 6 carbon atoms; R^{a}, R^{b}, R^{c} and R^{d} are each independently a hydrogen atom, a substituted or unsubstituted alkyl group having from 1 to 18 carbon atoms, or a substituted or unsubstituted alkenyl group having from 2 to 18 carbon atoms; X is -OR⁶ or -N(R⁶) (R⁷); and R⁶ and R⁷, which may be the same or different, are each a hydrogen atom, a substituted or unsubstituted alkyl group having from 1 to 22 carbon atoms, a substituted or unsubstituted alkenyl group having from 2 to 22 carbon atoms, or an aryl group having from 6 to 22 carbon atoms.

6. Use of a compound of formula (I) for improving storage stability of a latent image of a silver halide photographic material: 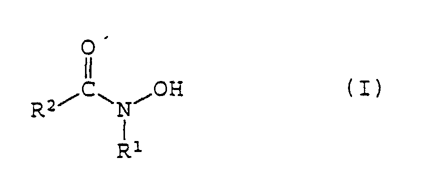 wherein R¹ is a substituted or unsubstituted alkyl group in which the alkyl moiety has from 1 to 6 carbon atoms and R² is a group of formula (I)-A:
R⁴ - L - R³- (I)-A
wherein R³ is an alkylene or alkenylene group having from 2 to 20 carbon atoms; R⁴ is a substituted or unsubstituted alkyl group having from 1 to 30 carbon atoms, a substituted or unsubstituted aryl group having from 6 to 20 carbon atoms, or a substituted or unsubstituted heterocyclic group having from 2 to 20 carbon atoms; L is -CO-, -N(R⁵), -S-, -SO₂-, -O-, -SO-, -COO-, -OCO-, -SO₂O-, -OSO₂, -SO₂N(R⁵)-, -N(R⁵)SO₂-, -N(R⁵)CO- or -CON(R⁵)-; and R⁵ is a hydrogen atom or a substituted or unsubstituted alkyl group having from 1 to 18 carbon atoms, provided that when L is -O-, R⁴ is a substituted or unsubstituted alkyl group having from 1 to 40 carbon atoms.

## Patentansprüche

1. Verbindung der Formel (II'): 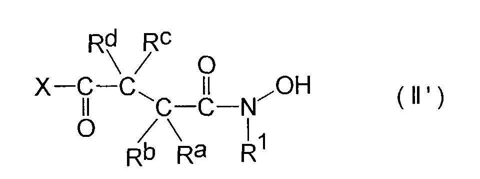 worin R¹ eine substituierte oder unsubstituierte Alkylgruppe mit 1 bis 6 Kohlenstoffatomen ist, R^{a}, R^{b}, R^{c} und R^{d} sind jeweils unabhängig voneinander ein Wasserstoffatom, eine substituierte oder unsubstituierte Alkylgruppe mit 1 bis 18 Kohlenstoffatomen oder eine substituierte oder unsubstituierte Alkenylgruppe mit 2 bis 18 Kohlenstoffatomen; X ist -OR⁶ oder -N(R⁶) (R⁷); R⁶ ist eine substituierte oder unsubstituierte Alkylgruppe mit 6 bis 22 Kohlenstoffatomen oder eine substituierte oder unsubstituierte Alkenylgruppe mit 6 bis 22 Kohlenstoffatomen, und R⁷ ist eine Alkylgruppe mit 6 bis 18 Kohlenstoffatomen, mit der. Massgabe, dass, wenn alle R^{a}, R^{b}, R^{c} und R^{d} Wasserstoffatome sind, R⁶ und R⁷, sofern vorhanden, jeweils eine substituierte oder unsubstituierte Alkylgruppe mit 14 oder mehr Kohlenstoffatomen darstellen, und dass die Verbindung der Formel (II') nicht N-Methyl-3-(dioctadecylcarbamoyl)propanhydroxamsäure ist.

2. Verbindung gemäss Anspruch 1, worin R^{a}, R^{b}, R^{c} und R^{d} alle Wasserstoffatome darstellen, R⁶ ist eine Myristylgruppe, eine Palmitylgruppe oder eine Stearylgruppe und R¹ ist eine Methylgruppe, eine Ethylgruppe oder eine n-Hexylgruppe.

3. Verbindung gemäss Anspruch 1, die die folgende Strukturformel aufweist: 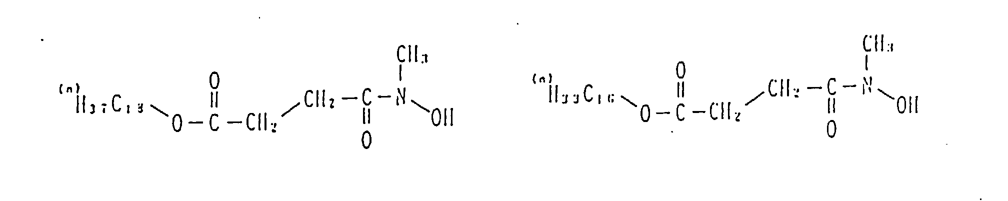 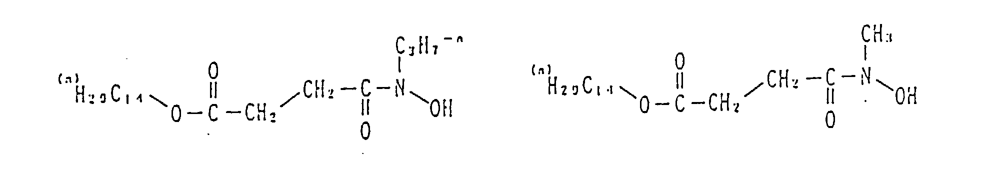 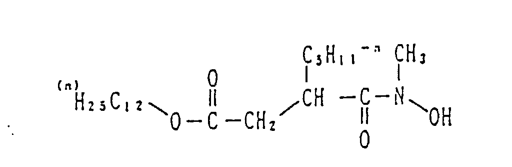 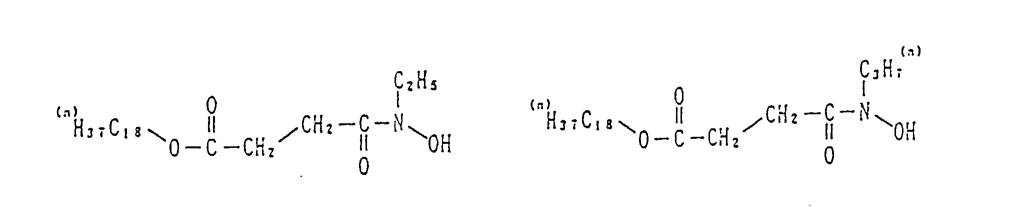 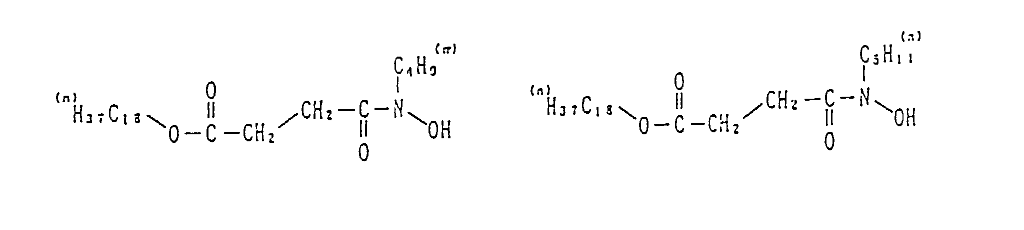 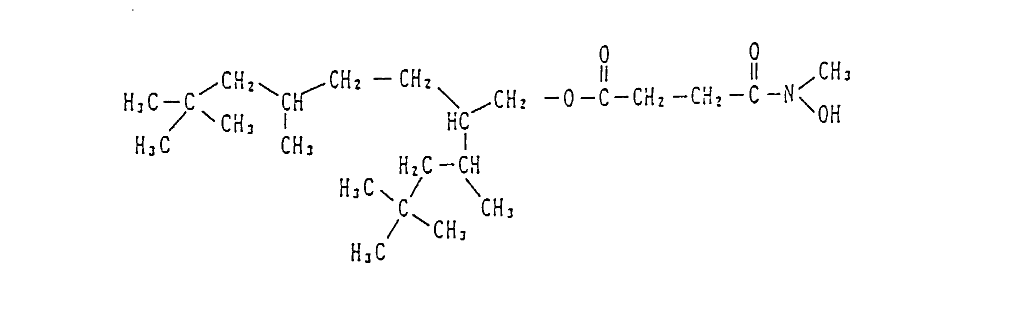 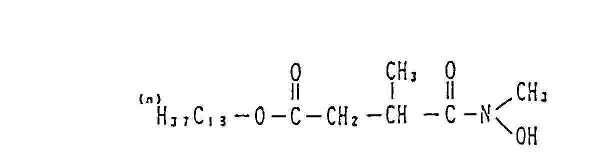 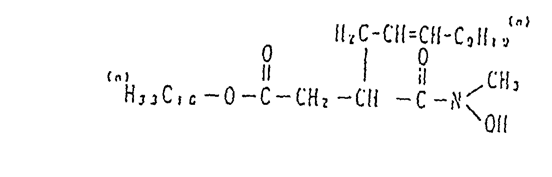 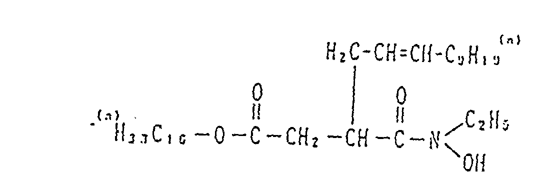 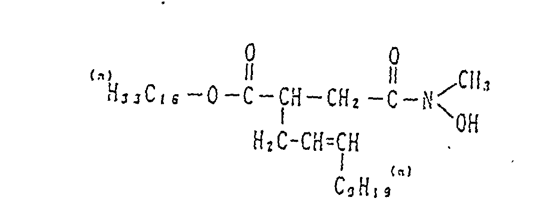 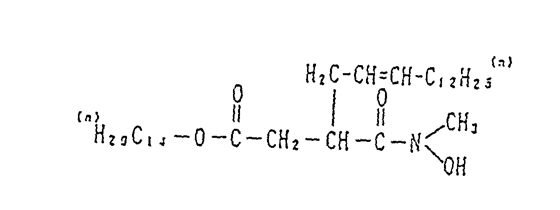 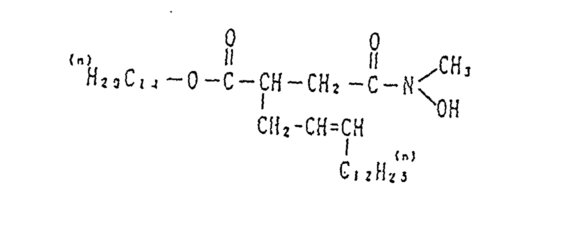 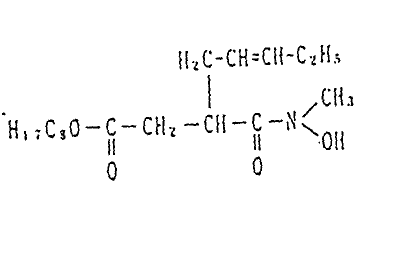 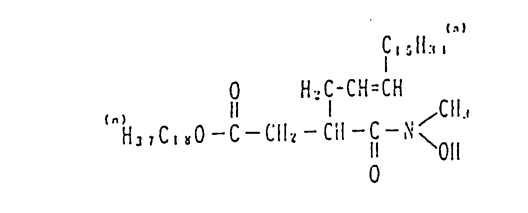 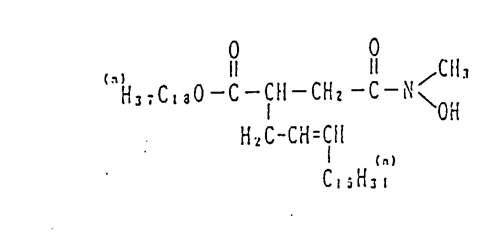 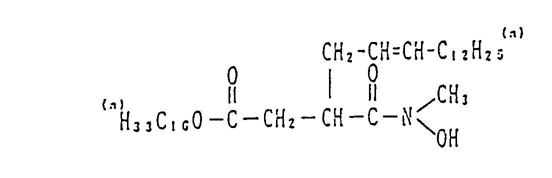 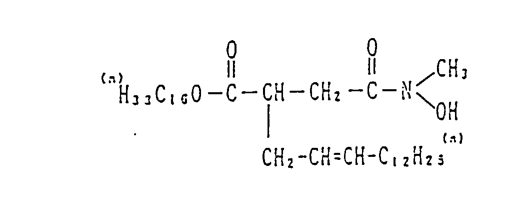 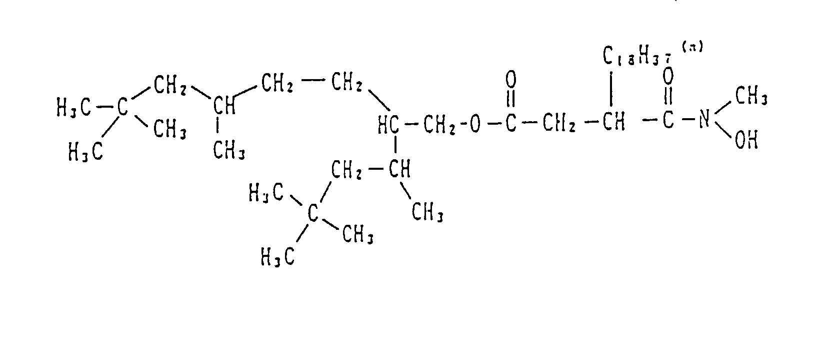 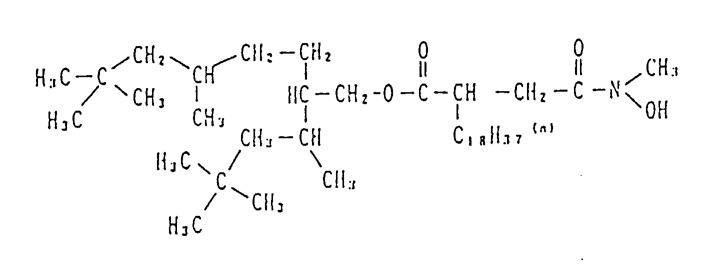 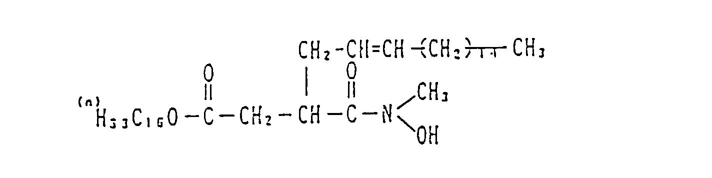 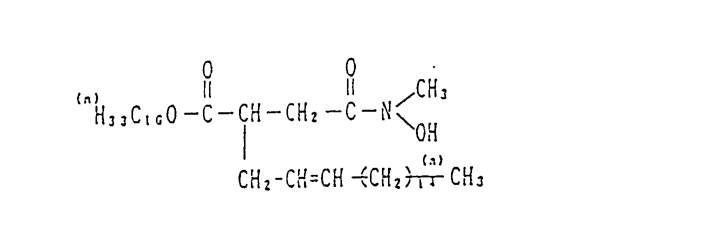 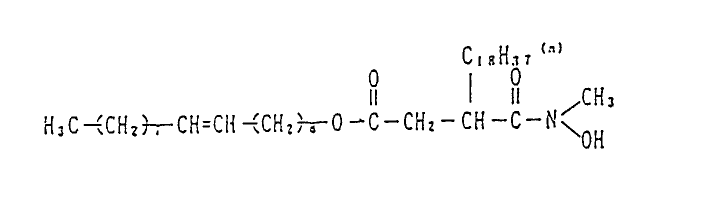 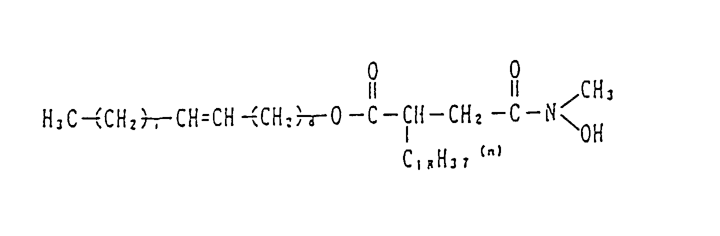 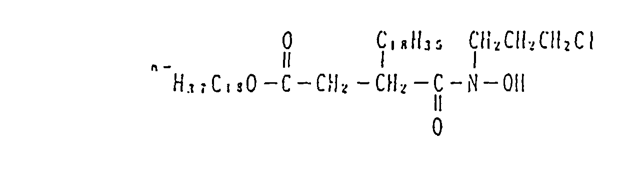 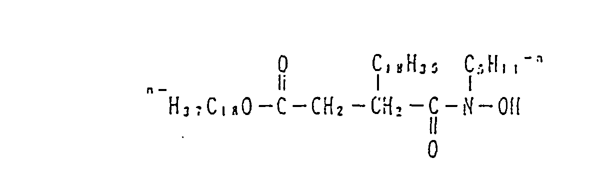 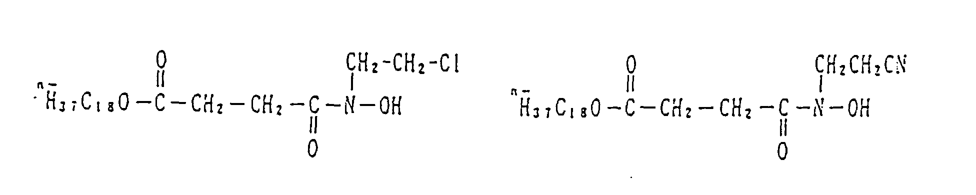 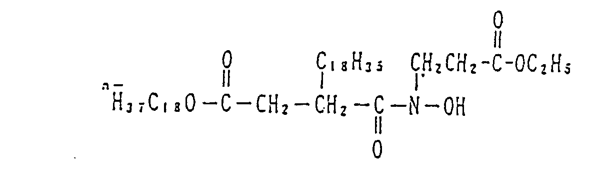 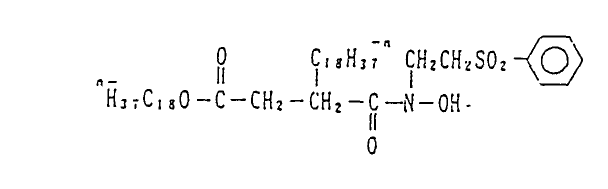 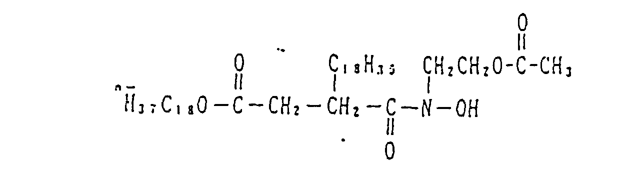 oder

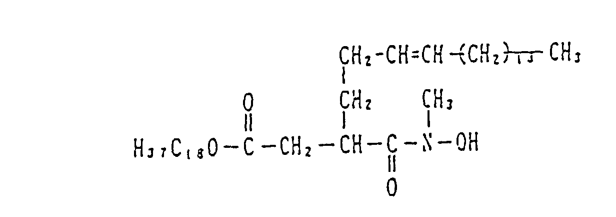

4. Fotografisches Silberhalogenidmaterial, das einen Träger und darauf aufgebracht mindestens eine lichtempfindliche Silberhalogenid-Emulsionsschicht umfasst, worin das fotografische Silberhalogenidmaterial eine Verbindung der Formel (II) enthält: 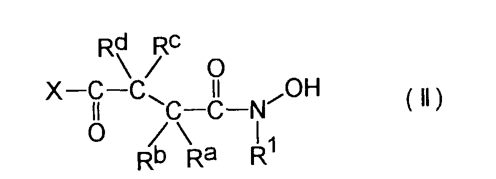 worin R¹ eine substituierte oder unsubstituierte Alkylgruppe mit 1 bis 6 Kohlenstoffatomen ist, R^{a}, R^{b}, R^{c} und R^{d} sind jeweils unabhängig voneinander ein Wasserstoffatom, eine substituierte oder unsubstituierte Alkylgruppe mit 1 bis 18 Kohlenstoffatomen oder eine substituierte oder unsubstituierte Alkenylgruppe mit 2 bis 18 Kohlenstoffatomen; X ist -OR⁶ oder -N(R⁶) (R⁷); und R⁶ und R⁷, die identisch oder voneinander verschieden sein können, sind jeweils ein Wasserstoffatom, eine substituierte oder unsubstituierte Alkylgruppe mit 1 bis 22 Kohlenstoffatomen, eine substituierte oder unsubstituierte Alkenylgruppe mit 2 bis 22 Kohlenstoffatomen oder eine Arylgruppe mit 6 bis 22 Kohlenstoffatomen.

5. Verwendung einer Verbindung der Formel (II) zur Verbesserung der Lagerstabilität eines Latentbildes eines fotografischen Silberhalogenidmaterials: 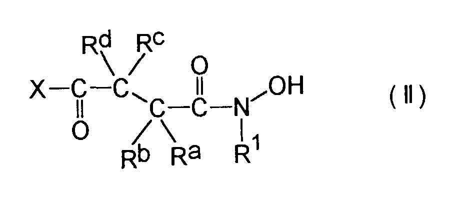 worin R¹ eine substituierte oder unsubstituierte Alkylgruppe mit 1 bis 6 Kohlenstoffatomen ist, R^{a}, R^{b}, R^{c} und R^{d} sind jeweils unabhängig voneinander ein Wasserstoffatom, eine substituierte oder unsubstituierte Alkylgruppe mit 1 bis 18 Kohlenstoffatomen oder eine substituierte oder unsubstituierte Alkenylgruppe mit 2 bis 18 Kohlenstoffatomen; X ist -OR⁶ oder -N(R⁶) (R⁷); und R⁶ und R⁷, die identisch oder voneinander verschieden sein können, sind jeweils ein Wasserstoffatom, eine substituierte oder unsubstituierte Alkylgruppe mit 1 bis 22 Kohlenstoffatomen, eine substituierte oder unsubstituierte Alkenylgruppe mit 2 bis 22 Kohlenstoffatomen oder eine Arylgruppe mit 6 bis 22 Kohlenstoffatomen.

6. Verwendung einer Verbindung der Formel (I) zur Verbesserung der Lagerstabilität eines Latentbildes eines fotografischen Silberhalogenidmaterials: 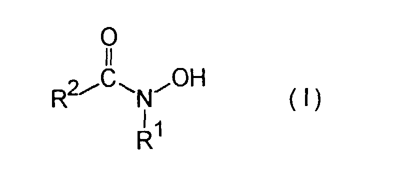 worin R¹ eine substituierte oder unsubstituierte Alkylgruppe ist, worin die Alkyleinheit 1 bis 6 Kohlenstoffatome aufweist, und R² ist eine Gruppe der Formel (I)-A:
R⁴―L―R³― (I)-A
worin R³ eine Alkylen- oder Alkenylengruppe mit 2 bis 20 Kohlenstoffatomen ist, R⁴ ist eine substituierte oder unsubstituierte Alkylgruppe mit 1 bis 30 Kohlenstoffatomen, eine substituierte oder unsubstituierte Arylgruppe mit 6 bis 20 Kohlenstoffatomen oder eine substituierte oder unsubstituierte heterocyclische Gruppe mit 2 bis 20 Kohlenstoffatomen; L ist -CO-, -N(R⁵), -S-, -SO₂-, -O-, -SO-, -COO-, -OCO-, -SO₂O-, -OSO₂, -SO₂N(R⁵)-, -N(R⁵)SO₂-, -N(R⁵)CO- oder -CON(R⁵)-; und R⁵ ist ein Wasserstoffatom oder eine substituierte oder unsubstituierte Alkylgruppe mit 1 bis 18 Kohlenstoffatomen, mit der Massgabe, dass, wenn L -O-ist, R⁴ eine substituierte oder unsubstituierte Alkylgruppe mit 1 bis 40 Kohlenstoffatomen ist.

## Revendications

1. Composé de formule (II)': 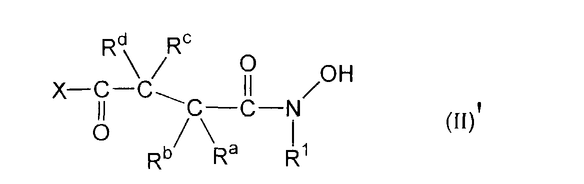 dans laquelle R¹ est un groupe alkyle substitué ou non substitué ayant 1 à 6 atomes de carbone, R^{a}, R^{b}, R^{c} et R^{d} sont chacun indépendamment un atome d'hydrogène, un groupe alkyle substitué ou non substitué ayant 1 à 18 atomes de carbone, ou un groupe alcényle substitué ou non substitué ayant 2 à 18 atomes de carbone ; X est -OR⁶ ou -N(R⁶)(R⁷); R⁶ est un groupe alkyle substitué ou non substitué ayant 6 à 22 atomes de carbone, ou un groupe alcényle substitué ou non substitué ayant 6 à 22 atomes de carbone, et R⁷ est un groupe alkyle ayant 6 à 18 atomes de carbone, sous réserve que quand tous les R^{a}, R^{b}, R^{c} et R^{d} sont des atomes d'hydrogène, R⁶ et R⁷, s'ils sont présents, sont chacun un groupe alkyle substitué ou non substitué ayant 14 atomes de carbone ou plus ; et que le composé de formule (II)' n'est pas l'acide N-méthyl-3-(dioctadécylcarbamoyl)-propanohydroxamique.

2. Composé selon la revendication 1, caractérisé en ce que R^{a}, R^{b}, R^{c} et R^{d} sont tous des atomes d'hydrogène, R⁶ est un groupe myristyle, un groupe palmityle ou un groupe stéaryle, et R¹ est un groupe méthyle, un groupe éthyle ou un groupe n-hexyle.

3. Composé selon la revendication 1, ayant la formule structurelle : 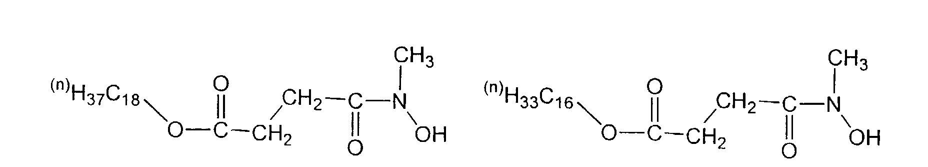 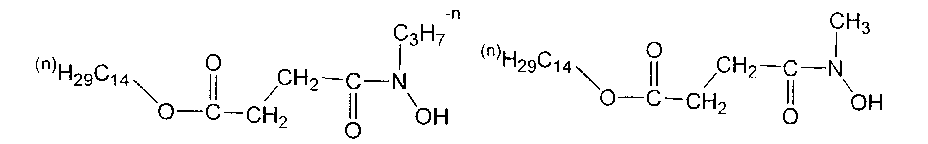 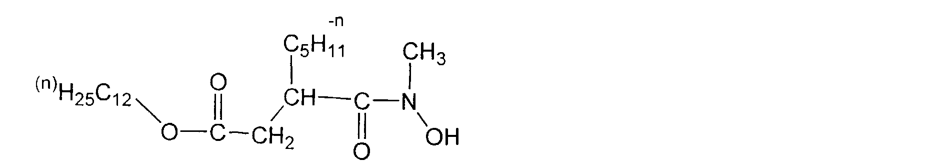 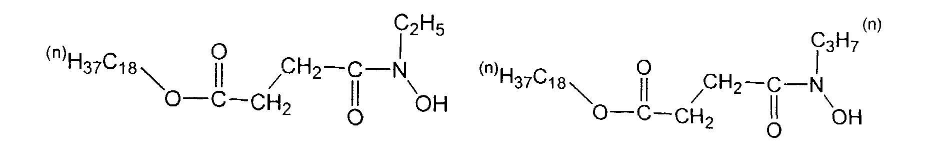 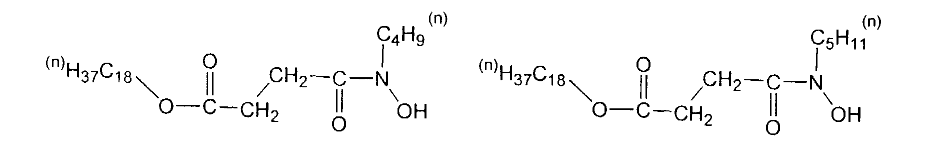 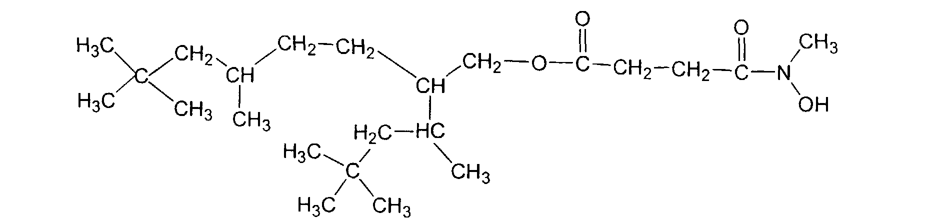 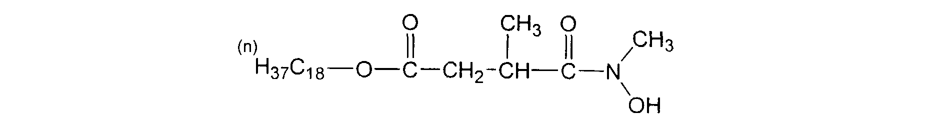 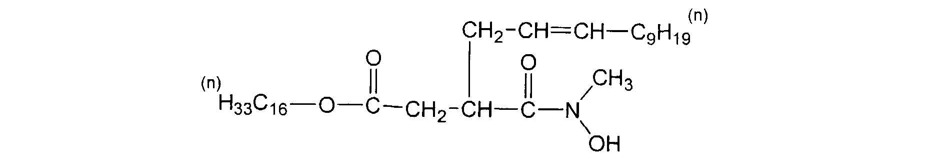 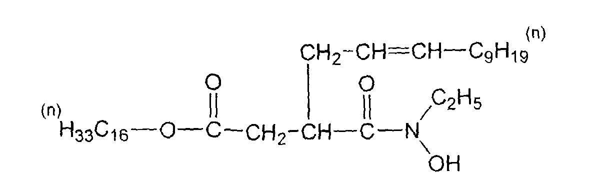 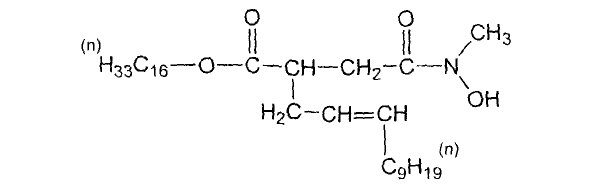 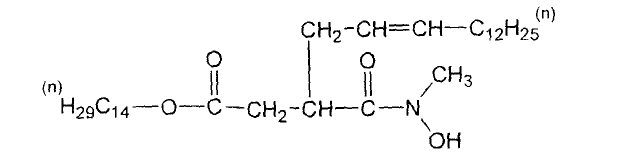 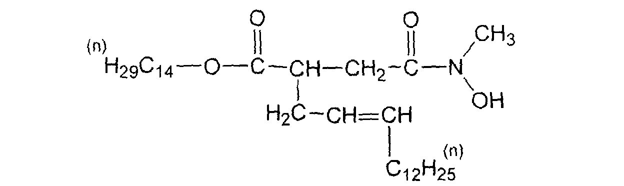 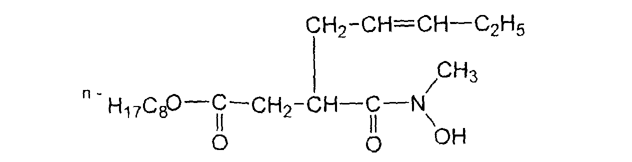 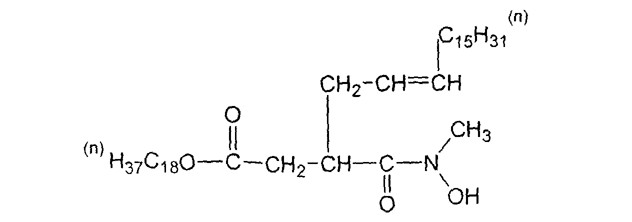 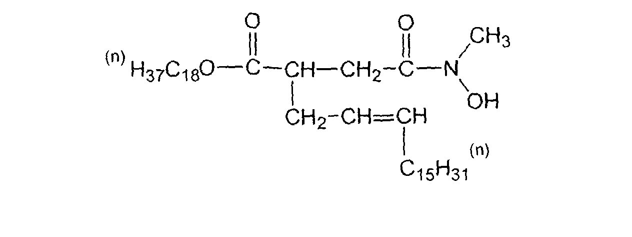 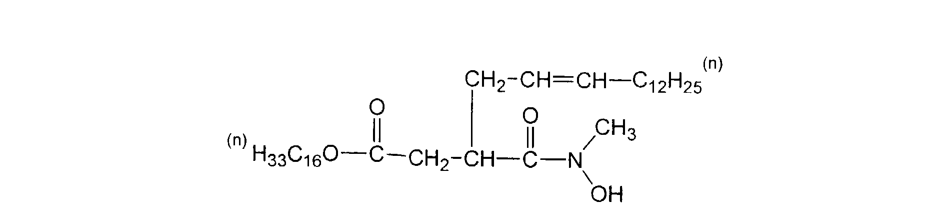 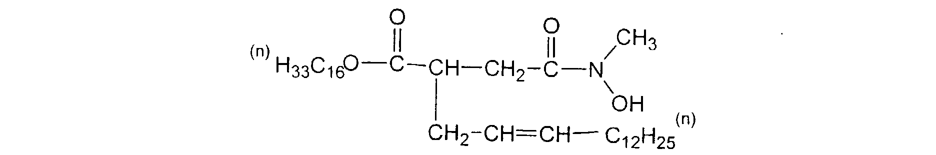 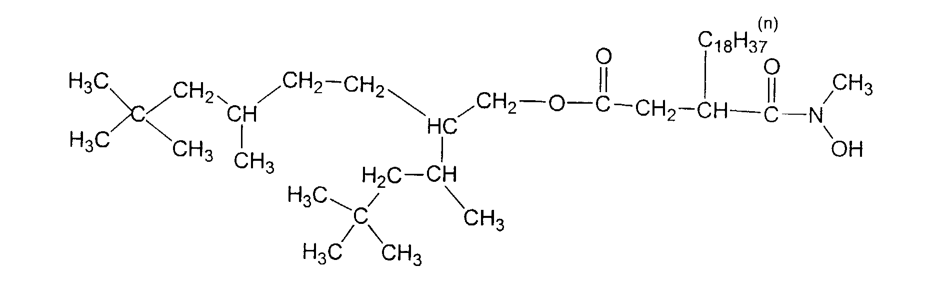 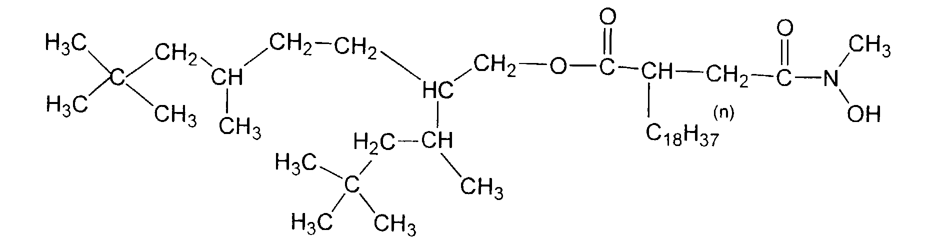 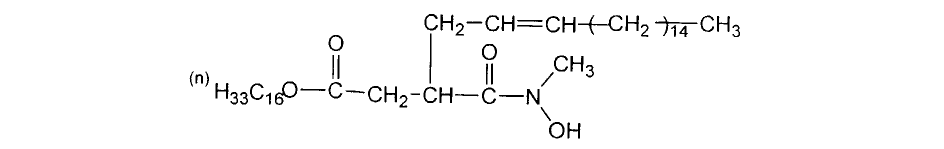 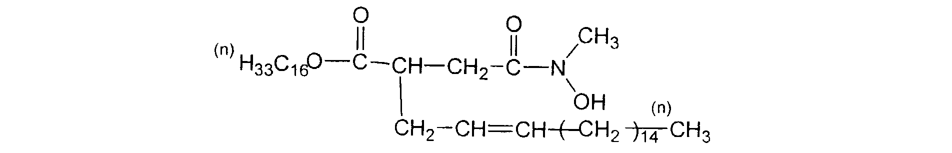 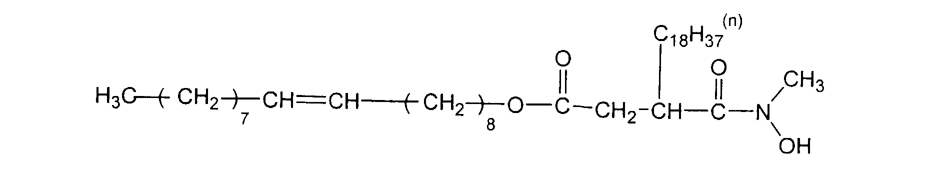 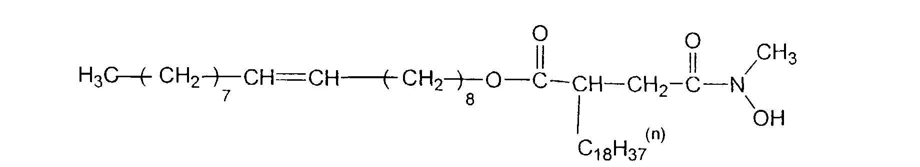 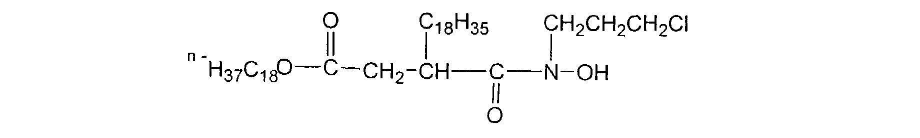 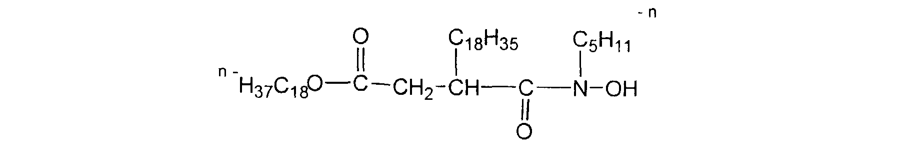 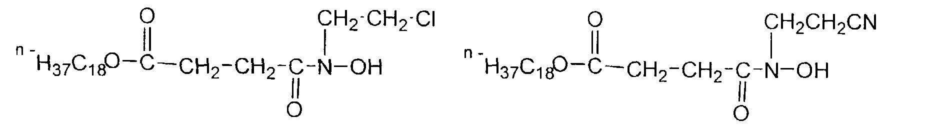 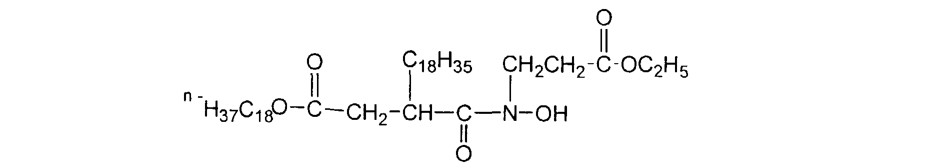 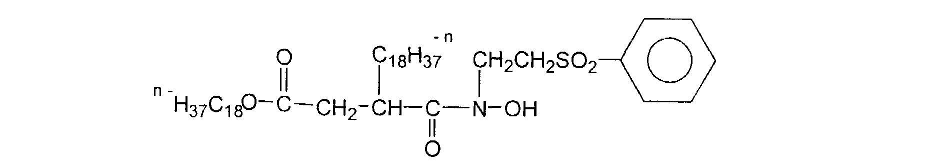 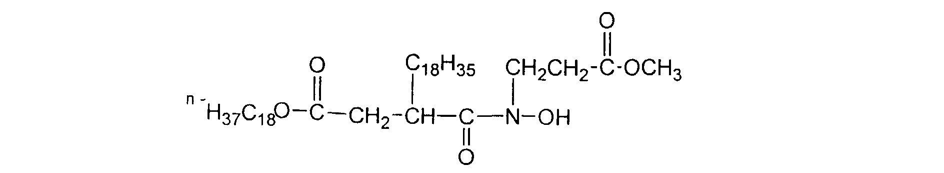 ou

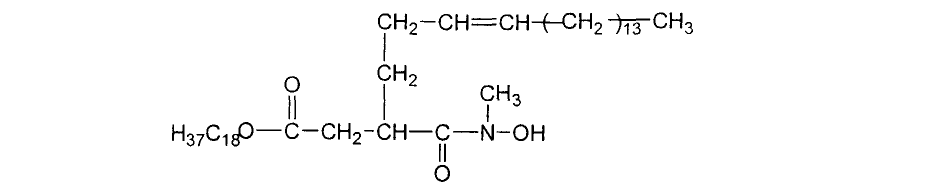

4. Matériau photographique à l'halogénure d'argent comprenant un support ayant sur celui-ci au moins une couche-d'émulsion d'halogénure d'argent photosensible, caractérisé en ce que le matériau photographique à l'halogénure d'argent contient un composé de formule (II) : 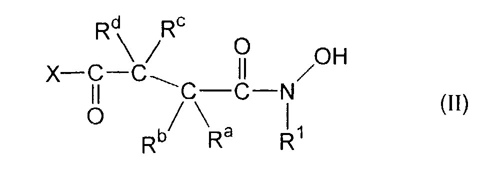 dans laquelle R¹ est un groupe alkyle substitué ou non substitué ayant 1 à 6 atomes de carbone, R^{a}, R^{b}, R^{c} et R^{d} sont chacun indépendamment un atome d'hydrogène, un groupe alkyle substitué ou non substitué ayant 1 à 18 atomes de carbone, ou un groupe alcényle substitué ou non substitué ayant 2 à 18 atomes de carbone ; X est -OR⁶ ou -N(R⁶)(R⁷); et R⁶ et R⁷, qui peuvent être identiques ou différents, sont chacun un atome d'hydrogène, un groupe alkyle substitué ou non substitué ayant 1 à 22 atomes de carbone, un groupe alcényle substitué ou non substitué ayant 2 à 22 atomes de carbone, ou un groupe aryle ayant 6 à 22 atomes de carbone.

5. Utilisation d'un composé de formule (II) pour améliorer la stabilité au stockage d'une image latente d'un matériau photographique à l'halogénure d'argent : 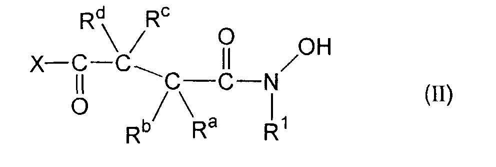 dans laquelle R¹ est un groupe alkyle substitué ou non substitué ayant 1 à 6 atomes de carbone, R^{a}, R^{b}, R^{c} et R^{d} sont chacun indépendamment un atome d'hydrogène, un groupe alkyle substitué ou non substitué ayant 1 à 18 atomes de carbone, ou un groupe alcényle substitué ou non substitué ayant 2 à 18 atomes de carbone ; X est -OR⁶ ou -N(R⁶)(R⁷); et R⁶ et R⁷, qui peuvent être identiques ou différents, sont chacun un atome d'hydrogène, un groupe alkyle substitué ou non substitué ayant 1 à 22 atomes de carbone, un groupe alcényle substitué ou non substitué ayant 2 à 22 atomes de carbone, ou un groupe aryle ayant 6 à 22 atomes de carbone.

6. Utilisation d'un composé de formule (I) pour améliorer la stabilité au stockage d'une image latente d'un matériau photographique à l'halogénure d'argent: 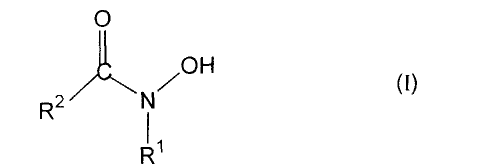 dans laquelle R¹ est un groupe alkyle substitué ou non substitué dans lequel le groupement alkyle a 1 à 6 atomes de carbone et R² est un groupe de formule (I)-A:
R⁴―L―R³- (I)-A
dans laquelle R³ est un groupe alkylène ou alcénylène substitué ou non substitué ayant 2 à 20 atomes de carbone ; R⁴ est un groupe alkyle substitué ou non substitué ayant 1 à 30 atomes de carbone, un groupe aryle substitué ou non substitué ayant 6 à 20 atomes de carbone, ou un groupe hétérocyclique substitué ou non substitué ayant 2 à 20 atomes de carbone; L est -CO-, -N(R⁵)-, -S-, -SO₂-, -O-, -SO-, -COO-, -OCO-, -SO₂O-, -OSO₂, -SO₂N(R⁵)-, -N(R⁵)SO₂-, -N(R⁵)CO- ou -CON(R⁵)-; et R⁵ est un atome d'hydrogène ou un groupe alkyle substitué ou non substitué ayant 1 à 18 atomes de carbone, sous réserve que quand L est -O-, R⁴ est un groupe alkyle substitué ou non substitué ayant 1 à 40 atomes de carbone.
